# EUROPEAN PATENT APPLICATION

(11) **EP 4 036 222 A1**
(43) Date of publication of application: **03.08.2022**
(21) Application number: 20867583.5
(22) Date of filing: 25.09.2020
(51) Int. Cl.: C12N 5/078, A61K 35/17, A61P 35/00

(54) **MODIFIED IMMUNE CELL AND USE THEREOF**

(30) Priority: 26.09.2019 CN 201910917070
(71) Applicant: Oricell Therapeutics Co., Ltd., Shanghai 201203 (CN)
(72) Inventor: LING, Youguo, Shanghai 201203 (CN); GUO, Hao, Shanghai 201203 (CN); CHEN, Siye, Shanghai 201203 (CN); LI, Huijiao, Shanghai 201203 (CN); YANG, Yue, Shanghai 201203 (CN); LI, Xiaopei, Shanghai 201203 (CN); YANG, Qi, Shanghai 201203 (CN); YANG, Huanfeng, Shanghai 201203 (CN); HE, Xiaowen, Shanghai 201203 (CN)
(74) Representative: Böhm, Brigitte
(86) International application number: PCT/CN2020/117936
(87) International publication number: WO 2021/057932

(57) **Abstract**

Provided is a modified immune cell, comprising a chimeric antigen receptor and/or a coding element therefor, or comprising a T cell receptor and/or a coding element therefor. The immune cell further comprises: leptin and/or a functional fragment thereof; and/or, a leptin receptor and/or a functional fragment thereof, and the expression quantity of the leptin receptor and/or the functional fragment thereof in the immune cell is increased compared with that in an immune cell without the corresponding modification. In addition, also provided is another modified immune cell, comprising leptin and/or a functional fragment thereof, and/or, a leptin receptor and/or a functional fragment thereof, and a low-density lipoprotein-receptor-related protein or a fragment thereof.

Sequence Listing

<110>  ORICELL THERAPEUTICS CO., LTD.

<120>  MODIFIED IMMUNE CELL AND USE THEREOF

<130>  0065-PA-027EP

<160>  48

<170>  PatentIn version 3.5

<210>  1
<211>  263
<212>  PRT
<213>  Artificial Sequence

<220>
<223>  GPC3 scFv

<400>  1

Met Ala Leu Pro Val Thr Ala Leu Leu Leu Pro Leu Ala Leu Leu Leu
1               5                   10                  15

His Ala Ala Arg Pro Asp Val Val Met Thr Gln Ser Pro Leu Ser Leu
            20                  25                  30

Pro Val Thr Pro Gly Glu Pro Ala Ser Ile Ser Cys Arg Ser Ser Gln
        35                  40                  45

Ser Leu Val His Ser Asn Gly Asn Thr Tyr Leu His Trp Tyr Leu Gln
    50                  55                  60

Lys Pro Gly Gln Ser Pro Gln Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Ser Gln Asn Thr His Val Pro Pro Thr Phe Gly Gln Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys
145                 150                 155                 160

Lys Pro Gly Ala Ser Val Lys Val Ser Cys Lys Ala Ser Gly Tyr Thr

165                 170                 175

Phe Thr Asp Tyr Glu Met His Trp Val Arg Gln Ala Pro Gly Gln Gly
            180                 185                 190

Leu Glu Trp Met Gly Ala Leu Asp Pro Lys Thr Gly Asp Thr Ala Tyr
        195                 200                 205

Ser Gln Lys Phe Lys Gly Arg Val Thr Leu Thr Ala Asp Glu Ser Thr
    210                 215                 220

Ser Thr Ala Tyr Met Glu Leu Ser Ser Leu Arg Ser Glu Asp Thr Ala
225                 230                 235                 240

Val Tyr Tyr Cys Thr Arg Phe Tyr Ser Tyr Thr Tyr Trp Gly Gln Gly
                245                 250                 255

Thr Leu Val Thr Val Ser Ser
            260

<210>  2
<211>  242
<212>  PRT
<213>  Artificial Sequence

<220>
<223>  CD19 scFv

<400>  2

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45

Tyr His Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Gly Gly Gly Gly Ser
            100                 105                 110

Gly Gly Gly Gly Ser Gly Gly Gly Gly Ser Glu Val Lys Leu Gln Glu
        115                 120                 125

Ser Gly Pro Gly Leu Val Ala Pro Ser Gln Ser Leu Ser Val Thr Cys
    130                 135                 140

Thr Val Ser Gly Val Ser Leu Pro Asp Tyr Gly Val Ser Trp Ile Arg
145                 150                 155                 160

Gln Pro Pro Arg Lys Gly Leu Glu Trp Leu Gly Val Ile Trp Gly Ser
                165                 170                 175

Glu Thr Thr Tyr Tyr Asn Ser Ala Leu Lys Ser Arg Leu Thr Ile Ile
            180                 185                 190

Lys Asp Asn Ser Lys Ser Gln Val Phe Leu Lys Met Asn Ser Leu Gln
        195                 200                 205

Thr Asp Asp Thr Ala Ile Tyr Tyr Cys Ala Lys His Tyr Tyr Tyr Gly
    210                 215                 220

Gly Ser Tyr Ala Met Asp Tyr Trp Gly Gln Gly Thr Ser Val Thr Val
225                 230                 235                 240

Ser Ser

<210>  3
<211>  246
<212>  PRT
<213>  Artificial Sequence

<220>
<223>  BCMA scFv

<400>  3

Asp Ile Val Leu Thr Gln Ser Pro Ala Ser Leu Ala Val Ser Leu Gly
1               5                   10                  15

Glu Arg Ala Thr Ile Asn Cys Arg Ala Ser Glu Ser Val Ser Val Ile
            20                  25                  30

Gly Ala His Leu Ile His Trp Tyr Gln Gln Lys Pro Gly Gln Pro Pro
        35                  40                  45

Lys Leu Leu Ile Tyr Leu Ala Ser Asn Leu Glu Thr Gly Val Pro Ala
    50                  55                  60

Arg Phe Ser Gly Ser Gly Ser Gly Thr Asp Phe Thr Leu Thr Ile Ser
65                  70                  75                  80

Ser Leu Gln Ala Glu Asp Ala Ala Ile Tyr Tyr Cys Leu Gln Ser Arg
                85                  90                  95

Ile Phe Pro Arg Thr Phe Gly Gln Gly Thr Lys Leu Glu Ile Lys Gly
            100                 105                 110

Ser Thr Ser Gly Ser Gly Lys Pro Gly Ser Gly Glu Gly Ser Thr Lys
        115                 120                 125

Gly Gln Val Gln Leu Val Gln Ser Gly Ser Glu Leu Lys Lys Pro Gly
    130                 135                 140

Ala Ser Val Lys Val Ser Cys Lys Ala Ser Gly Tyr Thr Phe Thr Asp
145                 150                 155                 160

Tyr Ser Ile Asn Trp Val Arg Gln Ala Pro Gly Gln Gly Leu Glu Trp
                165                 170                 175

Met Gly Trp Ile Asn Thr Glu Thr Arg Glu Pro Ala Tyr Ala Tyr Asp
            180                 185                 190

Phe Arg Gly Arg Phe Val Phe Ser Leu Asp Thr Ser Val Ser Thr Ala
        195                 200                 205

Tyr Leu Gln Ile Ser Ser Leu Lys Ala Glu Asp Thr Ala Val Tyr Tyr
    210                 215                 220

Cys Ala Arg Asp Tyr Ser Tyr Ala Met Asp Tyr Trp Gly Gln Gly Thr
225                 230                 235                 240

Leu Val Thr Val Ser Ser
                245

<210>  4
<211>  259
<212>  PRT
<213>  Artificial Sequence

<220>
<223>  MSLN scFv

<400>  4

Gln Val Gln Leu Gln Gln Ser Gly Pro Gly Leu Val Thr Pro Ser Gln
1               5                   10                  15

Thr Leu Ser Leu Thr Cys Ala Ile Ser Gly Asp Ser Val Ser Ser Asn
            20                  25                  30

Ser Ala Thr Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Met Ser Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Gly Met Met Thr Tyr Tyr Tyr Gly Met Asp Val
            100                 105                 110

Trp Gly Gln Gly Thr Thr Val Thr Val Ser Ser Gly Ile Leu Gly Ser
        115                 120                 125

Gly Gly Gly Gly Ser Gly Gly Gly Gly Ser Gly Gly Gly Gly Ser Gln
    130                 135                 140

Pro Val Leu Thr Gln Ser Ser Ser Leu Ser Ala Ser Pro Gly Ala Ser
145                 150                 155                 160

Ala Ser Leu Thr Cys Thr Leu Arg Ser Gly Ile Asn Val Gly Pro Tyr
                165                 170                 175

Arg Ile Tyr Trp Tyr Gln Gln Lys Pro Gly Ser Pro Pro Gln Tyr Leu
            180                 185                 190

Leu Asn Tyr Lys Ser Asp Ser Asp Lys Gln Gln Gly Ser Gly Val Pro
        195                 200                 205

Ser Arg Phe Ser Gly Ser Lys Asp Ala Ser Ala Asn Ala Gly Val Leu
    210                 215                 220

Leu Ile Ser Gly Leu Arg Ser Glu Asp Glu Ala Asp Tyr Tyr Cys Met
225                 230                 235                 240

Ile Trp His Ser Ser Ala Ala Val Phe Gly Gly Gly Thr Gln Leu Thr
                245                 250                 255

Val Leu Ser

<210>  5
<211>  241
<212>  PRT
<213>  Artificial Sequence

<220>
<223>  HER2 scFv

<400>  5

Gln Val Gln Leu Gln Gln Ser Gly Pro Glu Leu Lys Lys Pro Gly Glu
1               5                   10                  15

Thr Val Lys Ile Ser Cys Lys Ala Ser Gly Tyr Pro Phe Thr Asn Tyr
            20                  25                  30

Gly Met Asn Trp Val Lys Gln Ala Pro Gly Gln Gly Leu Lys Trp Met
        35                  40                  45

Gly Trp Ile Asn Thr Ser Thr Gly Glu Ser Thr Phe Ala Asp Asp Phe
    50                  55                  60

Lys Gly Arg Phe Asp Phe Ser Leu Glu Thr Ser Ala Asn Thr Ala Tyr
65                  70                  75                  80

Leu Gln Ile Asn Asn Leu Lys Ser Glu Asp Ser Ala Thr Tyr Phe Cys
                85                  90                  95

Ala Arg Trp Glu Val Tyr His Gly Tyr Val Pro Tyr Trp Gly Gln Gly
            100                 105                 110

Thr Thr Val Thr Val Ser Ser Gly Gly Gly Gly Ser Gly Gly Gly Gly
        115                 120                 125

Ser Gly Gly Gly Gly Ser Asp Ile Gln Leu Thr Gln Ser His Lys Phe
    130                 135                 140

Leu Ser Thr Ser Val Gly Asp Arg Val Ser Ile Thr Cys Lys Ala Ser
145                 150                 155                 160

Gln Asp Val Tyr Asn Ala Val Ala Trp Tyr Gln Gln Lys Pro Gly Gln
                165                 170                 175

Ser Pro Lys Leu Leu Ile Tyr Ser Ala Ser Ser Arg Tyr Thr Gly Val
            180                 185                 190

Pro Ser Arg Phe Thr Gly Ser Gly Ser Gly Pro Asp Phe Thr Phe Thr
        195                 200                 205

Ile Ser Ser Val Gln Ala Glu Asp Leu Ala Val Tyr Phe Cys Gln Gln
    210                 215                 220

His Phe Arg Thr Pro Phe Thr Phe Gly Ser Gly Thr Lys Leu Glu Ile
225                 230                 235                 240

Lys

<210>  6
<211>  46
<212>  PRT
<213>  Artificial Sequence

<220>
<223>  CD8 hinge region

<400>  6

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp Ile
        35                  40                  45

<210>  7
<211>  23
<212>  PRT
<213>  Artificial Sequence

<220>
<223>  CD8 transmembrane domain

<400>  7

Tyr Ile Trp Ala Pro Leu Ala Gly Thr Cys Gly Val Leu Leu Leu Ser
1               5                   10                  15

Leu Val Ile Thr Leu Tyr Cys
            20

<210>  8
<211>  41
<212>  PRT
<213>  Artificial Sequence

<220>
<223>  CD28 intracellular domain

<400>  8

Arg Ser Lys Arg Ser Arg Leu Leu His Ser Asp Tyr Met Asn Met Thr

1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  9
<211>  42
<212>  PRT
<213>  Artificial Sequence

<220>
<223>  41BB intracellular domain

<400>  9

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  10
<211>  112
<212>  PRT
<213>  Artificial Sequence

<220>
<223>  CD3zeta intracellular domain

<400>  10

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  11
<211>  18
<212>  PRT
<213>  Artificial Sequence

<220>
<223>  T2A

<400>  11

Glu Gly Arg Gly Ser Leu Leu Thr Cys Gly Asp Val Glu Glu Asn Pro
1               5                   10                  15

Gly Pro

<210>  12
<211>  19
<212>  PRT
<213>  Artificial Sequence

<220>
<223>  P2A

<400>  12

Ala Thr Asn Phe Ser Leu Leu Lys Gln Ala Gly Asp Val Glu Glu Asn
1               5                   10                  15

Pro Gly Pro

<210>  13
<211>  167
<212>  PRT
<213>  Artificial Sequence

<220>
<223>  secretory leptin

<400>  13

<210>  14
<211>  267
<212>  PRT
<213>  Artificial Sequence

<220>
<223>  membrane-bound leptin

<400>  14

Met His Trp Gly Thr Leu Cys Gly Phe Leu Trp Leu Trp Pro Tyr Leu
1               5                   10                  15

Phe Tyr Val Gln Ala Val Pro Ile Gln Lys Val Gln Asp Asp Thr Lys
            20                  25                  30

Thr Leu Ile Lys Thr Ile Val Thr Arg Ile Asn Asp Ile Ser His Thr
        35                  40                  45

Gln Ser Val Ser Ser Lys Gln Lys Val Thr Gly Leu Asp Phe Ile Pro
    50                  55                  60

Gly Leu His Pro Ile Leu Thr Leu Ser Lys Met Asp Gln Thr Leu Ala
65                  70                  75                  80

Val Tyr Gln Gln Ile Leu Thr Ser Met Pro Ser Arg Asn Val Ile Gln
                85                  90                  95

Ile Ser Asn Asp Leu Glu Asn Leu Arg Asp Leu Leu His Val Leu Ala
            100                 105                 110

Phe Ser Lys Ser Cys His Leu Pro Trp Ala Ser Gly Leu Glu Thr Leu
        115                 120                 125

Asp Ser Leu Gly Gly Val Leu Glu Ala Ser Gly Tyr Ser Thr Glu Val
    130                 135                 140

Val Ala Leu Ser Arg Leu Gln Gly Ser Leu Gln Asp Met Leu Trp Gln
145                 150                 155                 160

Leu Asp Leu Ser Pro Gly Cys Ser Gly Gly Gly Ser Gly Gly Gly Gly
                165                 170                 175

Ser Gly Gly Gly Gly Ser Gly Gly Gly Gly Ser Gly Gly Gly Ser Leu
            180                 185                 190

Gln Thr Thr Thr Pro Ala Pro Arg Pro Pro Thr Pro Ala Pro Thr Ile
        195                 200                 205

Ala Ser Gln Pro Leu Ser Leu Arg Pro Glu Ala Cys Arg Pro Ala Ala
    210                 215                 220

Gly Gly Ala Val His Thr Arg Gly Leu Asp Phe Ala Cys Asp Ile Asp
225                 230                 235                 240

Ala Gly Leu Tyr Val Ile Val Pro Val Ile Ile Ser Ser Ser Ile Leu
                245                 250                 255

Leu Leu Gly Thr Leu Leu Ile Ser His Gln Arg
            260                 265

<210>  15
<211>  28
<212>  PRT
<213>  Artificial Sequence

<220>
<223>  transmembrane domain of leptin receptor

<400>  15

Asp Ala Gly Leu Tyr Val Ile Val Pro Val Ile Ile Ser Ser Ser Ile
1               5                   10                  15

Leu Leu Leu Gly Thr Leu Leu Ile Ser His Gln Arg
            20                  25

<210>  16
<211>  21
<212>  PRT
<213>  Artificial Sequence

<220>
<223>  signal peptide of leptin receptor

<400>  16

Met Ile Cys Gln Lys Phe Cys Val Val Leu Leu His Trp Glu Phe Ile
1               5                   10                  15

Tyr Val Ile Thr Ala
            20

<210>  17
<211>  530
<212>  PRT
<213>  Artificial Sequence

<220>
<223>  leptin receptor

<400>  17

Ile Lys Val Pro Met Arg Gly Pro Glu Phe Trp Arg Ile Ile Asn Gly
1               5                   10                  15

Asp Thr Met Lys Lys Glu Lys Asn Val Thr Leu Leu Trp Lys Pro Leu
            20                  25                  30

Met Lys Asn Asp Ser Leu Cys Ser Val Gln Arg Tyr Val Ile Asn His
        35                  40                  45

His Thr Ser Cys Asn Gly Thr Trp Ser Glu Asp Val Gly Asn His Thr
    50                  55                  60

Lys Phe Thr Phe Leu Trp Thr Glu Gln Ala His Thr Val Thr Val Leu
65                  70                  75                  80

Ala Ile Asn Ser Ile Gly Ala Ser Val Ala Asn Phe Asn Leu Thr Phe
                85                  90                  95

Ser Trp Pro Met Ser Lys Val Asn Ile Val Gln Ser Leu Ser Ala Tyr
            100                 105                 110

Pro Leu Asn Ser Ser Cys Val Ile Val Ser Trp Ile Leu Ser Pro Ser
        115                 120                 125

Asp Tyr Lys Leu Met Tyr Phe Ile Ile Glu Trp Lys Asn Leu Asn Glu
    130                 135                 140

Asp Gly Glu Ile Lys Trp Leu Arg Ile Ser Ser Ser Val Lys Lys Tyr
145                 150                 155                 160

Tyr Ile His Asp His Phe Ile Pro Ile Glu Lys Tyr Gln Phe Ser Leu
                165                 170                 175

Tyr Pro Ile Phe Met Glu Gly Val Gly Lys Pro Lys Ile Ile Asn Ser
            180                 185                 190

Phe Thr Gln Asp Asp Ile Glu Lys His Gln Ser Asp Ala Gly Leu Tyr
        195                 200                 205

Val Ile Val Pro Val Ile Ile Ser Ser Ser Ile Leu Leu Leu Gly Thr
    210                 215                 220

Leu Leu Ile Ser His Gln Arg Met Lys Lys Leu Phe Trp Glu Asp Val
225                 230                 235                 240

Pro Asn Pro Lys Asn Cys Ser Trp Ala Gln Gly Leu Asn Phe Gln Lys
                245                 250                 255

Pro Glu Thr Phe Glu His Leu Phe Ile Lys His Thr Ala Ser Val Thr
            260                 265                 270

Cys Gly Pro Leu Leu Leu Glu Pro Glu Thr Ile Ser Glu Asp Ile Ser
        275                 280                 285

Val Asp Thr Ser Trp Lys Asn Lys Asp Glu Met Met Pro Thr Thr Val
    290                 295                 300

Val Ser Leu Leu Ser Thr Thr Asp Leu Glu Lys Gly Ser Val Cys Ile
305                 310                 315                 320

Ser Asp Gln Phe Asn Ser Val Asn Phe Ser Glu Ala Glu Gly Thr Glu
                325                 330                 335

Val Thr Tyr Glu Asp Glu Ser Gln Arg Gln Pro Phe Val Lys Tyr Ala
            340                 345                 350

Thr Leu Ile Ser Asn Ser Lys Pro Ser Glu Thr Gly Glu Glu Gln Gly
        355                 360                 365

Leu Ile Asn Ser Ser Val Thr Lys Cys Phe Ser Ser Lys Asn Ser Pro

370                 375                 380

Leu Lys Asp Ser Phe Ser Asn Ser Ser Trp Glu Ile Glu Ala Gln Ala
385                 390                 395                 400

Phe Phe Ile Leu Ser Asp Gln His Pro Asn Ile Ile Ser Pro His Leu
                405                 410                 415

Thr Phe Ser Glu Gly Leu Asp Glu Leu Leu Lys Leu Glu Gly Asn Phe
            420                 425                 430

Pro Glu Glu Asn Asn Asp Lys Lys Ser Ile Tyr Tyr Leu Gly Val Thr
        435                 440                 445

Ser Ile Lys Lys Arg Glu Ser Gly Val Leu Leu Thr Asp Lys Ser Arg
    450                 455                 460

Val Ser Cys Pro Phe Pro Ala Pro Cys Leu Phe Thr Asp Ile Arg Val
465                 470                 475                 480

Leu Gln Asp Ser Cys Ser His Phe Val Glu Asn Asn Ile Asn Leu Gly
                485                 490                 495

Thr Ser Ser Lys Lys Thr Phe Ala Ser Tyr Met Pro Gln Phe Gln Thr
            500                 505                 510

Cys Ser Thr Gln Thr His Lys Ile Met Glu Asn Lys Met Cys Asp Leu
        515                 520                 525

Thr Val
    530

<210>  18
<211>  370
<212>  PRT
<213>  Artificial Sequence

<220>
<223>  LRP6

<400>  18

Gly Glu Pro Pro Thr Cys Ser Pro Gln Gln Phe Thr Cys Phe Thr Gly
1               5                   10                  15

Glu Ile Asp Cys Ile Pro Val Ala Trp Arg Cys Asp Gly Phe Thr Glu
            20                  25                  30

Cys Glu Asp His Ser Asp Glu Leu Asn Cys Pro Val Cys Ser Glu Ser
        35                  40                  45

Gln Phe Gln Cys Ala Ser Gly Gln Cys Ile Asp Gly Ala Leu Arg Cys
    50                  55                  60

Asn Gly Asp Ala Asn Cys Gln Asp Lys Ser Asp Glu Lys Asn Cys Glu
65                  70                  75                  80

Val Leu Cys Leu Ile Asp Gln Phe Arg Cys Ala Asn Gly Gln Cys Ile
                85                  90                  95

Gly Lys His Lys Lys Cys Asp His Asn Val Asp Cys Ser Asp Lys Ser
            100                 105                 110

Asp Glu Leu Asp Cys Tyr Pro Thr Glu Glu Pro Ala Pro Gln Ala Thr
        115                 120                 125

Asn Thr Val Gly Ser Val Ile Gly Val Ile Val Thr Ile Phe Val Ser
    130                 135                 140

Gly Thr Val Tyr Phe Ile Cys Gln Arg Met Leu Cys Pro Arg Met Lys
145                 150                 155                 160

Gly Asp Gly Glu Thr Met Thr Asn Asp Tyr Val Val His Gly Pro Ala
                165                 170                 175

Ser Val Pro Leu Gly Tyr Val Pro His Pro Ser Ser Leu Ser Gly Ser
            180                 185                 190

Leu Pro Gly Met Ser Arg Gly Lys Ser Met Ile Ser Ser Leu Ser Ile
        195                 200                 205

Met Gly Gly Ser Ser Gly Pro Pro Tyr Asp Arg Ala His Val Thr Gly
    210                 215                 220

Ala Ser Ser Ser Ser Ser Ser Ser Thr Lys Gly Thr Tyr Phe Pro Ala
225                 230                 235                 240

Ile Leu Asn Pro Pro Pro Ser Pro Ala Thr Glu Arg Ser His Tyr Thr
                245                 250                 255

Met Glu Phe Gly Tyr Ser Ser Asn Ser Pro Ser Thr His Arg Ser Tyr
            260                 265                 270

Ser Tyr Arg Pro Tyr Ser Tyr Arg His Phe Ala Pro Pro Thr Thr Pro
        275                 280                 285

Cys Ser Thr Asp Val Cys Asp Ser Asp Tyr Ala Pro Ser Arg Arg Met

290                 295                 300

Thr Ser Val Ala Thr Ala Lys Gly Tyr Thr Ser Asp Leu Asn Tyr Asp
305                 310                 315                 320

Ser Glu Pro Val Pro Pro Pro Pro Thr Pro Arg Ser Gln Tyr Leu Ser
                325                 330                 335

Ala Glu Glu Asn Tyr Glu Ser Cys Pro Pro Ser Pro Tyr Thr Glu Arg
            340                 345                 350

Ser Tyr Ser His His Leu Tyr Pro Pro Pro Pro Ser Pro Cys Thr Asp
        355                 360                 365

Ser Ser
    370

<210>  19
<211>  243
<212>  PRT
<213>  Artificial Sequence

<220>
<223>  TL6

<400>  19

Thr Asn Thr Val Gly Ser Val Ile Gly Val Ile Val Thr Ile Phe Val
1               5                   10                  15

Ser Gly Thr Val Tyr Phe Ile Cys Gln Arg Met Leu Cys Pro Arg Met
            20                  25                  30

Lys Gly Asp Gly Glu Thr Met Thr Asn Asp Tyr Val Val His Gly Pro
        35                  40                  45

Ala Ser Val Pro Leu Gly Tyr Val Pro His Pro Ser Ser Leu Ser Gly
    50                  55                  60

Ser Leu Pro Gly Met Ser Arg Gly Lys Ser Met Ile Ser Ser Leu Ser
65                  70                  75                  80

Ile Met Gly Gly Ser Ser Gly Pro Pro Tyr Asp Arg Ala His Val Thr
                85                  90                  95

Gly Ala Ser Ser Ser Ser Ser Ser Ser Thr Lys Gly Thr Tyr Phe Pro
            100                 105                 110

Ala Ile Leu Asn Pro Pro Pro Ser Pro Ala Thr Glu Arg Ser His Tyr
        115                 120                 125

Thr Met Glu Phe Gly Tyr Ser Ser Asn Ser Pro Ser Thr His Arg Ser
    130                 135                 140

Tyr Ser Tyr Arg Pro Tyr Ser Tyr Arg His Phe Ala Pro Pro Thr Thr
145                 150                 155                 160

Pro Cys Ser Thr Asp Val Cys Asp Ser Asp Tyr Ala Pro Ser Arg Arg
                165                 170                 175

Met Thr Ser Val Ala Thr Ala Lys Gly Tyr Thr Ser Asp Leu Asn Tyr
            180                 185                 190

Asp Ser Glu Pro Val Pro Pro Pro Pro Thr Pro Arg Ser Gln Tyr Leu
        195                 200                 205

Ser Ala Glu Glu Asn Tyr Glu Ser Cys Pro Pro Ser Pro Tyr Thr Glu
    210                 215                 220

Arg Ser Tyr Ser His His Leu Tyr Pro Pro Pro Pro Ser Pro Cys Thr
225                 230                 235                 240

Asp Ser Ser

<210>  20
<211>  362
<212>  PRT
<213>  Artificial Sequence

<220>
<223>  LRP5

<400>  20

Gly Glu Pro Pro Thr Cys Ser Pro Asp Gln Phe Ala Cys Ala Thr Gly
1               5                   10                  15

Glu Ile Asp Cys Ile Pro Gly Ala Trp Arg Cys Asp Gly Phe Pro Glu
            20                  25                  30

Cys Asp Asp Gln Ser Asp Glu Glu Gly Cys Pro Val Cys Ser Ala Ala
        35                  40                  45

Gln Phe Pro Cys Ala Arg Gly Gln Cys Val Asp Leu Arg Leu Arg Cys
    50                  55                  60

Asp Gly Glu Ala Asp Cys Gln Asp Arg Ser Asp Glu Ala Asp Cys Asp
65                  70                  75                  80

Ala Ile Cys Leu Pro Asn Gln Phe Arg Cys Ala Ser Gly Gln Cys Val
                85                  90                  95

Leu Ile Lys Gln Gln Cys Asp Ser Phe Pro Asp Cys Ile Asp Gly Ser
            100                 105                 110

Asp Glu Leu Met Cys Glu Ile Thr Lys Pro Pro Ser Asp Asp Ser Pro
        115                 120                 125

Ala His Ser Ser Ala Ile Gly Pro Val Ile Gly Ile Ile Leu Ser Leu
    130                 135                 140

Phe Val Met Gly Gly Val Tyr Phe Val Cys Gln Arg Val Val Cys Gln
145                 150                 155                 160

Arg Tyr Ala Gly Ala Asn Gly Pro Phe Pro His Glu Tyr Val Ser Gly
                165                 170                 175

Thr Pro His Val Pro Leu Asn Phe Ile Ala Pro Gly Gly Ser Gln His
            180                 185                 190

Gly Pro Phe Thr Gly Ile Ala Cys Gly Lys Ser Met Met Ser Ser Val
        195                 200                 205

Ser Leu Met Gly Gly Arg Gly Gly Val Pro Leu Tyr Asp Arg Asn His
    210                 215                 220

Val Thr Gly Ala Ser Ser Ser Ser Ser Ser Ser Thr Lys Ala Thr Leu
225                 230                 235                 240

Tyr Pro Pro Ile Leu Asn Pro Pro Pro Ser Pro Ala Thr Asp Pro Ser
                245                 250                 255

Leu Tyr Asn Met Asp Met Phe Tyr Ser Ser Asn Ile Pro Ala Thr Ala
            260                 265                 270

Arg Pro Tyr Arg Pro Tyr Ile Ile Arg Gly Met Ala Pro Pro Thr Thr
        275                 280                 285

Pro Cys Ser Thr Asp Val Cys Asp Ser Asp Tyr Ser Ala Ser Arg Trp
    290                 295                 300

Lys Ala Ser Lys Tyr Tyr Leu Asp Leu Asn Ser Asp Ser Asp Pro Tyr
305                 310                 315                 320

Pro Pro Pro Pro Thr Pro His Ser Gln Tyr Leu Ser Ala Glu Asp Ser
                325                 330                 335

Cys Pro Pro Ser Pro Ala Thr Glu Arg Ser Tyr Phe His Leu Phe Pro
            340                 345                 350

Pro Pro Pro Ser Pro Cys Thr Asp Ser Ser
        355                 360

<210>  21
<211>  231
<212>  PRT
<213>  Artificial Sequence

<220>
<223>  TL5

<400>  21

Ser Ala Ile Gly Pro Val Ile Gly Ile Ile Leu Ser Leu Phe Val Met
1               5                   10                  15

Gly Gly Val Tyr Phe Val Cys Gln Arg Val Val Cys Gln Arg Tyr Ala
            20                  25                  30

Gly Ala Asn Gly Pro Phe Pro His Glu Tyr Val Ser Gly Thr Pro His
        35                  40                  45

Val Pro Leu Asn Phe Ile Ala Pro Gly Gly Ser Gln His Gly Pro Phe
    50                  55                  60

Thr Gly Ile Ala Cys Gly Lys Ser Met Met Ser Ser Val Ser Leu Met
65                  70                  75                  80

Gly Gly Arg Gly Gly Val Pro Leu Tyr Asp Arg Asn His Val Thr Gly
                85                  90                  95

Ala Ser Ser Ser Ser Ser Ser Ser Thr Lys Ala Thr Leu Tyr Pro Pro
            100                 105                 110

Ile Leu Asn Pro Pro Pro Ser Pro Ala Thr Asp Pro Ser Leu Tyr Asn
        115                 120                 125

Met Asp Met Phe Tyr Ser Ser Asn Ile Pro Ala Thr Ala Arg Pro Tyr
    130                 135                 140

Arg Pro Tyr Ile Ile Arg Gly Met Ala Pro Pro Thr Thr Pro Cys Ser
145                 150                 155                 160

Thr Asp Val Cys Asp Ser Asp Tyr Ser Ala Ser Arg Trp Lys Ala Ser
                165                 170                 175

Lys Tyr Tyr Leu Asp Leu Asn Ser Asp Ser Asp Pro Tyr Pro Pro Pro
            180                 185                 190

Pro Thr Pro His Ser Gln Tyr Leu Ser Ala Glu Asp Ser Cys Pro Pro
        195                 200                 205

Ser Pro Ala Thr Glu Arg Ser Tyr Phe His Leu Phe Pro Pro Pro Pro
    210                 215                 220

Ser Pro Cys Thr Asp Ser Ser
225                 230

<210>  22
<211>  26
<212>  PRT
<213>  Artificial Sequence

<220>
<223>  linker SG3(SG4)3SG3SLQ

<400>  22

Ser Gly Gly Gly Ser Gly Gly Gly Gly Ser Gly Gly Gly Gly Ser Gly
1               5                   10                  15

Gly Gly Gly Ser Gly Gly Gly Ser Leu Gln
            20                  25

<210>  23
<211>  486
<212>  PRT
<213>  Artificial Sequence

<220>
<223>  CAR1(GPC3-41BB)

<400>  23

Met Ala Leu Pro Val Thr Ala Leu Leu Leu Pro Leu Ala Leu Leu Leu
1               5                   10                  15

His Ala Ala Arg Pro Asp Val Val Met Thr Gln Ser Pro Leu Ser Leu
            20                  25                  30

Pro Val Thr Pro Gly Glu Pro Ala Ser Ile Ser Cys Arg Ser Ser Gln
        35                  40                  45

Ser Leu Val His Ser Asn Gly Asn Thr Tyr Leu His Trp Tyr Leu Gln
    50                  55                  60

Lys Pro Gly Gln Ser Pro Gln Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Ser Gln Asn Thr His Val Pro Pro Thr Phe Gly Gln Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys
145                 150                 155                 160

Lys Pro Gly Ala Ser Val Lys Val Ser Cys Lys Ala Ser Gly Tyr Thr
                165                 170                 175

Phe Thr Asp Tyr Glu Met His Trp Val Arg Gln Ala Pro Gly Gln Gly
            180                 185                 190

Leu Glu Trp Met Gly Ala Leu Asp Pro Lys Thr Gly Asp Thr Ala Tyr
        195                 200                 205

Ser Gln Lys Phe Lys Gly Arg Val Thr Leu Thr Ala Asp Glu Ser Thr
    210                 215                 220

Ser Thr Ala Tyr Met Glu Leu Ser Ser Leu Arg Ser Glu Asp Thr Ala
225                 230                 235                 240

Val Tyr Tyr Cys Thr Arg Phe Tyr Ser Tyr Thr Tyr Trp Gly Gln Gly
                245                 250                 255

Thr Leu Val Thr Val Ser Ser Thr Thr Thr Pro Ala Pro Arg Pro Pro
            260                 265                 270

Thr Pro Ala Pro Thr Ile Ala Ser Gln Pro Leu Ser Leu Arg Pro Glu
        275                 280                 285

Ala Cys Arg Pro Ala Ala Gly Gly Ala Val His Thr Arg Gly Leu Asp
    290                 295                 300

Phe Ala Cys Asp Ile Tyr Ile Trp Ala Pro Leu Ala Gly Thr Cys Gly
305                 310                 315                 320

Val Leu Leu Leu Ser Leu Val Ile Thr Leu Tyr Cys Lys Arg Gly Arg

325                 330                 335

Lys Lys Leu Leu Tyr Ile Phe Lys Gln Pro Phe Met Arg Pro Val Gln
            340                 345                 350

Thr Thr Gln Glu Glu Asp Gly Cys Ser Cys Arg Phe Pro Glu Glu Glu
        355                 360                 365

Glu Gly Gly Cys Glu Leu Arg Val Lys Phe Ser Arg Ser Ala Asp Ala
    370                 375                 380

Pro Ala Tyr Gln Gln Gly Gln Asn Gln Leu Tyr Asn Glu Leu Asn Leu
385                 390                 395                 400

Gly Arg Arg Glu Glu Tyr Asp Val Leu Asp Lys Arg Arg Gly Arg Asp
                405                 410                 415

Pro Glu Met Gly Gly Lys Pro Arg Arg Lys Asn Pro Gln Glu Gly Leu
            420                 425                 430

Tyr Asn Glu Leu Gln Lys Asp Lys Met Ala Glu Ala Tyr Ser Glu Ile
        435                 440                 445

Gly Met Lys Gly Glu Arg Arg Arg Gly Lys Gly His Asp Gly Leu Tyr
    450                 455                 460

Gln Gly Leu Ser Thr Ala Thr Lys Asp Thr Tyr Asp Ala Leu His Met
465                 470                 475                 480

Gln Ala Leu Pro Pro Arg
                485

<210>  24
<211>  469
<212>  PRT
<213>  Artificial Sequence

<220>
<223>  CAR2(BCMA-41BB)

<400>  24

Asp Ile Val Leu Thr Gln Ser Pro Ala Ser Leu Ala Val Ser Leu Gly
1               5                   10                  15

Glu Arg Ala Thr Ile Asn Cys Arg Ala Ser Glu Ser Val Ser Val Ile
            20                  25                  30

Gly Ala His Leu Ile His Trp Tyr Gln Gln Lys Pro Gly Gln Pro Pro
        35                  40                  45

Lys Leu Leu Ile Tyr Leu Ala Ser Asn Leu Glu Thr Gly Val Pro Ala
    50                  55                  60

Arg Phe Ser Gly Ser Gly Ser Gly Thr Asp Phe Thr Leu Thr Ile Ser
65                  70                  75                  80

Ser Leu Gln Ala Glu Asp Ala Ala Ile Tyr Tyr Cys Leu Gln Ser Arg
                85                  90                  95

Ile Phe Pro Arg Thr Phe Gly Gln Gly Thr Lys Leu Glu Ile Lys Gly
            100                 105                 110

Ser Thr Ser Gly Ser Gly Lys Pro Gly Ser Gly Glu Gly Ser Thr Lys
        115                 120                 125

Gly Gln Val Gln Leu Val Gln Ser Gly Ser Glu Leu Lys Lys Pro Gly
    130                 135                 140

Ala Ser Val Lys Val Ser Cys Lys Ala Ser Gly Tyr Thr Phe Thr Asp
145                 150                 155                 160

Tyr Ser Ile Asn Trp Val Arg Gln Ala Pro Gly Gln Gly Leu Glu Trp
                165                 170                 175

Met Gly Trp Ile Asn Thr Glu Thr Arg Glu Pro Ala Tyr Ala Tyr Asp
            180                 185                 190

Phe Arg Gly Arg Phe Val Phe Ser Leu Asp Thr Ser Val Ser Thr Ala
        195                 200                 205

Tyr Leu Gln Ile Ser Ser Leu Lys Ala Glu Asp Thr Ala Val Tyr Tyr
    210                 215                 220

Cys Ala Arg Asp Tyr Ser Tyr Ala Met Asp Tyr Trp Gly Gln Gly Thr
225                 230                 235                 240

Leu Val Thr Val Ser Ser Thr Thr Thr Pro Ala Pro Arg Pro Pro Thr
                245                 250                 255

Pro Ala Pro Thr Ile Ala Ser Gln Pro Leu Ser Leu Arg Pro Glu Ala
            260                 265                 270

Cys Arg Pro Ala Ala Gly Gly Ala Val His Thr Arg Gly Leu Asp Phe
        275                 280                 285

Ala Cys Asp Ile Tyr Ile Trp Ala Pro Leu Ala Gly Thr Cys Gly Val

290                 295                 300

Leu Leu Leu Ser Leu Val Ile Thr Leu Tyr Cys Lys Arg Gly Arg Lys
305                 310                 315                 320

Lys Leu Leu Tyr Ile Phe Lys Gln Pro Phe Met Arg Pro Val Gln Thr
                325                 330                 335

Thr Gln Glu Glu Asp Gly Cys Ser Cys Arg Phe Pro Glu Glu Glu Glu
            340                 345                 350

Gly Gly Cys Glu Leu Arg Val Lys Phe Ser Arg Ser Ala Asp Ala Pro
        355                 360                 365

Ala Tyr Gln Gln Gly Gln Asn Gln Leu Tyr Asn Glu Leu Asn Leu Gly
    370                 375                 380

Arg Arg Glu Glu Tyr Asp Val Leu Asp Lys Arg Arg Gly Arg Asp Pro
385                 390                 395                 400

Glu Met Gly Gly Lys Pro Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465

<210>  25
<211>  789
<212>  DNA
<213>  Artificial Sequence

<220>
<223>  encoding GPC3 scFv

<400>  25
atggctctgc ctgtgaccgc tctgctgctg cctctggctc tgctgctgca cgccgcaaga       60

cctgatgtcg tgatgaccca gtccccactg tccctgccag tgacaccagg agagcctgca      120

tccatctctt gccggagctc ccagtctctg gtgcacagca acggcaatac ctacctgcac      180

tggtatctgc agaagccagg ccagagcccc cagctgctga tctacaaggt gtccaaccgg      240

ttctctggag tgccagaccg gttcagcggc tccggctctg gcaccgattt cacactgaag 300

atcagcaggg tggaggcaga ggacgtgggc gtgtactatt gctcccagaa tacccacgtg 360

ccccctacat ttggccaggg caccaagctg gagatcaagg gaggaggagg cagcggcgga 420

ggaggctccg gcggcggcgg ctctcaggtg cagctggtgc agtccggagc agaggtgaag 480

aagcctggag ccagcgtgaa ggtgtcctgt aaggcctctg gctacacctt cacagattat 540

gagatgcact gggtgcggca ggcacctgga cagggactgg agtggatggg cgccctggac 600

ccaaagaccg gcgatacagc ctactctcag aagtttaagg gcagggtgac cctgacagcc 660

gacgagagca cctccacagc ctatatggag ctgtctagcc tgcgcagcga ggataccgcc 720

gtgtactatt gcacccgctt ctacagttac acttattggg ggcagggcac tctggtcaca 780

gtctcttca 789

<210> 26
<211> 726
<212> DNA
<213> Artificial Sequence

<220>
<223> encoding CD19 scFv

<400> 26
gacatccaga tgacacagac tacatcctcc ctgtctgcct ctctgggaga cagagtcacc 60

atcagttgca gggcaagtca ggacattagt aaatatttaa attggtatca gcagaaacca 120

gatggaactg ttaaactcct gatctaccat acatcaagat tacactcagg agtcccatca 180

aggttcagtg gcagtgggtc tggaacagat tattctctca ccattagcaa cctggagcaa 240

gaagatattg ccacttactt ttgccaacag ggtaatacgc ttccgtacac gttcggaggg 300

gggaccaagc tggagatcac aggtggcggt ggctcgggcg gtggtgggtc gggtggcggc 360

ggatctgagg tgaaactgca ggagtcagga cctggcctgg tggcgccctc acagagcctg 420

tccgtcacat gcactgtctc aggggtctca ttacccgact atggtgtaag ctggattcgc 480

cagcctccac gaaagggtct ggagtggctg ggagtaatat ggggtagtga aaccacatac 540

tataattcag ctctcaaatc cagactgacc atcatcaagg acaactccaa gagccaagtt 600

ttcttaaaaa tgaacagtct gcaaactgat gacacagcca tttactactg tgccaaacat 660

tattactacg gtggtagcta tgctatggac tactggggcc aaggaacctc agtcaccgtc 720

tcctca 726

<210> 27
<211> 738
<212> DNA
<213> Artificial Sequence

<220>
<223>  encoding BCMA scFv

<400>  27
gacatcgtgc tgacccagag ccccgcttct ttagctgtgt ctttaggaga gagggccacc      60

atcaattgtc gtgcctccga gtccgtgtcc gtgattggcg cccatttaat ccactggtac     120

cagcagaaac ccggccagcc ccccaagctg ctgatctatt tagccagcaa tctggagacc     180

ggcgtgcccg ccagatttag cggaagcggc tccggcaccg attttacttt aaccatttcc     240

tctttacaag ctgaggacgc cgccatctac tactgtttac agagcagaat ctttcctcgt     300

accttcggcc aaggtaccaa gctggaaatc aagggatcca ccagcggctc cggaaagccc     360

ggttccggag agggcagcac aaagggccaa gttcagctcg tgcagagcgg ctccgagctg     420

aagaagcccg gcgcttccgt gaaggtgagc tgcaaggcca gcggatacac cttcaccgac     480

tactccatca actgggttcg tcaagctccc ggtcaaggtc tggagtggat gggctggatc     540

aacaccgaaa ccagagagcc cgcctatgcc tacgacttca gaggtcgttt cgtgttctct     600

ttagacacaa gcgtgagcac cgcctattta cagatcagct ctttaaaggc cgaggatacc     660

gccgtgtact actgcgctcg tgactacagc tacgccatgg actactgggg ccaaggtaca     720

ctggtgaccg tgtccagc                                                   738

<210>  28
<211>  777
<212>  DNA
<213>  Artificial Sequence

<220>
<223>  encoding MSLN scFv

<400>  28
caggtgcagc tgcaacagag cggccctggc ctggtgacac cttcccagac cctcagcctc      60

acctgcgcca tctccggcga ttccgtgtcc agcaatagcg ccacctggaa ctggatcaga     120

cagagcccta gcagaggcct ggagtggctg ggcaggacct attataggag caagtggtac     180

aacgactacg ccgtgtccgt caagtccaga atgagcatca accccgacac cagcaagaat     240

cagttcagcc tgcagctgaa cagcgtgacc cccgaggaca cagccgtgta ttactgcgcc     300

aggggcatga tgacctacta ctacggcatg gacgtgtggg gccaaggcac cacagtgacc     360

gtgagcagcg gcattctggg cagcggagga ggcggatccg gcggcggagg cagcggagga     420

ggaggcagcc agcctgtgct gacccagtcc agctccctgt ccgcttcccc tggagcctcc     480

gcttccctga cctgcaccct gaggtccggc atcaatgtgg gcccctacag gatctactgg     540

tatcagcaga agcctggcag ccccccccag tacctgctga actacaagag cgacagcgac     600

aagcagcagg gcagcggagt gcctagcaga ttcagcggat ccaaggacgc ctccgccaat     660

gctggcgtgc tgctgatctc cggcctgaga agcgaggacg aagccgacta ctactgcatg     720

atctggcaca gcagcgctgc cgtgtttgga ggcggcaccc aactgacagt cctgtcc 777

<210> 29
<211> 723
<212> DNA
<213> Artificial Sequence

<220>
<223> encoding HER2 scFv

<400> 29
caggtccagc tgcagcagag cggacccgag ctcaagaagc ccggcgagac cgtgaagatc 60

agctgtaagg ccagcggcta cccctttacc aactatggaa tgaactgggt gaagcaagct 120

cccggccagg gcctgaagtg gatgggctgg atcaacacaa gcaccggaga aagcaccttc 180

gctgacgact tcaagggcag atttgacttc tccctcgaga cctccgccaa caccgcctac 240

ctgcagatca acaatctgaa gtccgaggac agcgctacct acttctgcgc cagatgggag 300

gtgtaccatg gctacgtccc ttattggggc caaggcacca ccgtgaccgt gagcagcgga 360

ggaggcggtt ctggaggcgg cggcagcggc ggcggcggca gcgacattca gctgacccag 420

tcccacaagt tcctgagcac ctccgtgggc gacagggtga gcattacctg caaggccagc 480

caggacgtgt acaatgccgt ggcctggtac cagcagaagc ctggacagag ccccaagctg 540

ctgatctaca gcgcttcctc caggtacacc ggcgtccctt ccagatttac cggctccggc 600

tccggccccg acttcacctt caccatcagc tccgtgcagg ccgaagacct ggccgtgtac 660

ttctgccaac agcacttcag gacccccttc acctttggca gcggaaccaa gctggagatc 720

aaa 723

<210> 30
<211> 138
<212> DNA
<213> Artificial Sequence

<220>
<223> encoding CD8 hinge region

<400> 30
accacgacgc cagcgccgcg accaccaaca ccggcgccca ccatcgcgtc gcagcccctg 60

tccctgcgcc cagaggcgtg ccggccagcg gcggggggcg cagtgcacac gagggggctg 120

gacttcgcct gtgatatc 138

<210> 31
<211> 69
<212> DNA
<213> Artificial Sequence

<220>
<223> encoding CD8 transmembrane domain

<400>  31
tacatctggg cgcccttggc cgggacttgt ggggtccttc tcctgtcact ggttatcacc      60

ctttactgc                                                              69


<210>  32
<211>  123
<212>  DNA
<213>  Artificial Sequence

<220>
<223>  encoding CD28 intracellular domain

<400>  32
aggagtaaga ggagcaggct cctgcacagt gactacatga acatgactcc ccgccgcccc      60

gggcccaccc gcaagcatta ccagccctat gccccaccac gcgacttcgc agcctatcgc     120

tcc                                                                   123


<210>  33
<211>  126
<212>  DNA
<213>  Artificial Sequence

<220>
<223>  encoding 41BB intracellular domain

<400>  33
aaacggggca gaaagaaact cctgtatata ttcaaacaac catttatgag accagtacaa      60

actactcaag aggaagatgg ctgtagctgc cgatttccag aagaagaaga aggaggatgt     120

gaactg                                                                126


<210>  34
<211>  339
<212>  DNA
<213>  Artificial Sequence

<220>
<223>  encoding CD3zeta intracellular domain

<400>  34
agagtgaagt tcagcaggag cgcagacgcc cccgcgtacc agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgctaa                            339


<210>  35
<211>  54

<212>  DNA
<213>  Artificial Sequence

<220>
<223>  encoding T2A

<400>  35
gagggcagag gaagtcttct aacatgcggt gacgtggagg agaatcccgg ccct          54


<210>  36
<211>  57
<212>  DNA
<213>  Artificial Sequence

<220>
<223>  encoding P2A

<400>  36
gccaccaatt ttagcctgct gaaacaggcc ggagacgtgg aggaaaaccc cggcccc       57


<210>  37
<211>  501
<212>  DNA
<213>  Artificial Sequence

<220>
<223>  encoding secretory leptin

<400>  37
atgcattggg gaaccctgtg cggattcttg tggctttggc cctatctttt ctatgtccaa      60

gctgtgccca tccaaaaagt ccaagatgac accaaaaccc tcatcaagac aattgtcacc     120

aggatcaatg acatttcaca cacgcagtca gtctcctcca aacagaaagt caccggtttg     180

gacttcattc ctgggctcca ccccatcctg accttatcca agatggacca gacactggca     240

gtctaccaac agatcctcac cagtatgcct tccagaaacg tgatccaaat atccaacgac     300

ctggagaacc tccgggatct tcttcacgtg ctggccttct ctaagagctg ccacttgccc     360

tgggccagtg gcctggagac cttggacagc ctggggggtg tcctggaagc ttcaggctac     420

tccacagagg tggtggccct gagcaggctg caggggtctc tgcaggacat gctgtggcag     480

ctggacctca gccctgggtg c                                               501


<210>  38
<211>  84
<212>  DNA
<213>  Artificial Sequence

<220>
<223>  encoding transmembrane domain of leptin receptor

<400>  38
gatgcaggtt tatatgtaat tgtgccagta attatttcct cttccatctt attgcttgga      60

acattattaa tatcacacca aaga                                             84

<210> 39
<211> 63
<212> DNA
<213> Artificial Sequence

<220>
<223> encoding signal peptide of leptin receptor

<400> 39
atgatttgtc aaaaattctg tgtggttttg ttacattggg aatttattta tgtgataact 60

gcg 63


<210> 40
<211> 1593
<212> DNA
<213> Artificial Sequence

<220>
<223> encoding leptin receptor

<400> 40
ataaaagttc ctatgagagg acctgaattt tggagaataa ttaatggaga tactatgaaa 60

aaggagaaaa atgtcacttt actttggaag cccctgatga aaaatgactc attgtgcagt 120

gttcagagat atgtgataaa ccatcatact tcctgcaatg gaacatggtc agaagatgtg 180

ggaaatcaca cgaaattcac tttcctgtgg acagagcaag cacatactgt tacggttctg 240

gccatcaatt caattggtgc ttctgttgca aattttaatt taaccttttc atggcctatg 300

agcaaagtaa atatcgtgca gtcactcagt gcttatcctt taaacagcag ttgtgtgatt 360

gtttcctgga tactatcacc cagtgattac aagctaatgt attttattat tgagtggaaa 420

aatcttaatg aagatggtga aataaaatgg cttagaatct cttcatctgt taagaagtat 480

tatatccatg atcattttat ccccattgag aagtaccagt tcagtcttta cccaatattt 540

atggaaggag tgggaaaacc aaagataatt aatagtttca ctcaagatga tattgaaaaa 600

caccagagtg atgcaggttt atatgtaatt gtgccagtaa ttatttcctc ttccatctta 660

ttgcttggaa cattattaat atcacaccaa agaatgaaaa agctattttg ggaagatgtt 720

ccgaacccca agaattgttc ctgggcacaa ggacttaatt ttcagaagcc agaaacgttt 780

gagcatcttt ttatcaagca tacagcatca gtgacatgtg gtcctcttct tttggagcct 840

gaaacaattt cagaagatat cagtgttgat acatcatgga aaaataaaga tgagatgatg 900

ccaacaactg tggtctctct actttcaaca acagatcttg aaaagggttc tgtttgtatt 960

agtgaccagt tcaacagtgt taacttctct gaggctgagg gtactgaggt aacctatgag 1020

gacgaaagcc agagacaacc ctttgttaaa tacgccacgc tgatcagcaa ctctaaacca 1080

agtgaaactg gtgaagaaca agggcttata aatagttcag tcaccaagtg cttctctagc 1140

aaaaattctc cgttgaagga ttctttctct aatagctcat gggagataga ggcccaggca 1200

ttttttatat tatcagatca gcatcccaac ataatttcac cacacctcac attctcagaa 1260

ggattggatg aacttttgaa attggaggga aatttccctg aagaaaataa tgataaaaag 1320

tctatctatt atttaggggt cacctcaatc aaaaagagag agagtggtgt gcttttgact 1380

gacaagtcaa gggtatcgtg cccattccca gccccctgtt tattcacgga catcagagtt 1440

ctccaggaca gttgctcaca ctttgtagaa aataatatca acttaggaac ttctagtaag 1500

aagacttttg catcttacat gcctcaattc caaacttgtt ctactcagac tcataagatc 1560

atggaaaaca agatgtgtga cctaactgtg taa 1593

<210> 41
<211> 1167
<212> DNA
<213> Artificial Sequence

<220>
<223> encoding LRP6

<400> 41
   atgggggccg tcctgaggag cctcctggcc tgcagcttct gtgtgctcct gagagcggaa 60
   cctccaacat gttctcctca gcagtttact tgtttcacgg gggaaattga ctgtatccct 120
   gtggcttggc ggtgcgatgg gtttactgaa tgtgaagacc acagtgatga actcaattgt 180
   cctgtatgct cagagtccca gttccagtgt gccagtgggc agtgtattga tggtgccctc 240
   cgatgcaatg gagatgcaaa ctgccaggac aaatcagatg agaagaactg tgaagtgctt 300
   tgtttaattg atcagttccg ctgtgccaat ggtcagtgca ttggaaagca caagaagtgt 360
   gatcataatg tggattgcag tgacaagtca gatgaactgg attgttatcc gactgaagaa 420
   ccagcaccac aggccaccaa tacagttggt tctgttattg gcgtaattgt caccattttt 480
   gtgtctggaa ctgtatactt tatctgccag aggatgttgt gtccacgtat gaagggagat 540
   ggggaaacta tgactaatga ctatgtagtt catggaccag cttctgtgcc tcttggttat 600
   gtgccacacc caagttcttt gtcaggatct cttccaggaa tgtctcgagg taaatcaatg 660
   atcagctccc tcagtatcat ggggggaagc agtggacccc cctatgaccg agcccatgtt 720
   acaggagcat catcaagtag ttcttcaagc accaaaggca cttacttccc tgcaattttg 780
   aaccctccac catccccagc cacagagcga tcacattaca ctatggaatt tggatattct 840
   tcaaacagtc cttccactca taggtcatac agctacaggc catatagcta ccggcacttt 900
   gcacccccca ccacaccctg cagcacagat gtttgtgaca gtgactatgc tcctagtcgg 960
   agaatgacct cagtggcaac agccaagggc tataccagtg acttgaacta tgattcagaa 1020
   cctgtgcccc cacctcccac accccgaagc caatacttgt cagcagagga gaactatgaa 1080
   agctgcccac cttctccata cacagagagg agctattctc atcacctcta cccaccgcca 1140
   ccctctccct gtacagactc ctcctga                                          1167

<210>  42
<211>  804
<212>  DNA
<213>  Artificial Sequence

<220>
<223>  encoding TL6

<400>  42
atgggggccg tcctgaggag cctcctggcc tgcagcttct gtgtgctcct gagagcgcca      60

gcaccacagg ccaccaatac agttggttct gttattggcg taattgtcac catttttgtg     120

tctggaactg tatactttat ctgccagagg atgttgtgtc cacgtatgaa gggagatggg     180

gaaactatga ctaatgacta tgtagttcat ggaccagctt ctgtgcctct tggttatgtg     240

ccacacccaa gttctttgtc aggatctctt ccaggaatgt ctcgaggtaa atcaatgatc     300

agctccctca gtatcatggg gggaagcagt ggacccccct atgaccgagc ccatgttaca     360

ggagcatcat caagtagttc ttcaagcacc aaaggcactt acttccctgc aattttgaac     420

cctccaccat ccccagccac agagcgatca cattacacta tggaatttgg atattcttca     480

aacagtcctt ccactcatag gtcatacagc tacaggccat atagctaccg gcactttgca     540

ccccccacca caccctgcag cacagatgtt tgtgacagtg actatgctcc tagtcggaga     600

atgacctcag tggcaacagc caagggctat accagtgact tgaactatga ttcagaacct     660

gtgcccccac ctcccacacc ccgaagccaa tacttgtcag cagaggagaa ctatgaaagc     720

tgcccacctt ctccatacac agagaggagc tattctcatc acctctaccc accgccaccc     780

tctccctgta cagactcctc ctga                                            804

<210>  43
<211>  1089
<212>  DNA
<213>  Artificial Sequence

<220>
<223>  encoding LRP5

<400>  43
ggagagccgc ccacctgctc cccggaccag tttgcatgtg ccacagggga gatcgactgt      60

atccccgggg cctggcgctg tgacggcttt cccgagtgcg atgaccagag cgacgaggag     120

ggctgccccg tgtgctccgc cgcccagttc ccctgcgcgc ggggtcagtg tgtggacctg     180

cgcctgcgct gcgacggcga ggcagactgt caggaccgct cagacgaggc ggactgtgac     240

gccatctgcc tgcccaacca gttccggtgt gcgagcggcc agtgtgtcct catcaaacag     300

cagtgcgact ccttccccga ctgtatcgac ggctccgacg agctcatgtg tgaaatcacc     360

aagccgccct cagacgacag cccggcccac agcagtgcca tcgggcccgt cattggcatc     420

atcctctctc tcttcgtcat gggtggtgtc tattttgtgt gccagcgcgt ggtgtgccag 480

cgctatgcgg gggccaacgg gcccttcccg cacgagtatg tcagcgggac cccgcacgtg 540

cccctcaatt tcatagcccc gggcggttcc cagcatggcc ccttcacagg catcgcatgc 600

ggaaagtcca tgatgagctc cgtgagcctg atggggggcc ggggcggggt gcccctctac 660

gaccggaacc acgtcacagg ggcctcgtcc agcagctcgt ccagcacgaa ggccacgctg 720

tacccgccga tcctgaaccc gccgccctcc ccggccacgg acccctccct gtacaacatg 780

gacatgttct actcttcaaa cattccggcc actgcgagac cgtacaggcc ctacatcatt 840

cgaggaatgg cgcccccgac gacgccctgc agcaccgacg tgtgtgacag cgactacagc 900

gccagccgct ggaaggccag caagtactac ctggatttga actcggactc agacccctat 960

ccacccccac ccacgcccca cagccagtac ctgtcggcgg aggacagctg cccgccctcg 1020

cccgccaccg agaggagcta cttccatctc ttcccgcccc ctccgtcccc ctgcacggac 1080

tcatcctga 1089

<210> 44
<211> 696
<212> DNA
<213> Artificial Sequence

<220>
<223> encoding TL5

<400> 44
agtgccatcg ggcccgtcat tggcatcatc ctctctctct tcgtcatggg tggtgtctat 60

tttgtgtgcc agcgcgtggt gtgccagcgc tatgcggggg ccaacgggcc cttcccgcac 120

gagtatgtca gcgggacccc gcacgtgccc ctcaatttca tagccccggg cggttcccag 180

catggcccct tcacaggcat cgcatgcgga aagtccatga tgagctccgt gagcctgatg 240

gggggccggg gcggggtgcc cctctacgac cggaaccacg tcacaggggc ctcgtccagc 300

agctcgtcca gcacgaaggc cacgctgtac ccgccgatcc tgaacccgcc gccctccccg 360

gccacggacc cctccctgta caacatggac atgttctact cttcaaacat tccggccact 420

gcgagaccgt acaggcccta catcattcga ggaatggcgc ccccgacgac gccctgcagc 480

accgacgtgt gtgacagcga ctacagcgcc agccgctgga aggccagcaa gtactacctg 540

gatttgaact cggactcaga cccctatcca cccccaccca cgccccacag ccagtacctg 600

tcggcggagg acagctgccc gccctcgccc gccaccgaga ggagctactt ccatctcttc 660

ccgccccctc cgtccccctg cacggactca tcctga 696

<210> 45
<211> 78
<212> DNA

<213>  Artificial Sequence

<220>
<223>  encoding linker SG3(SG4)3SG3SLQ

<400>  45
agtggagggg gatctggggg cggggggtct ggaggaggtg ggagtggggg gggaggctcc      60

ggaggcggct ccctccaa                                                   78


<210>  46
<211>  578
<212>  DNA
<213>  Artificial Sequence

<220>
<223>  IRES

<400>  46
tccctccccc ccccctaacg ttactggccg aagccgcttg gaataaggcc ggtgtgcgtt      60

tgtctatatg ttattttcca ccatattgcc gtcttttggc aatgtgaggg cccggaaacc     120

tggccctgtc ttcttgacga gcattcctag gggtctttcc cctctcgcca aaggaatgca     180

aggtctgttg aatgtcgtga aggaagcagt tcctctggaa gcttcttgaa gacaaacaac     240

gtctgtagcg accctttgca ggcagcggaa ccccccacct ggcgacaggt gcctctgcgg     300

ccaaaagcca cgtgtataag atacacctgc aaaggcggca caaccccagt gccacgttgt     360

gagttggata gttgtggaaa gagtcaaatg gctctcctca agcgtattca acaaggggct     420

gaaggatgcc cagaaggtac cccattgtat gggatctgat ctggggcctc ggtgcacatg     480

ctttacatgt gtttagtcga ggttaaaaaa acgtctaggc cccccgaacc acggggacgt     540

ggttttcctt tgaaaaacac gatgataata tggccaca                             578

<210>  47
<211>  263
<212>  PRT
<213>  Artificial Sequence

<220>
<223>  L1H2 scFv

<400>  47

Met Ala Leu Pro Val Thr Ala Leu Leu Leu Pro Leu Ala Leu Leu Leu
1               5                   10                  15


His Ala Ala Arg Pro Asp Val Val Met Thr Gln Ser Pro Leu Ser Leu
            20                  25                  30


Pro Val Thr Leu Gly Gln Pro Ala Ser Ile Ser Cys Arg Ser Ser Gln
        35                  40                  45


Ser Leu Val His Ser Asn Gly Tyr Thr Tyr Leu His Trp Tyr Gln Gln

50                  55                  60

Arg Pro Gly Gln Ser Pro Arg Leu Leu Ile Tyr Lys Val Ser Tyr Arg
65                  70                  75                  80

Gly Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Gly Gln Ser Gly Leu Thr Pro Pro Thr Phe Gly Ser Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys
145                 150                 155                 160

Lys Pro Gly Ala Ser Val Lys Val Ser Cys Lys Ala Ser Gly Tyr Asp
                165                 170                 175

Phe Thr Ala Tyr Glu Met His Trp Val Arg Gln Ala Pro Gly Gln Gly
            180                 185                 190

Leu Glu Trp Met Gly Ala Leu Asp Pro Lys Thr Gly Gln Thr Ala Tyr
        195                 200                 205

Ser Gln Lys Phe Gln Gly Arg Val Thr Leu Thr Ala Asp Lys Ser Ile
    210                 215                 220

Ser Thr Ala Tyr Met Glu Leu Ser Arg Leu Arg Ser Asp Asp Thr Val
225                 230                 235                 240

Val Tyr Tyr Cys Thr Arg Phe Tyr Ser Tyr Ala Tyr Trp Gly Gln Gly
                245                 250                 255

Thr Leu Val Thr Val Ser Ser
            260

<210>  48
<211>  789
<212>  DNA
<213>  Artificial Sequence

<220>
<223>  encoding L1H2 scFv

<400>  48
atggctctgc ctgtgaccgc tctgctgctg cctctggctc tgctgctgca cgccgcaaga      60

cctgacgtgg tgatgaccca gtcccctctg tctttacccg ttacactggg ccagcccgct     120

tccatcagct gcagaagcag ccagtcttta gtgcacagca acggctacac ctatttacac     180

tggtaccagc agagacccgg ccagagcccc agactgctga tctacaaggt gtcataccgt     240

ggatccggcg tgcccgatag gtttagcggc agcggtagcg gcaccgattt cactttaaag     300

atctctcgtg tcgaggccga ggatgtgggc gtttactact gcggccagag cggcctcacc     360

ccccctacct tcggctccgg caccaagctg gagatcaaag gaggcggagg atctggagga     420

ggagggtctg gaggcggcgg gagccaagtg cagctggtgc agagcggcgc cgaggtgaag     480

aaacccggag ccagcgtgaa ggtgagctgt aaggccagcg gctatgactt caccgcctac     540

gagatgcact gggtgagaca agctcccggt caaggtctcg agtggatggg cgctttagat     600

cccaagaccg ggcagaccgc ctactcccag aagttccaag gtcgtgtgac tttaaccgcc     660

gacaagagca tctccaccgc ctacatggag ctgtctcgtc tgaggagcga cgataccgtg     720

gtgtactatt gcactcgttt ttactcctac gcctactggg gccaaggtac actggtgacc     780

gtgagctcc                                                             789

## Description

### Field of the Invention

The present application relates to the field of biomedicine, and specifically to a modified immune cell and a use thereof.

### Background of the Invention

Tumor is a disease that seriously threatens human health. In recent years, with the progress of molecular biology, genomics and proteomics, a series of molecularly targeted drugs have come out one after another for the treatment of tumors. While with the rapid development of immunology, based on the discovery that the recognition specificity of cytotoxic lymphocytes (CTLs) to target cells depends on the T lymphocyte receptor (TCR), further researches have been conducted, that is, the scFv of antibodies against tumor cell-associated antigens is fused with the T lymphocyte receptor to form a chimeric antigen receptor (CAR), which is genetically modified on the surface of T lymphocytes by means of lentiviral infection for the treatment of tumor. Such CAR-T lymphocytes can selectively target T lymphocytes to tumor cells and specifically kill tumor cells in a non-limiting manner with the major histocompatibility complex (MHC). However, adoptive immunotherapy based on immune effector cells has achieved certain effects in some tumors, but the efficacy in most tumors is still unsatisfactory.

### Summary of the Invention

The present application provides a modified immune cell, which includes a chimeric antigen receptor and/or a coding element therefor, or including a T cell receptor and/or a coding element therefor, and the modified immune cell further includes:
a leptin and/or a functional fragment thereof; and/or,
a leptin receptor and/or a functional fragment thereof, and the expression quantity of the leptin receptor and/or the functional fragment thereof in the modified immune cell is increased compared with that in an immune cell without the corresponding modification.

In some embodiments, the modified immune cell includes lymphocytes.

In some embodiments, the modified immune cell includes T cells.

In some embodiments, the leptin and/or the functional fragment thereof is derived from human.

In some embodiments, the leptin receptor and/or the functional fragment thereof is derived from human.

In some embodiments, the leptin is secretory leptin or membrane-bound leptin.

In some embodiments, the leptin and/or the functional fragment thereof includes an amino acid sequence as set forth in any one of SEQ ID NOs: 13-14.

In some embodiments, the functional fragment of the leptin receptor includes a domain of the leptin receptor selected from a group consisting of: an intracellular region, a BOX 1 region, an FN3 region, a C2 region and a transmembrane region.

In some embodiments, the leptin receptor and/or the functional fragment thereof includes an amino acid sequence as set forth in any one of SEQ ID NOs: 15-17.

In some embodiments, the chimeric antigen receptor does not include the leptin and/or the functional fragment thereof.

In some embodiments, the chimeric antigen receptor does not include the leptin receptor and/or the functional fragment thereof.

In some embodiments, the chimeric antigen receptor includes an intracellular domain, the intracellular domain includes a signaling domain and/or a costimulatory domain.

In some embodiments, the signaling domain includes a signaling domain of CD3zeta.

In some embodiments, the costimulatory domain includes a costimulatory domain of 4-1BB. In some embodiments, the chimeric antigen receptor includes a hinge region.

In some embodiments, the hinge region includes a hinge region of CD8.

In some embodiments, the chimeric antigen receptor includes a transmembrane region.

In some embodiments, the transmembrane region includes a transmembrane region of CD8.

In some embodiments, the chimeric antigen receptor includes a targeting moiety.

In some embodiments, the targeting moiety includes scFv.

In some embodiments, the targeting moiety specifically binds to and/or recognizes a tumor antigen.

In some embodiments, the targeting moiety specifically binds to and/or recognizes a target selected from a group consisting of: CD19, HER2, Mesothelin, GPC3 and BCMA.

In some embodiments, the chimeric antigen receptor includes an amino acid sequence as set forth in any one of SEQ ID NOs: 23-24.

In another aspect, the present application also provides a pharmaceutical composition, which includes the modified immune cell of the present application and optionally a pharmaceutically acceptable carrier.

In another aspect, the present application also provides a use of the modified immune cell of the present application or the pharmaceutical composition of the present application in the preparation of a drug for treating tumor.

In another aspect, the present application also provides a method for promoting immune cell proliferation, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In another aspect, the present application also provides a method for promoting the production of memory immune cells, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In another aspect, the present application also provides a method for enhancing the killing ability of an immune cell against tumor, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In another aspect, the present application also provides a method for inhibiting an immune cell from expressing an immune negative regulatory protein, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In some embodiments, the step 2) in the method of the present application includes:
introducing a polynucleotide encoding the leptin and/or the functional fragment thereof into the modified immune cell, and/or introducing a polynucleotide encoding the leptin receptor and/or the functional fragment thereof into the modified immune cell.

In some embodiments, the polynucleotide encoding the leptin and/or the functional fragment thereof, and/or the polynucleotide encoding the leptin receptor and/or the functional fragment thereof is contained in a plasmid.

In some embodiments, the plasmid includes a viral vector.

In some embodiments, the modified immune cell includes T cells.

In some embodiments, the increased expression quantity includes that, the expression quantity of the leptin receptor and/or the functional fragment thereof in the modified immune cell is increased compared with that in an immune cell without the corresponding modification.

In some embodiments, the leptin and/or the functional fragment thereof is derived from human.

In some embodiments, the leptin receptor and/or the functional fragment thereof is derived from human.

In some embodiments, the leptin is secretory leptin or membrane-bound leptin.

In some embodiments, the leptin and/or the functional fragment thereof includes an amino acid sequence as set forth in any one of SEQ ID NOs: 13-14.

In some embodiments, the functional fragment of the leptin receptor includes a domain of the leptin receptor selected from a group consisting of: an intracellular region, a BOX 1 region, an FN3 region, a C2 region and a transmembrane region.

In some embodiments, the leptin receptor and/or the functional fragment thereof includes an amino acid sequence as set forth in any one of SEQ ID NOs: 15-17.

In some embodiments, the chimeric antigen receptor does not include the leptin and/or the functional fragment thereof and/or the leptin receptor and/or the functional fragment thereof.

In some embodiments, the chimeric antigen receptor includes an intracellular domain, the intracellular domain includes a signaling domain and/or a costimulatory domain.

In some embodiments, the signaling domain includes a signaling domain of CD3zeta.

In some embodiments, the costimulatory domain includes a costimulatory domain of 4-1BB.

In some embodiments, the chimeric antigen receptor includes a hinge region.

In some embodiments, the hinge region includes a hinge region of CD8.

In some embodiments, the chimeric antigen receptor includes a transmembrane region.

In some embodiments, the transmembrane region includes a transmembrane region of CD8.

In some embodiments, the chimeric antigen receptor includes a targeting moiety.

In some embodiments, the targeting moiety includes scFv.

In some embodiments, the targeting moiety specifically binds to and/or recognizes a tumor antigen.

In some embodiments, the targeting moiety specifically binds to and/or recognizes a target selected from a group consisting of: CD19, HER2, Mesothelin, GPC3 and BCMA.

In some embodiments, the chimeric antigen receptor includes an amino acid sequence as set forth in any one of SEQ ID NOs: 23-24.

In another aspect, the present application also provides a method for inhibiting tumor cell proliferation and/or killing tumor cell, which includes: contacting the modified immune cell of the present application with the tumor cell.

In some embodiments, the method includes an in vitro method or an ex vivo method.

In some embodiments, the tumor includes solid tumor and/or non-solid tumor.

In some embodiments, the tumor includes B lymphocytic leukemia, breast cancer, stomach cancer, pancreatic cancer, multiple myeloma, mesothelioma, lung cancer and/or liver cancer.

In another aspect, the present application provides another modified immune cell, which includes leptin and/or a functional fragment thereof, and/or, a leptin receptor and/or a functional fragment thereof, and a low-density lipoprotein-receptor-related protein or a fragment thereof.

In some embodiments, the modified immune cell includes a T cell.

In some embodiments, the leptin and/or the functional fragment thereof is derived from human.

In some embodiments, the leptin receptor and/or the functional fragment thereof is derived from human.

In some embodiments, the leptin is secretory leptin or membrane-bound leptin.

In some embodiments, the leptin and/or the functional fragment thereof includes an amino acid sequence as set forth in any one of SEQ ID NOs: 13-14.

In some embodiments, the functional fragment of the leptin receptor includes a domain of the leptin receptor selected from a group consisting of: an intracellular region, a BOX 1 region, an FN3 region, a C2 region and a transmembrane region.

In some embodiments, the leptin receptor and/or the functional fragment thereof includes an amino acid sequence as set forth in any one of SEQ ID NOs: 15-17.

In some embodiments, the low-density lipoprotein-receptor-related protein or the fragment thereof is selected from a group consisting of: a low-density lipoprotein-receptor-related protein 6 or a truncated body thereof, and a low-density lipoprotein-receptor-related protein 5 or a truncated body thereof.

In some embodiments, the truncated body of the low-density lipoprotein-receptor-related protein 6 includes an intracellular region of the low-density lipoprotein-receptor-related protein 6; and/or, the truncated body of the low-density lipoprotein-receptor-related protein 5 includes an intracellular region of the low-density lipoprotein-receptor-related protein 5.

In some embodiments, the truncated body of the low-density lipoprotein-receptor-related protein 6 includes a functional domain selected from a group consisting of: a transmembrane region of the low-density lipoprotein-receptor-related protein 6 and an LDLR region of the low-density lipoprotein-receptor-related protein 6; and/or, wherein the truncated body of the low-density lipoprotein-receptor-related protein 5 includes a functional domain selected from a group consisting of: a transmembrane region of the low-density lipoprotein-receptor-related protein 5 and an LDLR region of the low-density lipoprotein-receptor-related protein 5.

In some embodiments, the low-density lipoprotein-receptor-related protein or the fragment thereof includes an amino acid sequence as set forth in any one of SEQ ID NOs: 18-21.

In some embodiments, the modified immune cell further includes a chimeric antigen receptor or a T cell receptor.

In some embodiments, the chimeric antigen receptor does not include the leptin and/or the functional fragment thereof.

In some embodiments, the chimeric antigen receptor does not include the leptin receptor and/or the functional fragment thereof.

In some embodiments, the chimeric antigen receptor does not include the low-density lipoprotein-receptor-related protein or the fragment thereof.

In some embodiments, the chimeric antigen receptor includes an intracellular domain, the intracellular domain includes a signaling domain and/or a costimulatory domain.

In some embodiments, the signaling domain includes a signaling domain of CD3zeta.

In some embodiments, the costimulatory domain includes a costimulatory domain of 4-1BB.

In some embodiments, the chimeric antigen receptor includes a hinge region.

In some embodiments, the hinge region includes a hinge region of CD8.

In some embodiments, the chimeric antigen receptor includes a transmembrane region.

In some embodiments, the transmembrane region includes a transmembrane region of CD8.

In some embodiments, the chimeric antigen receptor includes a targeting moiety.

In some embodiments, the targeting moiety includes scFv.

In some embodiments, the targeting moiety specifically binds to and/or recognizes a tumor antigen.

In some embodiments, the targeting moiety specifically binds to and/or recognizes a target selected from a group consisting of: CD19, HER2, Mesothelin, GPC3 and BCMA.

In some embodiments, the chimeric antigen receptor includes an amino acid sequence as set forth in any one of SEQ ID NOs: 23-24.

In another aspect, the present application also provides a pharmaceutical composition, which includes the modified immune cell of the present application and optionally a pharmaceutically acceptable carrier.

In another aspect, the present application also provides a use of the modified immune cell of the present application or the pharmaceutical composition of the present application in the preparation of a drug for treating tumor.

In another aspect, the present application also provides a method for promoting immune cell proliferation, which includes steps of:
allowing a modified immune cell to express leptin and/or a functional fragment thereof, and/or, increasing the expression quantity of a leptin receptor and/or a functional fragment thereof in the modified immune cell,
and allowing the modified immune cell to express a low-density lipoprotein-receptor-related protein or a fragment thereof.

In another aspect, the present application also provides a method for promoting the production of memory immune cells, which includes steps of:
allowing a modified immune cell to express leptin and/or a functional fragment thereof, and/or, increasing the expression quantity of a leptin receptor and/or a functional fragment thereof in the modified immune cell,
and allowing the modified immune cell to express a low-density lipoprotein-receptor-related protein or a fragment thereof.

In another aspect, the present application also provides a method for enhancing the killing ability of an immune cell against tumor, which includes steps of:
allowing a modified immune cell to express leptin and/or a functional fragment thereof, and/or, increasing the expression quantity of a leptin receptor and/or a functional fragment thereof in the modified immune cell,
and allowing the modified immune cell to express a low-density lipoprotein-receptor-related protein or a fragment thereof.

In another aspect, the present application also provides a method for inhibiting an immune cell from expressing an immune negative regulatory protein, which includes steps of:
allowing a modified immune cell to express leptin and/or a functional fragment thereof, and/or, increasing the expression quantity of a leptin receptor and/or a functional fragment thereof in the modified immune cell,
and allowing the modified immune cell to express a low-density lipoprotein-receptor-related protein or a fragment thereof.

In some embodiments, the methods of the present application include a step of: introducing a polynucleotide encoding the leptin and/or the functional fragment thereof into the modified immune cell, and/or introducing a polynucleotide encoding the leptin receptor and/or the functional fragment thereof into the modified immune cell.

In some embodiments, the methods of the present application include a step of: introducing a polynucleotide encoding the low-density lipoprotein-receptor-related protein or the fragment thereof into the modified immune cell.

In some embodiments, the polynucleotide encoding the leptin and/or the functional fragment thereof, and/or the polynucleotide encoding the leptin receptor and/or the functional fragment thereof is contained in a plasmid.

In some embodiments, the polynucleotide encoding the low-density lipoprotein-receptor-related protein or the fragment thereof is contained in a plasmid.

In some embodiments, the plasmid includes a viral vector.

In some embodiments, the modified immune cell includes T cells.

In some embodiments, the leptin and/or the functional fragment thereof is derived from human.

In some embodiments, the leptin receptor and/or the functional fragment thereof is derived from human.

In some embodiments, the leptin is secretory leptin or membrane-bound leptin.

In some embodiments, the leptin and/or the functional fragment thereof includes an amino acid sequence as set forth in any one of SEQ ID NOs: 13-14.

In some embodiments, the functional fragment of the leptin receptor includes a domain of the leptin receptor selected from a group consisting of: an intracellular region, a BOX 1 region, an FN3 region, a C2 region and a transmembrane region.

In some embodiments, the leptin receptor and/or the functional fragment thereof includes an amino acid sequence as set forth in any one of SEQ ID NOs: 15-17.

In some embodiments, the low-density lipoprotein-receptor-related protein or the fragment thereof is selected from a group consisting of: a low-density lipoprotein-receptor-related protein 6 or a truncated body thereof, and a low-density lipoprotein-receptor-related protein 5 or a truncated body thereof.

In some embodiments, the truncated body of the low-density lipoprotein-receptor-related protein 6 includes an intracellular region of the low-density lipoprotein-receptor-related protein 6; and/or, the truncated body of the low-density lipoprotein-receptor-related protein 5 includes an intracellular region of the low-density lipoprotein-receptor-related protein 5.

In some embodiments, the truncated body of the low-density lipoprotein-receptor-related protein 6 includes a functional domain selected from a group consisting of: a transmembrane region of the low-density lipoprotein-receptor-related protein 6 and an LDLR region of the low-density lipoprotein-receptor-related protein 6; and/or, wherein the truncated body of the low-density lipoprotein-receptor-related protein 5 includes a functional domain selected from a group consisting of: a transmembrane region of the low-density lipoprotein-receptor-related protein 5 and an LDLR region of the low-density lipoprotein-receptor-related protein 5.

In some embodiments, the low-density lipoprotein-receptor-related protein or the fragment thereof includes an amino acid sequence as set forth in any one of SEQ ID NOs: 18-21.

In some embodiments, the modified immune cell further includes a chimeric antigen receptor or a T cell receptor.

In some embodiments, the chimeric antigen receptor does not include the leptin and/or the functional fragment thereof, and/or, the leptin receptor and/or the functional fragment thereof.

In some embodiments, the chimeric antigen receptor does not include the low-density lipoprotein-receptor-related protein or the fragment thereof.

In some embodiments, the chimeric antigen receptor includes an intracellular domain, the intracellular domain includes a signaling domain and/or a costimulatory domain.

In some embodiments, the signaling domain includes a signaling domain of CD3zeta.

In some embodiments, the costimulatory domain includes a costimulatory domain of 4-1BB. In some embodiments, the chimeric antigen receptor includes a hinge region.

In some embodiments, the hinge region includes a hinge region of CD8.

In some embodiments, the chimeric antigen receptor includes a transmembrane region.

In some embodiments, the transmembrane region includes a transmembrane region of CD8.

In some embodiments, the chimeric antigen receptor includes a targeting moiety.

In some embodiments, the targeting moiety includes scFv.

In some embodiments, the targeting moiety specifically binds to and/or recognizes a tumor antigen.

In some embodiments, the targeting moiety specifically binds to and/or recognizes a target selected from a group consisting of: CD19, HER2, Mesothelin, GPC3 and BCMA.

In some embodiments, the chimeric antigen receptor includes an amino acid sequence as set forth in any one of SEQ ID NOs: 23-24.

In another aspect, the present application provides a method for inhibiting tumor cell proliferation and/or killing tumor cell, which includes: contacting the modified immune cell of the present application with the tumor cell.

In some embodiments, the method includes an in vitro method or an ex vivo method.

In some embodiments, the tumor includes solid tumor and/or non-solid tumor.

In some embodiments, the tumor includes B lymphocytic leukemia, breast cancer, stomach cancer, pancreatic cancer, multiple myeloma, mesothelioma, lung cancer and/or liver cancer.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

Figs. 1A-1H show the flow cytometry results of memory T cells of GPC3-targeted modified immune cells of the present application (CCR7+CD45RO-).
Figs. 2A-2H show the flow cytometry results of memory T cells of GPC3-targeted modified immune cells of the present application (CCR7+CD62L+).
Figs. 3A-3H show the flow cytometry results of memory T cells of BCMA-targeted modified immune cells of the present application (CCR7+CD45RO-).
Figs. 4A-4H show the flow cytometry results of memory T cells of BCMA-targeted modified immune cells of the present application (CCR7+CD62L+).
Figs. 5A-5H show the flow cytometry results of memory T cells of GPC3-targeted modified CD8+ immune cells of the present application in CD4 cells (CCR7+CD45RO-).
Figs. 6A-6H show the flow cytometry results of memory T cells of GPC3-targeted modified CD4+ immune cells of the present application in CD8 cells (CCR7+CD45RO-).
Figs. 7A-7F show the expression of negative regulatory proteins by the modified immune cells of the present application.
Fig. 8 shows the amplification of GPC3/MSLN/HER2-associated CAR-T.
Fig. 9 shows the flow cytometry results of memory T cells of MSLN-targeted modified immune cells with different structures of the present application.
Fig. 10 shows the flow cytometry results of memory T cells of HER2-targeted modified immune cells with different structures of the present application.
Fig. 11 shows the flow cytometry results of memory T cells of CD19-targeted modified immune cells with different structures of the present application.
Fig. 12 shows the amplification results of immune cells modified with different structures at different target sites of the present application under repeated stimulation.
Fig. 13 shows the results of tumor suppression and recurrence inhibition in mice with the GPC3-targeted modified immune cells of the present application.
Fig. 14 shows the results of body weight changes in mice with the GPC3-targeted modified immune cells of the present application.
Fig. 15 shows the results of cytokine changes in mice with the GPC3-targeted modified immune cells of the present application.
Fig. 16 shows the results of cell changes in mice with the GPC3-targeted modified immune cells of the present application.
Fig. 17 shows the detection results on the contents of modified cells in the spleen (A) and bone marrow (B) of mice after secondary tumor-loading with the GPC3-targeted modified immune cells of the present application.
Fig. 18 shows the results of tumor suppression and recurrence inhibition in mice with the BCMA-targeted modified immune cells of the present application.
Fig. 19 shows the results of cytokine in mice with the BCMA-targeted modified immune cells of the present application.

### Detailed Description of the Embodiments

The implementation of the present application will be illustrated in the following specific examples, and other advantages and effects of the present application will be easily known by those familiar with this technology from the content disclosed in the specification.

The following is a further description of the present application: In the present invention, unless otherwise specified, the scientific and technical terms used herein have the meanings commonly understood by those skilled in the art. In addition, protein and nucleic acid chemistry, molecular biology, cell and tissue culture, microbiology, immunology related terms and laboratory procedures used herein are all terms and routine procedures widely used in the corresponding fields. At the same time, in order to better understand the present invention, definitions and explanations of related terms are provided below.

In the present application, the term "immune cell" generally refers to cells participating in immune responses, for example, promoting the immune effector responses. In the present application, the immune cell may include lymphocytes. In addition, in the present application, the immune cell may include T cells, B cells, natural killer (NK) cells, mast cells, and phagocytes derived from bone marrow.

In the present application, the term "Chimeric Antigen Receptor" (CAR) generally refers to a fusion protein comprising an extracellular domain capable of binding an antigen and at least one intracellular domain. CAR is the core component of chimeric antigen receptor T cells (CAR-T), which may include a targeting moiety (for example, the moiety that binds tumor-associated antigens (TAAs)), a hinge region, a transmembrane region and an intracellular domain. In the present application, the CAR may be combined with the activated intracellular domain of a T cell receptor based on the antigen specificity of the antibody. Genetically modified T cells expressing CAR can specifically recognize and eliminate malignant cells expressing the target antigen.

In the present application, the term "T cell receptor" (TCR) generally refers to a T cell antigen receptor, which is a molecular structure that T cells specifically recognize and bind to antigen peptide-MHC molecules, and usually exists in the form of a complex with CD3 molecules on the surface of T cells. The TCR of a majority of T cells is composed of alpha and beta peptide chains, and the TCR of a minority of T cells is composed of gamma and delta peptide chains.

In the present application, the term "encoding" generally refers to the inherent property of a specific sequence of nucleotides in a polynucleotide such as a gene, cDNA, or mRNA as a template for synthesizing other polymers and macromolecules in biological processes, where the polymers and macromolecules have defined nucleotide sequences (i.e., rRNA, tRNA and mRNA) or defined amino acid sequences and biological properties derived therefrom. Therefore, if the transcription and translation of mRNA corresponding to a gene produces a protein in a cell or other biological systems, the gene, cDNA or RNA encodes the protein.

In the present application, the term "leptin" generally refers to a class of protein hormones secreted by adipose tissue. Its precursor consists of 167 amino acid residues, and there are 21 amino acid residue signal peptides at the N-terminal. The signal peptides of the precursor are cut off in blood to become 146 amino acids with a molecular weight of 16 KD, thus forming Leptin. Leptin has a wide range of biological effects, the most important of which is to act on the metabolic regulatory center of the hypothalamus, thereby playing the roles of suppressing appetite, reducing energy intake, increasing energy consumption, and inhibiting fat synthesis.

In the present application, the term "leptin receptor" generally refers to a class of transmembrane receptors, which mainly consist of an extracellular region, a transmembrane region and an intracellular region. There are many forms of human leptin receptors, which are divided into two types: long-type (OB-RL) and short-type (OB-RS). The extracellular region and the transmembrane region of both are exactly identical, and the main difference is the length of the intracellular region and the composition of the amino acid sequences. Long-type receptors of leptin are mainly expressed in hypothalamus, including arcuate nucleus, paraventricular nucleus, dorsomedial hypothalamic nucleus and lateral hypothalamic area, while short-type receptors are mostly distributed in adipose tissue, myocardium, lung, ovary and hematopoietic tissue, etc.

In the present application, the term "functional fragment thereof" generally refers to a fragment of a larger polypeptide or polynucleotide which retains the same activity or capability as that of its larger counterpart. The activity level of the functional fragment may be the same as that of its larger counterpart, or less or more. For example, the functional fragment of leptin may be such a polypeptide which is composed of less amino acids than a full-length leptin protein, but may still retain the activity of the full-length leptin.

In the present application, the term "lymphocyte" usually mainly includes natural killer (NK) cells, T cells, or B cells. NK cells are a class of cytotoxic lymphocytes, representing major components of the innate immune system. NK cells reject tumors and virus-infected cells. NK cells function through apoptosis or programmed cell death methods. NK cells are called "natural killer" because they can kill cells without the need of activation. T cells paly a major role in cell-mediated immunity (without involving antibodies). The T cell receptors (TCRs) of T cells differentiate T cells from other types of lymphocytes. The thymus (a specialized organ of the immune system) is the main factor for the maturation of T cells. There are six types of T cells: helper T cells (e.g., CD4+ cells), cytotoxic T cells (also known as TC, cytotoxic T lymphocytes, CTL, T-killing cells, cytolytic T cells, CD8+T cells, or killing T cells), memory T cells ((i) stem memory TSCM cells (similar to naive cells) are CD45RO-, CCR7+, CD45RA+, CD62L+ (L-selectin), CD27+, CD28+, and IL-7Rα+, but also display a large amount of CD95, IL-2Rβ, CXCR3, and LFA-1, and exhibit many functional properties characteristic of memory cells); (ii) central memory TCM cells express L-selectin and CCR7, which secret IL-2, but do not produce IFNγ or IL-4; and (iii) effector memory TEM cells do not express L-selectin or CCR7, but produce effector cytokines like IFNγ and IL-4), regulatory T cells (Treg, inhibitory T cells, or CD4+CD25+ regulatory T cells), natural killer T cells (NKTs) and γδT cells. In another aspect, B cells play a major role in humoral immunity (involving antibodies). B cells produce antibodies and antigens, and function as antigen presenting cells (APCs) and transform into memory B cells after activation by antigen interaction.

In the present application, the term "intracellular domain" generally refers to an intracellular domain of any truncated portion sufficient to transduce an activation signal. In the present application, the intracellular domain may include a signaling domain and/or a costimulatory domain.

In the present application, the term "signaling domain" generally refers to a domain capable of transducing signals located inside the cells. In the present application, the signaling domain can transmit signals into cells. For example, the signaling domain may be a signaling domain of the chimeric antigen receptor. For example, in some embodiments, the signaling domain may be selected from a signaling domain of CD3zeta, a signaling domain of CD3delta and a signaling domain of CD3epsilon.

In the present application, the term "costimulatory domain" generally refers to an intracellular domain that can provide immune costimulatory molecules, and the costimulatory molecules are cell surface molecules required for an effective response of lymphocytes to antigens. In the present application, the costimulatory domain may include a costimulatory domain of 4-1BB, a costimulatory domain of CD27 and/or a costimulatory domain of CD28.

In the present application, the term "hinge region" generally refers to a part of a heavy chain molecule linking the CH1 domain to the CH2 domain. In the present application, the hinge region may be a hinge region of CAR. For example, the hinge region may be selected from a hinge region of CD8, a hinge region of IgG1 and a hinge region of IgG4.

In the present application, the term "transmembrane region" generally refers to a domain of a peptide, a polypeptide or a protein capable of spanning the cytoplasmic membrane. In the present application, the transmembrane region may be a transmembrane region of CAR. For example, the transmembrane region may be selected from a group consisting of: a transmembrane region of CD8, a transmembrane region of CD24 and a transmembrane region of CD28.

In the present application, the term "targeting moiety" generally refers to a type of part that acts against certain specific tissues and cells. For example, the targeting moiety can specifically target tumor antigens. In the present application, the targeting moiety may include antibodies or antigen-binding fragments thereof.

In the present application, the term "specifically bind to and/or recognize" generally refers to measurable and reproducible interactions, such as the binding between targets and antibodies, which can determine the presence of the targets in the presence of a heterogeneous population of molecules, including biomolecules. For example, an antibody specifically binding to a target (which may be an epitope) is an antibody that binds to the target with greater affinity, avidity, easier, and/or for a greater duration than it binds to other targets. In one embodiment, the extent to which the antibody binds to an irrelevant target is lower than about 10% of the extent to which the antibody binds to the target, as measured by, for example, radioimmunoassay (RIA). In some embodiments, the antibody specifically binding to the target has a dissociation constant (KD) of < 1 × 10⁻⁶ M, < 1 × 10⁻⁷ M, < 1 × 10⁻⁸ M, < 1 × 10⁻⁹ M or < 1 × 10⁻¹⁰ M. In some embodiments, the antibody specifically binds to the epitope on the protein, and the epitope is conservative among proteins of different species. In another embodiment, specifical binding may include, but does not require exclusive binding.

In the present application, the term "tumor antigen" generally refers to antigen molecules present on tumor cells or normal cells. The tumor antigen may include: embryonic protein, glycoprotein antigen and squamous cell antigen. The tumor antigen may be selected from a group consisting of: an EDB domain of fibronectin, an EDA domain of fibronectin and cell necrotic regions.

In the present application, the term "antigen-binding fragment" generally refers to fragments with antigen-binding activity. In the present application, the antigen-binding fragment may be selected from a group consisting of: Fab, Fab', F(ab')₂, F(ab)₂, dAb, an isolated complementarity determining region (CDR), Fv and scFv.

In the present application, the term "pharmaceutical composition" generally refers to a composition suitable for administration to patients, human patients. For example, the pharmaceutical composition of the present application may include the modified immune cell and optionally a pharmaceutically acceptable carrier. In some embodiments, the acceptable ingredients of the pharmaceutical composition are not toxic to recipients at the doses and concentrations used. The pharmaceutical composition of the present application includes, but not limited to, liquid, frozen and lyophilized composition.

In the present application, the term "pharmaceutically acceptable carrier" generally refers to one of the components of the pharmaceutical composition, which may include a buffering agent, an antioxidant, a preservative, a low molecular weight polypeptide, a protein, a hydrophilic polymer, an amino acid, a carbohydrate, a chelating agent, a counter-ion, a metal complex and/or a nonionic surfactant and the like. For example, the pharmaceutically acceptable carrier may include an excipient. For example, the excipient may be selected from a group consisting of: starch, dextrin, sucrose, lactose, magnesium stearate, calcium sulfate, carboxymethyl cellulose, talc, calcium alginate gel, chitosan and nanospheres, and the like. For example, the pharmaceutically acceptable carrier may also be selected from a group consisting of: a pH regulator, an osmotic pressure regulator, a solubilizer and a bacteriostatic agent.

In the present application, the term "tumor" generally refers to neoplasm or solid lesion formed by the growth of abnormal cells. In the present application, the tumor may be solid tumor or blood tumor. For example, the tumor may be selected from a group consisting of: B lymphocytic leukemia, breast cancer, stomach cancer, pancreatic cancer, multiple myeloma, mesothelioma, lung cancer and liver cancer.

In the present application, the term "subject" generally refers to human or non-human animals, including, but not limited to, cat, dog, horse, pig, cow, sheep, rabbit, mouse, rat, or monkey.

In the present application, the term "administration" generally refers to giving the subject (e.g., the patient) a certain dose of substances. Administration can be conducted through any suitable ways, including parenterally, intrapulmonarily and intranasally, as well as (if required by topical treatment) intralesional administration. Parenteral infusion includes, e.g., intramuscular, intravenous, intra-artery, intraperitoneal or subcutaneous administration. Administration may be done through any suitable routes, for example by injection (such as intravenous or subcutaneous injection), depending in part on whether the administration is transient or long-term. Various dosing schedules are contemplated herein, including, but not limited to, a single administration or multiple administrations at various time points, bolus administration, and pulse infusion.

In the present application, the term "low-density lipoprotein-receptor-related protein (LR P)" generally refers to a class of cell surface proteins, belonging to a kind of endocytic rec eptors, which is one member of low-density-lipoprotein receptor (LDLR) gene family. In the present application, the low-density lipoprotein-receptor-related protein or the fragment there of may be selected from a group consisting of: a low-density lipoprotein-receptor-related pr otein 6 or a truncated body thereof, and a low-density lipoprotein-receptor-related protein 5 or a truncated body thereof.

In the present application, the term "low-density lipoprotein-receptor-related protein 6" or "LRP6" generally refers to any native LRP6 deriving from any vertebrate sources (including mammal, such as primates (e.g., human) and rodents (e.g., mice and rats)), unless otherwise indicated. This term covers "full-length" unprocessed LRP6 as well as any forms of LRP6 produced by intracellular processing. This term also includes naturally occurring LRP6 variants, e.g., splice variants or allelic variants.

In the present application, the term "low-density lipoprotein-receptor-related protein 5" or "LRP5" generally refers to any native LRP5 deriving from any vertebrate sources (including mammal, such as primates (e.g., human) and rodents (e.g., mice and rats)), unless otherwise indicated. This term covers "full-length" unprocessed LRP5 as well as any forms of LRP5 produced by intracellular processing. This term also includes naturally occurring LRP5 variants, e.g., splice variants or allelic variants.

In the present application, the term "truncated body" generally refers to anything less than the whole. For example, the amino acid sequence of the truncated body of the low-density lipoprotein-receptor-related protein 6 may include any amino acid sequences shorter than the full-length or intact low-density lipoprotein-receptor-related protein 6. For example, in the present application, the truncated body of the low-density lipoprotein-receptor-related protein 6 may include an intracellular region, a transmembrane region or an LDLR region of the low-density lipoprotein-receptor-related protein 6. For another example, the truncated body of the low-density lipoprotein-receptor-related protein 5 may include an intracellular region, a transmembrane region or an LDLR region of the low-density lipoprotein-receptor-related protein 5.

In the present application, the term "plasmid" generally refers to DNA molecules other than chromosomes or nucleoids in organisms such as bacteria and yeast. The plasmids may exist in the cytoplasm and have the ability to replicate autonomously, enabling them to maintain a constant copy number in daughter cells and express the genetic information they carry. The plasmids are used as gene vectors in genetic engineering research. For example, in the present application, the polynucleotide encoding the leptin and/or the functional fragment thereof, and/or the polynucleotide encoding the leptin receptor and/or the functional fragment thereof may be contained in a plasmid. In the present application, the plasmid may include a viral vector.

In the present application, the term "retroviral vector" generally refers to that it can clone and express foreign genes, but cannot self-package into proliferative viral particles. Most of these viruses have reverse transcriptase. Retrovirus contains at least three kinds of genes: *gag*, genes containing proteins that make up the center and structure of the virus; *pol*, genes containing reverse transcriptase and *env*, containing genes that make up the viral coat. Through retroviral transfection, retroviral vectors can randomly and stably integrate their own genome and the foreign genes they carry into the host cell genome, for example, they can integrate CAR molecules into the host cells.

In the present application, the term "lentiviral vector" generally refers to a diploid RNA viral vector that is a retrovirus. Lentiviral vectors are vectors prepared by, based on the genome of the lentivirus, removing multiple sequence structures related to the viral activity to make it biologically safe, and then introducing the sequence and expression structure of the target gene required for the experiment into the genome backbone. Through lentiviral vector transfection, retroviral vectors can randomly and stably integrate their own genome and the foreign genes they carry into the host cell genome, for example, they can integrate CAR molecules into the host cells.

In the present application, the term "transposon" generally refers to a discrete DNA fragment containing a transposase gene. The flank is a terminal inverted repeat (TIR) containing a transposase binding site. The transposase can bind to the TIR and transfer the transposon to a new site.

In the present application, the term "homologue" generally refers to proteins or polypeptides having at least about 80% (e.g., at least about 80%, about 83%, about 85%, about 90%, about 91%, about 92%, about 93%, about 94%, about 95%, about 96%, about 97%, about 98%, about 99% or higher) sequence homology to the amino acid sequence of the protein and/or the polypeptide.

In the present application, the terms "identity" or "homology" are used interchangeably, which generally refers to the similarity, likeness or correlation between two or more sequences. The "percentage of sequence homology" can be calculated by: comparing two sequences to be aligned in a comparison window to determine the number of positions where the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys and Met) are present in both sequences so as to obtain the number of matching positions, dividing the number of matching positions by the total number of positions in the comparison window (i.e., window size), and multiplying the result by 100, to generate the percentage of sequence homology. The alignment for determining the percentage of sequence homology can be achieved in a variety of ways known in the art, for example, by using publicly available computer softwares, such as BLAST, BLAST-2, ALIGN or Megalign (DNASTAR) softwares. A person skilled in the art can determine appropriate parameters for aligning sequences, including any algorithms needed to achieve the maximum alignment over the full-length sequence range being compared or within the target sequence region. The homology can also be determined by the following methods: PASTA and BLAST. A description of PASTA algorithm can be found in "Improved tools for biological sequence comparison" to W. R. Pearson and D. J. Lipman, Proc. Natl. Acad. Sci., 85: 2444-2448, 1988; and "Rapid and Sensitive Protein Similarity Searches" to D. J. Lipman and W. R. Pearson, Science, 227: 1435-1441, 1989. A description of BLAST algorithm can be found in "Basic Local Alignment Search Tool" to S. Altschul, W. Gish, W. Miller, E. W. Myers and D. Lipman, Journal of Molecular Biology, 215: 403-410, 1990.

In the present application, the term "include" generally refers to the inclusion of explicitly specified features, but not excluding other elements.

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Modified immune cell

In one aspect, the present application provides a modified immune cell, which includes a chimeric antigen receptor and/or a coding element therefor, or including a T cell receptor and/or a coding element therefor, and the modified immune cell further includes: leptin and/or a functional fragment thereof; and/or, a leptin receptor and/or a functional fragment thereof, and the expression quantity of the leptin receptor and/or the functional fragment thereof in the modified immune cell is increased compared with that in an immune cell without the corresponding modification.

The expression quantity of the leptin receptor and/or the functional fragment thereof can be determined by methods such as Western Blot (WB), ELISA, flow cytometry, PCR.

In the present application, the modified immune cell may include lymphocytes. In some embodiments, the modified immune cell may include T cells. The T cells may include memory stem cell-like T cells (TSCMs) and/or central memory T cells (TCMs). In some embodiments, the TSCM may be CCR7⁺ and/or CD62L⁺. In some other embodiments, the TSCM may also be selected from a group consisting of: CD45RA⁺ or CD45RA⁻, CD45RO⁺ or CD45RO⁻, CD27⁺, CD28⁺, CD127⁺, CD122⁺, CD95⁺, CD3⁺, CD4⁺ and CD8⁺.

### Leptin and leptin receptor

In the present application, the leptin and/or the functional fragment thereof may be derived from human. The leptin receptor and/or the functional fragment thereof may also be derived from human.

In the present application, the leptin may be secretory leptin or membrane-bound leptin. For example, the secretory leptin may include an amino acid sequence as set forth in SEQ ID NO: 13. The membrane-bound leptin may include an amino acid sequence as set forth in SEQ ID NO: 14, which may be synthesized by sequentially linking the amino acid sequences as set forth in SEQ ID NO: 13, SEQ ID NO: 22, SEQ ID NO: 6, SEQ ID NO: 15.

In the present application, the leptin and/or the functional fragment thereof may include an amino acid sequence as set forth in any one of SEQ ID NOs: 13-14.

In the present application, the leptin receptor may include sequences with self-activating functions so that the downstream signaling pathway of the leptin receptor can be continuously activated without binding leptin. The functional fragment of the leptin receptor may include a domain of the leptin receptor selected from a group consisting of: an intracellular region, a BOX 1 region, an FN3 region, a C2 region and a transmembrane region. For example, the functional fragment of the leptin receptor may include a leptin receptor transmembrane region (e.g., it may include an amino acid sequence as set forth in SEQ ID NO: 15). For another example, the functional fragment of the leptin receptor may include a leptin receptor signal peptide (e.g., it may include an amino acid sequence as set forth in SEQ ID NO: 16). In addition, the intracellular region of the leptin receptor may be the amino acid sequence at positions 865-1165 in the full-length sequence of the leptin receptor, the BOX 1 region of the leptin receptor may be the amino acid sequence at positions 871-878 in the full-length sequence of the leptin receptor, the FN3 region of the leptin receptor may be the amino acid sequence at positions 732-820 or 636-723 or 536-620 or 235-317 in the full-length sequence of the leptin receptor, the C2 region of the leptin receptor may be the amino acid sequence at positions 329-424 in the full-length sequence of the leptin receptor, the transmembrane region of the leptin receptor may be the amino acid sequence at positions 841-864 in the full-length sequence of the leptin receptor, and the above domains of the leptin receptor have important functions in leptin signaling, protein dimerization or multimer formation.

In the present application, the leptin receptor and/or the functional fragment thereof may include an amino acid sequence as set forth in any one of SEQ ID NOs: 15-17.

In addition, it should be noted that, the sequence of the leptin and/or the functional fragment thereof, and the sequence of the leptin receptor and/or the functional fragment thereof may include one or more amino acid substitutions, additions and/or deletions. For example, the sequence of the leptin and/or the functional fragment thereof may be at least 80%, 85%, 90%, 95%, 96%, 97%, 98% or 99% homologous to its source sequence. For another example, the sequence of the leptin receptor and/or the functional fragment thereof may be at least 80%, 85%, 90%, 95%, 96%, 97%, 98% or 99% homologous to its source sequence.

### Chimeric antigen receptor

In the present application, the chimeric antigen receptor may not include the leptin and/or the functional fragment thereof. The chimeric antigen receptor may also not include the leptin receptor and/or the functional fragment thereof. In some instances, the chimeric antigen receptor may include the leptin and/or the functional fragment thereof so as to form a protein complex. The chimeric antigen receptor may also include the leptin receptor and/or the functional fragment thereof so as to form a protein complex.

In the present application, the chimeric antigen receptor includes an intracellular domain, and the intracellular domain may include a signaling domain and/or a costimulatory domain. The signaling domain may include one or more selected from a group consisting of: a signaling domain of CD3zeta, a signaling domain of CD3delta and a signaling domain of CD3epsilon. For example, the signaling domain may include a signaling domain of CD3zeta (e.g., it may include an amino acid sequence as set forth in SEQ ID NO: 10). The costimulatory domain includes one or more selected from a group consisting of: a costimulatory domain of 4-1BB, a costimulatory domain of CD27 and a costimulatory domain of CD28. The costimulatory domain may include a costimulatory domain of 4-1BB (e.g., it may include an amino acid sequence as set forth in SEQ ID NO: 9). For another example, the costimulatory domain may include a costimulatory domain of CD28 (e.g., it may include an amino acid sequence as set forth in SEQ ID NO: 8). The nucleotide sequence encoding the costimulatory domain of 4-1BB may be as set forth in SEQ ID NO: 33, and the nucleotide sequence encoding the costimulatory domain of CD28 may be as set forth in SEQ ID NO: 32.

In the present application, the chimeric antigen receptor may include a hinge region. The hinge region may include one or more selected from a group consisting of: a hinge region of CD8, a hinge region of IgG1 and a hinge region of IgG4. For example, the hinge region may include a hinge region of CD8 (e.g., it may include an amino acid sequence as set forth in SEQ ID NO: 6).

In the present application, the chimeric antigen receptor may include a transmembrane region. The transmembrane region may include one or more selected from a group consisting of: a transmembrane region of CD8, a transmembrane region of CD28 and a transmembrane region of CD24. For example, the transmembrane region may include a transmembrane region of CD8 (e.g., it may include an amino acid sequence as set forth in SEQ ID NO: 7).

In the present application, the chimeric antigen receptor may include a targeting moiety. The targeting moiety may include an antibody or an antigen-binding fragment thereof. For example, the targeting moiety may include, but not limited to, recombinant antibody, monoclonal antibody, human antibody, humanized antibody, chimeric antibody, bispecific antibody, single-chain antibody, diabody, triabody, tetrabody, Fv fragment, scFv fragment, Fab fragment, Fab' fragment, F(ab')₂ fragment and camelized single-domain antibody.

In the present application, the antibody may include a murine antibody. In some embodiments, the murine antibody or the antigen-binding fragment thereof may also include light chain constant regions of murine kappa, lambda chains or variants thereof, or include heavy chain constant regions of murine IgGl, IgG2, IgG3 or IgG4 or variants thereof.

In the present application, the antibody may be a humanized antibody. In other words, the targeting moiety may be an antibody that immunospecifically binds to an antigen of interest and includes framework regions (FR) substantially having the amino acid sequence of a human antibody and complementarity determining regions (CDRs) substantially having the amino acid sequence of a non-human antibody, or variants, derivatives, analogues or fragments thereof. "Substantially" used herein refers to, in the case of CDR, that the amino acid sequence of CDR is at least 80%, at least 85%, at least 90%, at least 95%, at least 98% or at least 99% identical to the amino acid sequence of CDR of a non-human antibody. The humanized antibody may substantially include all, at least one and usually two variable domains (Fab, Fab', F(ab')2, FabC, Fv), wherein all or substantially all of the CDR regions correspond to CDR regions of non-human immunoglobulin (i.e., antibodies) and all or substantially all of the framework regions are framework regions having consensus sequences of human immunoglobulin. The humanized antibody also includes at least a part of immunoglobulin constant regions (e.g., Fc), which are generally the constant regions of human immunoglobulin. In some examples, the humanized antibody contains at least one variable domain of a light chain and a heavy chain. The antibody may also include CH1, hinge, CH2, CH3 and CH4 regions of a heavy chain. In some examples, the humanized antibody only contains a humanized light chain. In some examples, the humanized antibody only contains a humanized heavy chain. In a particular example, the humanized antibody only contains a humanized variable domain of a light chain and/or a humanized heavy chain.

In the present application, the antigen-binding fragment may include Fab, Fab', F(ab)₂, Fv fragments, F(ab')₂, scFv, di-scFv and/or dAb.

In the present application, the targeting moiety may include scFv. For example, the targeting moiety may include scFv of GPC3, whose amino acid sequence may be as set forth in SEQ ID NO: 1. For example, the targeting moiety may include scFv of L1H2 (targeting GPC3), whose amino acid sequence may be as set forth in SEQ ID NO: 47. For example, the targeting moiety may include scFv of CD 19, whose amino acid sequence may be as set forth in SEQ ID NO: 2. For example, the targeting moiety may include scFv of BCMA, whose amino acid sequence may be as set forth in SEQ ID NO: 3. For example, the targeting moiety may include scFv of Mesothelin (or MSLN), whose amino acid sequence may be as set forth in SEQ ID NO: 4. For example, the targeting moiety may include scFv of HER2, whose amino acid sequence may be as set forth in SEQ ID NO: 5. The nucleotide sequence encoding scFv of GPC3 may be as set forth in SEQ ID NO: 25. The nucleotide sequence encoding scFv of CD19 may be as set forth in SEQ ID NO: 26. The nucleotide sequence encoding scFv of BCMA may be as set forth in SEQ ID NO: 27. The nucleotide sequence encoding scFv of Mesothelin (or MSLN) may be as set forth in SEQ ID NO: 28. The nucleotide sequence encoding scFv of HER2 may be as set forth in SEQ ID NO: 29.

In the present application, the targeting moiety may specifically bind to and/or recognize a tumor antigen. In some embodiments, the targeting moiety may specifically bind to and/or recognize a target selected from a group consisting of: CD19, HER2, Mesothelin, GPC3 and BCMA.

In the present application, the chimeric antigen receptor can link splicing proteins. The splicing proteins may be T2A or P2A. For example, the splicing proteins may include an amino acid sequence as set forth in any one of SEQ ID NOs: 11-12.

In the present application, the chimeric antigen receptor can also link ribosome skipping sites. The ribosome skipping sites may be IRES. For example, the ribosome skipping sites may include a nucleotide sequence as set forth in SEQ ID NO: 46.

In the present application, the chimeric antigen receptor may include a targeting moiety, a hinge region, a transmembrane region and an intracellular domain in order from the N-terminus to the C-terminus. For example, the chimeric antigen receptor may include a targeting moiety, a hinge region, a transmembrane region, a costimulatory domain and a signaling domain in order from the N-terminus to the C-terminus. For example, the chimeric antigen receptor may include scFv of GPC3, a hinge region of CD8, a transmembrane region of CD8, a costimulatory domain of 4-1BB and a signaling domain of CD3zeta in order from the N-terminus to the C-terminus. For example, the chimeric antigen receptor may include an amino acid sequence as set forth in SEQ ID NO: 23. For example, the chimeric antigen receptor may be GPC3-41BB. For another example, the chimeric antigen receptor may include scFv of BCMA, a hinge region of CD8, a transmembrane region of CD8, a costimulatory domain of 4-1BB and a signaling domain of CD3zeta in order from the N-terminus to the C-terminus. For example, the chimeric antigen receptor may include an amino acid sequence as set forth in SEQ ID NO: 24. For example, the chimeric antigen receptor may be BCMA-41BB.

In the present application, the chimeric antigen receptor may include an amino acid sequence as set forth in any one of SEQ ID NOs: 23-24.

In addition, it should be noted that, the sequence of the chimeric antigen receptor may include one or more amino acid substitutions, additions and/or deletions. For example, the sequence of the chimeric antigen receptor may be at least 80%, 85%, 90%, 95%, 96%, 97%, 98% or 99% homologous to its source sequence.

Likewise, the sequence of the T cell receptor may include one or more amino acid substitutions, additions and/or deletions. For example, the sequence of the T cell receptor may be at least 80%, 85%, 90%, 95%, 96%, 97%, 98% or 99% homologous to its source sequence.

### Modified immune cell

In the present application, the modified immune cell may include a chimeric antigen receptor and/or a coding element therefor and leptin and/or a functional fragment thereof. For example, the modified immune cell may be GPC3-41BB-Li, wherein, the amino acid sequence of the chimeric antigen receptor is as set forth in SEQ ID NO: 23, the amino acid sequence of the leptin is as set forth in SEQ ID NO: 13, and the amino acid sequence of the splicing protein is as set forth in SEQ ID NO: 11. For another example, the modified immune cell may be BCMA-41BB-Li, wherein, the amino acid sequence of the chimeric antigen receptor is as set forth in SEQ ID NO: 24, the amino acid sequence of the leptin is as set forth in SEQ ID NO: 13, and the amino acid sequence of the splicing protein is as set forth in SEQ ID NO: 11.

In the present application, the modified immune cell may include a chimeric antigen receptor and/or a coding element therefor and a leptin receptor and/or a functional fragment thereof. For example, the modified immune cell may be GPC3-41BB-LiR, wherein, the amino acid sequence of the chimeric antigen receptor is as set forth in SEQ ID NO: 23, the amino acid sequence of the leptin receptor is as set forth in SEQ ID NO: 17, the amino acid sequence of the leptin receptor signal peptide is as set forth in SEQ ID NO: 16, and the amino acid sequence of the splicing protein is as set forth in SEQ ID NO: 11. For another example, the modified immune cell may be BCMA-41BB-LiR, wherein, the amino acid sequence of the chimeric antigen receptor is as set forth in SEQ ID NO: 24, the amino acid sequence of the leptin receptor is as set forth in SEQ ID NO: 17, the amino acid sequence of the leptin receptor signal peptide is as set forth in SEQ ID NO: 16, and the amino acid sequence of the splicing protein is as set forth in SEQ ID NO: 11.

In the present application, the modified immune cell may include a chimeric antigen receptor and/or a coding element therefor, leptin and/or a functional fragment thereof and a leptin receptor and/or a functional fragment thereof. For example, the modified immune cell may be GPC3-41BB-LiM, wherein, the amino acid sequence of the chimeric antigen receptor is as set forth in SEQ ID NO: 23, the amino acid sequence of the leptin is as set forth in SEQ ID NO: 13, the amino acid sequence of the leptin receptor transmembrane region is as set forth in SEQ ID NO: 15, the amino acid sequence of the splicing protein is as set forth in SEQ ID NO: 11, and the amino acid sequence of the linker is as set forth in SEQ ID NO: 22. For another example, the modified immune cell may be BCMA-41BB-LiM, wherein, the amino acid sequence of the chimeric antigen receptor is as set forth in SEQ ID NO: 24, the amino acid sequence of the leptin is as set forth in SEQ ID NO: 13, the amino acid sequence of the leptin receptor transmembrane region is as set forth in SEQ ID NO: 15, the amino acid sequence of the splicing protein is as set forth in SEQ ID NO: 11, and the amino acid sequence of the linker is as set forth in SEQ ID NO: 22.

In the present application, the modified immune cell may include a T cell receptor and/or a coding element therefor and leptin and/or a functional fragment thereof.

In the present application, the modified immune cell may include a T cell receptor and/or a coding element therefor and a leptin receptor and/or a functional fragment thereof.

In the present application, the modified immune cell may include a T cell receptor and/or a coding element therefor, leptin and/or a functional fragment thereof and a leptin receptor and/or a functional fragment thereof.

In addition, it should be noted that, the sequence of the modified immune cell of the present application may include one or more amino acid substitutions, additions and/or deletions. For example, the sequence of the modified immune cell may be at least 80%, 85%, 90%, 95%, 96%, 97%, 98% or 99% homologous to its source sequence.

### Modified immune cell

In another aspect, the present application provides another modified immune cell, which includes leptin and/or a functional fragment thereof, and/or, a leptin receptor and/or a functional fragment thereof, and a low-density lipoprotein-receptor-related protein or a fragment thereof.

In the present application, the modified immune cell may include lymphocytes. In some embodiments, the modified immune cell may include T cells. The T cells may include memory stem cell-like T cells (TSCMs) and/or central memory T cells (TCMs). In some embodiments, the TSCMs may be CCR7⁺ and/or CD62L⁺. In some other embodiments, the TSCMs may also be selected from a group consisting of: CD45RA⁺ or CD45RA⁻, CD45RO⁺ or CD45RO⁻, CD27⁺, CD28⁺, CD127⁺, CD122⁺, CD95⁺, CD3⁺, CD4⁺ and CD8⁺.

In the present application, the structures and properties of the leptin and/or the functional fragment thereof and the leptin receptor and/or the functional fragment thereof can be as described above, and will not be repeated here.

In the present application, the low-density lipoprotein-receptor-related protein or the fragment thereof may be selected from a group consisting of: a low-density lipoprotein-receptor-related protein 6 or a truncated body thereof, and a low-density lipoprotein-receptor-related protein 5 or a truncated body thereof. Wherein, the truncated body of the low-density lipoprotein-receptor-related protein 6 may include an intracellular region of the low-density lipoprotein-receptor-related protein 6; and/or, the truncated body of the low-density lipoprotein-receptor-related protein 5 may include an intracellular region of the low-density lipoprotein-receptor-related protein 5. In addition, the truncated body of the low-density lipoprotein-receptor-related protein 6 may also include a functional domain selected from a group consisting of: a transmembrane region of the low-density lipoprotein-receptor-related protein 6 and an LDLR region of the low-density lipoprotein-receptor-related protein 6; and/or, wherein the truncated body of the low-density lipoprotein-receptor-related protein 5 may also include a functional domain selected from a group consisting of: a transmembrane region of the low-density lipoprotein-receptor-related protein 5 and an LDLR region of the low-density lipoprotein-receptor-related protein 5. For example, the low-density lipoprotein-receptor-related protein or the fragment thereof may be a low-density lipoprotein-receptor-related protein 6 (i.e., LRP6), whose amino acid sequence may be as set forth in SEQ ID NO: 18. For example, the low-density lipoprotein-receptor-related protein or the fragment thereof may be the truncated body of a low-density lipoprotein-receptor-related protein 6 (i.e., TL6), whose amino acid sequence may be as set forth in SEQ ID NO: 19. For another example, the low-density lipoprotein-receptor-related protein or the fragment thereof may be a low-density lipoprotein-receptor-related protein 5 (i.e., LRP5), whose amino acid sequence may be as set forth in SEQ ID NO: 20. For another example, the low-density lipoprotein-receptor-related protein or the fragment thereof may be the truncated body of a low-density lipoprotein-receptor-related protein 5 (i.e., TL5), whose amino acid sequence may be as set forth in SEQ ID NO: 21. In addition, the nucleotide sequence encoding LRP6 may be as set forth in SEQ ID NO: 41, the nucleotide sequence encoding TL6 may be as set forth in SEQ ID NO: 42, the nucleotide sequence encoding LRP5 may be as set forth in SEQ ID NO: 43, and the nucleotide sequence encoding TL5 may be as set forth in SEQ ID NO: 44.

In the present application, the low-density lipoprotein-receptor-related protein or the fragment thereof may be a low-density lipoprotein-receptor-related protein 6 or a truncated body thereof. In other instances, the low-density lipoprotein-receptor-related protein or a fragment thereof may be a low-density lipoprotein-receptor-related protein 5 or a truncated body thereof. In some other instances, the low-density lipoprotein-receptor-related protein or the fragment thereof may also be a low-density lipoprotein-receptor-related protein 6 or a truncated body thereof, and a low-density lipoprotein-receptor-related protein 5 or a truncated body thereof.

In the present application, the low-density lipoprotein-receptor-related protein or the fragment thereof may include amino acid sequences as set forth in SEQ ID NOs: 18-21.

In addition, it should be noted that, the sequence of the low-density lipoprotein-receptor-related protein or the fragment thereof may include one or more amino acid substitutions, additions and/or deletions. For example, the sequence of the low-density lipoprotein-receptor-related protein or the fragment thereof may be at least 80%, 85%, 90%, 95%, 96%, 97%, 98% or 99% homologous to its source sequence.

In the present application, the modified immune cell may also include a chimeric antigen receptor or a T cell receptor. The chimeric antigen receptor may not include the leptin and/or the functional fragment thereof. The chimeric antigen receptor may also not include the leptin receptor and/or the functional fragment thereof. In addition, the chimeric antigen receptor may also not include the low-density lipoprotein-receptor-related protein or the fragment thereof. In some instances, the chimeric antigen receptor may include the leptin and/or the functional fragment thereof so as to form a protein complex. The chimeric antigen receptor may also include the leptin receptor and/or the functional fragment thereof so as to form a protein complex. In addition, the chimeric antigen receptor may also include the low-density lipoprotein-receptor-related protein or the fragment thereof so as to form a protein complex. The specific structure and properties of the chimeric antigen receptor can be as described above, and will not be repeated here.

In the present application, the modified immune cell may include a chimeric antigen receptor and/or a coding element therefor and a low-density lipoprotein-receptor-related protein or a fragment thereof. In some embodiments, the modified immune cell may include a chimeric antigen receptor and/or a coding element therefor and a low-density lipoprotein-receptor-related protein 6. For example, the modified immune cell may be GPC3-41BB-L6, wherein, the amino acid sequence of the chimeric antigen receptor is as set forth in SEQ ID NO: 23, the amino acid sequence of the low-density lipoprotein-receptor-related protein 6 is as set forth in SEQ ID NO: 18, and the amino acid sequence of the splicing protein is as set forth in SEQ ID NO: 11. For another example, the modified immune cell may be BCMA-41BB-L6, wherein, the amino acid sequence of the chimeric antigen receptor is as set forth in SEQ ID NO: 24, the amino acid sequence of the low-density lipoprotein-receptor-related protein 6 is as set forth in SEQ ID NO: 18, and the amino acid sequence of the splicing protein is as set forth in SEQ ID NO: 11.

In the present application, the modified immune cell may include a chimeric antigen receptor and/or a coding element therefor, a low-density lipoprotein-receptor-related protein or a fragment thereof and leptin and/or a functional fragment thereof. In some embodiments, the modified immune cell may include a chimeric antigen receptor and/or a coding element therefor, a low-density lipoprotein-receptor-related protein 6 and leptin and/or a functional fragment thereof. For example, the modified immune cell may be GPC3-41BB-L6-Li, wherein, the amino acid sequence of the chimeric antigen receptor is as set forth in SEQ ID NO: 23, the amino acid sequence of the low-density lipoprotein-receptor-related protein 6 is as set forth in SEQ ID NO: 18, the amino acid sequence of the leptin is as set forth in SEQ ID NO: 13, and the amino acid sequence of the splicing protein is as set forth in SEQ ID NO: 11 and SEQ ID NO: 12. For another example, the modified immune cell may be BCMA-41BB-L6-Li, wherein, the amino acid sequence of the chimeric antigen receptor is as set forth in SEQ ID NO: 24, the amino acid sequence of the low-density lipoprotein-receptor-related protein 6 is as set forth in SEQ ID NO: 18, the amino acid sequence of the leptin is as set forth in SEQ ID NO: 13, and the amino acid sequence of the splicing protein is as set forth in SEQ ID NO: 11 and SEQ ID NO: 12.

In the present application, the modified immune cell may include a chimeric antigen receptor and/or a coding element therefor, a low-density lipoprotein-receptor-related protein or a fragment thereof and a leptin receptor and/or a functional fragment thereof. In some embodiments, the modified immune cell may include a chimeric antigen receptor and/or a coding element therefor, a low-density lipoprotein-receptor-related protein 6 and a leptin receptor and/or a functional fragment thereof. For example, the modified immune cell may be GPC3-41BB-L6-LiR, wherein, the amino acid sequence of the chimeric antigen receptor is as set forth in SEQ ID NO: 23, the amino acid sequence of the low-density lipoprotein-receptor-related protein 6 is as set forth in SEQ ID NO: 18, the amino acid sequence of the leptin receptor signal peptide is as set forth in SEQ ID NO: 16, the amino acid sequence of the leptin receptor is as set forth in SEQ ID NO: 17, and the amino acid sequence of the splicing protein is as set forth in SEQ ID NO: 11 and SEQ ID NO: 12. For another example, the modified immune cell may be BCMA-41BB-L6-LiR, wherein, the amino acid sequence of the chimeric antigen receptor is as set forth in SEQ ID NO: 24, the amino acid sequence of the low-density lipoprotein-receptor-related protein 6 is as set forth in SEQ ID NO: 18, the amino acid sequence of the leptin receptor signal peptide is as set forth in SEQ ID NO: 16, the amino acid sequence of the leptin receptor is as set forth in SEQ ID NO: 17, and the amino acid sequence of the splicing protein is as set forth in SEQ ID NO: 11 and SEQ ID NO: 12.

In the present application, the modified immune cell may include a chimeric antigen receptor and/or a coding element therefor, a low-density lipoprotein-receptor-related protein or a fragment thereof, leptin and/or a functional fragment thereof and a leptin receptor and/or a functional fragment thereof. In some embodiments, the modified immune cell may include a chimeric antigen receptor and/or a coding element therefor, a low-density lipoprotein-receptor-related protein 6, leptin and/or a functional fragment thereof and a leptin receptor and/or a functional fragment thereof. For example, the modified immune cell may be GPC3-41BB-L6-LiM, wherein, the amino acid sequence of the chimeric antigen receptor is as set forth in SEQ ID NO: 23, the amino acid sequence of the low-density lipoprotein-receptor-related protein 6 is as set forth in SEQ ID NO: 18, the amino acid sequence of the leptin is as set forth in SEQ ID NO: 13, the amino acid sequence of the leptin receptor transmembrane region is as set forth in SEQ ID NO: 15, the amino acid sequence of the splicing protein is as set forth in SEQ ID NO: 11 and SEQ ID NO: 12, and the amino acid sequence of the linker is as set forth in SEQ ID NO: 22. For another example, the modified immune cell may be BCMA-41BB-L6-LiM, wherein, the amino acid sequence of the chimeric antigen receptor is as set forth in SEQ ID NO: 24, the amino acid sequence of the low-density lipoprotein-receptor-related protein 6 is as set forth in SEQ ID NO: 18, the amino acid sequence of the leptin is as set forth in SEQ ID NO: 13, the amino acid sequence of the leptin receptor transmembrane region is as set forth in SEQ ID NO: 15, the amino acid sequence of the splicing protein is as set forth in SEQ ID NO: 11 and SEQ ID NO: 12, and the amino acid sequence of the linker is as set forth in SEQ ID NO: 22.

In the present application, the modified immune cell may include a T cell receptor and/or a coding element therefor and a low-density lipoprotein-receptor-related protein or a fragment thereof.

In the present application, the modified immune cell may include a T cell receptor and/or a coding element therefor, a low-density lipoprotein-receptor-related protein or a fragment thereof and leptin and/or a functional fragment thereof.

In the present application, the modified immune cell may include a T cell receptor and/or a coding element therefor, a low-density lipoprotein-receptor-related protein or a fragment thereof and a leptin receptor and/or a functional fragment thereof.

In the present application, the modified immune cell may include a T cell receptor and/or a coding element therefor, a low-density lipoprotein-receptor-related protein or a fragment thereof, leptin and/or a functional fragment thereof and a leptin receptor and/or a functional fragment thereof.

In addition, it should be noted that, the sequence of the modified immune cell of the present application may include one or more amino acid substitutions, additions and/or deletions. For example, the sequence of the modified immune cell may be at least 80%, 85%, 90%, 95%, 96%, 97%, 98% or 99% homologous to its source sequence.

### Pharmaceutical composition

In another aspect, the present application also provides a pharmaceutical composition, which may include the modified immune cell of the present application and optionally a pharmaceutically acceptable carrier. Where, the pharmaceutically acceptable carrier may include a buffering agent, an antioxidant, a preservative, a low molecular weight polypeptide, a protein, a hydrophilic polymer, an amino acid, a carbohydrate, a chelating agent, a counter-ion, a metal complex and/or a nonionic surfactant and the like. For example, the pharmaceutically acceptable carrier may include an excipient, for exampel, the excipient may be selected from a group consisting of: starch, dextrin, sucrose, lactose, magnesium stearate, calcium sulfate, carboxymethyl cellulose, talc, calcium alginate gel, chitosan and nanospheres, and the like. For example, the pharmaceutically acceptable carrier may also be selected from a group consisting of: a pH regulator, an osmotic pressure regulator, a solubilizer and a bacteriostatic agent.

### Application and use

In another aspect, the present application also provides a use of the modified immune cell or the pharmaceutical composition in the preparation of a drug for treating tumor.

In another aspect, the present application provides a method for promoting immune cell proliferation, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In another aspect, the present application provides a method for promoting the production of memory immune cells, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In another aspect, the present application provides a method for enhancing the killing ability of an immune cell against tumor, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In another aspect, the present application provides a method for inhibiting an immune cell from expressing an immune negative regulatory protein, which includes steps of:
1) providing a modified immune cell including a chimeric antigen receptor and/or a coding element therefor, or including a T cell receptor and/or a coding element therefor; and
2) allowing the modified immune cell of 1) to express leptin and/or a functional fragment thereof, and/or increasing the expression quantity of a leptin receptor and/or a functional fragment thereof in the modified immune cell of 1).

In the present application, the immune negative regulatory protein may be TIM3, LAG3, PD1 or PDL1.

In the present application, the increased expression quantity may include that, the expression quantity of the leptin receptor and/or the functional fragment thereof in the modified immune cell is increased compared with that in an immune cell without the corresponding modification. The expression quantity of the leptin receptor and/or the functional fragment thereof can be determined by methods such as Western Blot (WB), ELISA, flow cytometry, PCR.

In the present application, the step 2) may include:
introducing a polynucleotide encoding the leptin and/or the functional fragment thereof into the modified immune cell, and/or introducing a polynucleotide encoding the leptin receptor and/or the functional fragment thereof into the modified immune cell.

In the present application, the polynucleotide encoding the leptin and/or the functional fragment thereof, and/or the polynucleotide encoding the leptin receptor and/or the functional fragment thereof may be contained in a plasmid. The plasmid may include a viral vector. The plasmid may also include a retroviral vector, a lentiviral vector or a transposon plasmid.

In the present application, the modified immune cell may include lymphocytes. In some embodiments, the modified immune cell may include T cells. The T cells may include memory stem cell-like T cells (TSCMs) and/or central memory T cells (TCMs). In some embodiments, the TSCMs may be CCR7⁺ and/or CD62L⁺. In some other embodiments, the TSCMs may also be selected from a group consisting of: CD45RA⁺ or CD45RA⁻, CD45RO⁺ or CD45RO⁻, CD27⁺, CD28⁺, CD127⁺, CD122⁺, CD95⁺, CD3⁺, CD4⁺ and CD8⁺.

In the present application, the structures and properties of the leptin and/or the functional fragment thereof and the leptin receptor and/or the functional fragment thereof can be as described above, and will not be repeated here.

In another aspect, the present application also provides a method for promoting immune cell proliferation, which includes steps of:
allowing a modified immune cell to express leptin and/or a functional fragment thereof, and/or, increasing the expression quantity of a leptin receptor and/or a functional fragment thereof in the modified immune cell, and allowing the modified immune cell to express a low-density lipoprotein-receptor-related protein or a fragment thereof.

In another aspect, the present application also provides a method for promoting the production of memory immune cells, which includes steps of:
allowing a modified immune cell to express leptin and/or a functional fragment thereof, and/or, increasing the expression quantity of a leptin receptor and/or a functional fragment thereof in the modified immune cell, and allowing the modified immune cell to express a low-density lipoprotein-receptor-related protein or a fragment thereof.

In another aspect, the present application also provides a method for enhancing the killing ability of an immune cell against tumor, which includes steps of:
allowing a modified immune cell to express leptin and/or a functional fragment thereof, and/or, increasing the expression quantity of a leptin receptor and/or a functional fragment thereof in the modified immune cell, and allowing the modified immune cell to express a low-density lipoprotein-receptor-related protein or a fragment thereof.

In another aspect, the present application also provides a method for inhibiting an immune cell from expressing an immune negative regulatory protein, which includes steps of:
allowing a modified immune cell to express leptin and/or a functional fragment thereof, and/or, increasing the expression quantity of a leptin receptor and/or a functional fragment thereof in the modified immune cell, and allowing the modified immune cell to express a low-density lipoprotein-receptor-related protein or a fragment thereof.

In the present application, the immune negative regulatory protein may be TIM3, LAG3, PD1 or PDL1.

In the present application, the increased expression quantity may include that, the expression quantity of the leptin receptor and/or the functional fragment thereof in the modified immune cell is increased compared with that in an immune cell without the corresponding modification. The expression quantity of the leptin receptor and/or the functional fragment thereof can be determined by methods such as Western Blot (WB), ELISA, flow cytometry, PCR.

In the present application, the method, including the method for promoting immune cell proliferation, the method for promoting the production of memory immune cells, the method for enhancing the killing ability of an immune cell against tumor or the method for inhibiting an immune cell from expressing an immune negative regulatory protein, may also include steps of: introducing a polynucleotide encoding the leptin and/or the functional fragment thereof into the modified immune cell, and/or introducing a polynucleotide encoding the leptin receptor and/or the functional fragment thereof into the modified immune cell.

In the present application, the method, including the method for promoting immune cell proliferation, the method for promoting the production of memory immune cells, the method for enhancing the killing ability of an immune cell against tumor or the method for inhibiting an immune cell from expressing an immune negative regulatory protein, may also include a step of: introducing a polynucleotide encoding the low-density lipoprotein-receptor-related protein or the fragment thereof into the modified immune cell.

In the present application, the polynucleotide encoding the leptin and/or the functional fragment thereof, and/or the polynucleotide encoding the leptin receptor and/or the functional fragment thereof may be contained in a plasmid.

In the present application, the polynucleotide encoding the low-density lipoprotein-receptor-related protein or the fragment thereof is contained in a plasmid.

The plasmid may include a viral vector. The plasmid may also include a retroviral vector, a lentiviral vector or a transposonplasmid.

In the present application, the modified immune cell may include lymphocytes. In some embodiments, the modified immune cell may include T cells. The T cells may include memory stem cell-like T cells (TSCMs) and/or central memory T cells (TCMs). In some embodiments, the TSCMs may be CCR7⁺ and/or CD62L⁺. In some other embodiments, the TSCMs may also be selected from a group consisting of: CD45RA⁺ or CD45RA⁻, CD45RO⁺ or CD45RO⁻, CD27⁺, CD28⁺, CD127⁺, CD122⁺, CD95⁺, CD3⁺, CD4⁺ and CD8⁺.

In the present application, the structures and properties of the leptin and/or the functional fragment thereof, the leptin receptor and/or the functional fragment thereof and the low-density lipoprotein-receptor-related protein or the fragment thereof can be as described above, and will not be repeated here.

In another aspect, the present application also provides a method for inhibiting tumor cell proliferation and/or killing tumor cell, which includes: contacting the modified immune cell of the present application with the tumor cell. In some embodiments, the contact may be administration, that is, giving the subject (e.g., a patient) a certain dose of the modified immune cell of the present application. Administration can be conducted through any suitable ways, including parenterally, intrapulmonarily and intranasally, as well as (if required by topical treatment) intralesional administration. Parenteral infusion includes, e.g., intramuscular, intravenous, intra-artery, intraperitoneal or subcutaneous administration. Administration may be done through any suitable routes, for example by injection (such as intravenous or subcutaneous injection), depending in part on whether the administration is transient or long-term. Various dosing schedules are contemplated herein, including, but not limited to, a single administration or multiple administrations at various time points, bolus administration, and pulse infusion.

In the present application, the method for inhibiting tumor cell proliferation and/or killing tumor cell, wherein the method may include an in vitro method or an ex vivo method.

In the present application, the tumor includes solid tumor and/or non-solid tumor. For example, the tumor may include B lymphocytic leukemia, breast cancer, stomach cancer, pancreatic cancer, multiple myeloma, mesothelioma, lung cancer and/or liver cancer.

Without intending to be limited by any theory, the following examples are only intended to illustrate the protein molecules, preparation methods and uses of the present application, and are not intended to limit the scope of the present invention. The examples do not include detailed descriptions of conventional methods, such as mehtods for constructing vectors and plasmids, methods for inserting protein-encoding genes into such vectors and plasmids, or methods for introducing plasmids into host cells.

### Examples

### Example 1. Construction of lentiviral vectors

The CAR-T, with targeting GPC3 and BCMA as examples, artificially synthesizes a fragment containing the CAR structure, and constructs it into a lentiviral vector (LV100A, System Biosciences Co.), and then transfects it according to the method documented in its instruction to obtain a lentivirus, thus obtaining GPC3-41BB, GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR; BCMA-41BB, BCMA-41BB-L6, BCMA-41BB-Li, BCMA-41BB-LiM, BCMA-41BB-LiR, BCMA-41BB-L6-Li, BCMA-41BB-L6-LiM, BCMA-41BB-L6-LiR lentiviruses, respectively.

The amino acid sequence of GPC3-41BB (from the N-terminus to the C-terminus) is assembled by splicing SEQ ID NO: 1, SEQ ID NO: 6, SEQ ID NO: 7, SEQ ID NO: 9, SEQ ID NO: 10 in order.

The amino acid sequence of GPC3-41BB-L6 (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 18 in order.

The amino acid sequence of GPC3-41BB-Li (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 13 in order.

The amino acid sequence of GPC3-41BB-LiM (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 13, SEQ ID NO: 22, SEQ ID NO: 6, SEQ ID NO: 15 in order.

The amino acid sequence of GPC3-41BB-LiR (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 16, SEQ ID NO: 17 in order.

The amino acid sequence of GPC3-41BB-L6-Li (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 13 in order.

The amino acid sequence of GPC3-41BB-L6-LiM (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 13, SEQ ID NO: 22, SEQ ID NO: 6, SEQ ID NO: 15 in order.

The amino acid sequence of GPC3-41BB-L6-LiR (from the N-terminus to the C-terminus) is assembled by splicing SEQ ID NO: 1, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 16, SEQ ID NO: 17 in order.

The amino acid sequence of L1H2-41BB-L6 (from the N-terminus to the C-terminus) is assembled by splicing SEQ ID NO: 47, SEQ ID NO: 6, SEQ ID NO: 7, SEQ ID NO: 9, SEQ ID NO: 10, SEQ ID NO: 11, SEQ ID NO: 18 in order.

The amino acid sequence of L1H2-41BB-LiR (from the N-terminus to the C-terminus) is assembled by splicing SEQ ID NO: 47, SEQ ID NO: 6, SEQ ID NO: 7, SEQ ID NO: 9, SEQ ID NO: 10, SEQ ID NO: 11, SEQ ID NO: 16, SEQ ID NO: 17 in order.

The amino acid sequence of L1H2-41BB-L6-Li (from the N-terminus to the C-terminus) is assembled by splicing SEQ ID NO: 47, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 13 in order.

The amino acid sequence of L1H2-41BB-L6-LiM (from the N-terminus to the C-terminus) is assembled by splicing SEQ ID NO: 47, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 13, SEQ ID NO: 22, SEQ ID NO: 6, SEQ ID NO: 15 in order. Correspondingly, the nucleotide sequence of GPC3-41BB (from the N-terminus to the C-terminus) is assembled by splicing SEQ ID NO: 25, SEQ ID NO: 30, SEQ ID NO: 31, SEQ ID NO: 33, SEQ ID NO: 34 in order.

The nucleotide sequence of GPC3-41BB-L6 (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 41 in order.

The nucleotide sequence of GPC3-41BB-Li (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 37 in order.

The nucleotide sequence of GPC3-41BB-LiM (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 37, SEQ ID NO: 45, SEQ ID NO: 30, SEQ ID NO: 38 in order.

The nucleotide sequence of GPC3-41BB-LiR (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 39, SEQ ID NO: 40 in order.

The nucleotide sequence of GPC3-41BB-L6-Li (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 37 in order.

The nucleotide sequence of GPC3-41BB-L6-LiM (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 37, SEQ ID NO: 45, SEQ ID NO: 30, SEQ ID NO: 38 in order.

The nucleotide sequence of GPC3-41BB-L6-LiR (from the N-terminus to the C-terminus) is assembled by splicing SEQ ID NO: 25, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 39, SEQ ID NO: 40 in order.

The nucleotide sequence of L1H2-41BB-L6 (from the N-terminus to the C-terminus) is assembled by splicing SEQ ID NO: 48, SEQ ID NO: 30, SEQ ID NO: 31, SEQ ID NO: 33, SEQ ID NO: 34, SEQ ID NO: 35, SEQ ID NO: 41 in order.

The nucleotide sequence of L1H2-41BB-LiR (from the N-terminus to the C-terminus) is assembled by splicing SEQ ID NO: 48, SEQ ID NO: 30, SEQ ID NO: 31, SEQ ID NO: 33, SEQ ID NO: 34, SEQ ID NO: 35, SEQ ID NO: 39, SEQ ID NO: 40 in order.

The nucleotide sequence of L1H2-41BB-L6-Li (from the N-terminus to the C-terminus) is assembled by splicing SEQ ID NO: 48, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 37 in order.

The nucleotide sequence of L1H2-41BB-L6-LiM (from the N-terminus to the C-terminus) is assembled by splicing SEQ ID NO: 48, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 37, SEQ ID NO: 45, SEQ ID NO: 30, SEQ ID NO: 38 in order. In addition, the amino acid sequence of BCMA-41BB (from the N-terminus to the C-terminus) is assembled by splicing SEQ ID NO: 3, SEQ ID NO: 6, SEQ ID NO: 7, SEQ ID NO: 9, SEQ ID NO: 10 in order.

The amino acid sequence of BCMA-41BB-L6 (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 18 in order.

The amino acid sequence of BCMA-41BB-Li (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 13 in order.

The amino acid sequence of BCMA-41BB-LiM (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 13, SEQ ID NO: 22, SEQ ID NO: 6, SEQ ID NO: 15 in order.

The amino acid sequence of BCMA-41BB-LiR (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 16, SEQ ID NO: 17 in order.

The amino acid sequence of BCMA-41BB-L6-Li (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 13 in order.

The amino acid sequence of BCMA-41BB-L6-LiM (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 13, SEQ ID NO: 22, SEQ ID NO: 6, SEQ ID NO: 15 in order.

The amino acid sequence of BCMA-41BB-L6-LiR (from the N-terminus to the C-terminus) is assembled by splicing SEQ ID NO: 3, SEQ ID NO: 6, SEQ ID NO: 7, SEQ ID NO: 9, SEQ ID NO: 10, SEQ ID NO: 11, SEQ ID NO: 18, SEQ ID NO: 12, SEQ ID NO: 16, SEQ ID NO: 17 in order.

Correspondingly, the nucleotide sequence ofBCMA-41BB (from the N-terminus to the C-terminus) is assembled by splicing SEQ ID NO: 27, SEQ ID NO: 30, SEQ ID NO: 31, SEQ ID NO: 33, SEQ ID NO: 34 in order.

The nucleotide sequence of BCMA-41BB-L6 (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 41 in order.

The nucleotide sequence of BCMA-41BB-Li (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 37 in order.

The nucleotide sequence of BCMA-41BB-LiM (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 37, SEQ ID NO: 45, SEQ ID NO: 30, SEQ ID NO: 38 in order.

The nucleotide sequence of BCMA-41BB-LiR (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 39, SEQ ID NO: 40 in order.

The nucleotide sequence of BCMA-41BB-L6-Li (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 37 in order.

The nucleotide sequence of BCMA-41BB-L6-LiM (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 37, SEQ ID NO: 45, SEQ ID NO: 30, SEQ ID NO: 38 in order.

The nucleotide sequence of BCMA-41BB-L6-LiR (from the N-terminus to the C-terminus) is assembled by splicing SEQ ID NO: 27, SEQ ID NO: 30, SEQ ID NO: 31, SEQ ID NO: 33, SEQ ID NO: 34, SEQ ID NO: 35, SEQ ID NO: 41, SEQ ID NO: 36, SEQ ID NO: 39, SEQ ID NO: 40 in order.

### Example 2. Lentiviral infection of T cells

The infection experiment was conducted following a conventional method known to those skilled in the art. The infection steps were briefly described as below:
1. Acquisition of peripheral blood mononuclear lymphocytes (PBMCs), >1×10⁷ cells were obtained through a blood apheresis system.
2. Treatment of cell culture dishes with experimental anti-human CD3/CD28 antibodies.
   Anti-human CD3 and anti-human CD28 antibodies were diluted with PBS to a final concentration of 1 ug/ml, the diluted antibody mixture was added into the cell culture dishes to cover the culture dishes, and incubated at room temperature for 2 hours, and then washed once with PBS after 2 hours, ready for use.
3. Activation of T cells
   The isolated PBMCs were resuspended with T lymphocyte culture solution (Xvivo15 culture medium + 5% FBS + 200 U/ml IL2 or Xvivo15 culture medium + 5%FBS + 20 ng/ml IL21 + 10 ng/ml IL7) to a final concentration of 1^{∗}10⁶ cells/ml, and cultured in the culture dishes treated in step 2 under conditions of 37°C + 5% CO₂ for a time period of 24 hours.
4. Infection of activated T cells

### 1) Formulation of infection reagents.

A certain amount of T cell culture solution was added into synperonic F108 with a final concentration of 1 mg/ml, mixed uniformly and heated in a water-bath pot to 37°C for later use.

### 2) Treatment on culture plates.

1 mg/ml of anti-human CD3 antibody and 0.5 mg/ml of anti-human CD28 antibody were diluted into a suitable amount of PBS buffer at a volume ratio of 1:1000, and retronectin (1 mg/ml) reagent was diluted into the PBS buffer at a volume ratio of 1:40. They were mixed uniformly and then plated into the cell dishes evenly, incubated at room temperature for 2 hours, and washed with PBS after 2 hours for later use.

### 3) Lentiviral infection of T cells

The activated T cells were diluted with the infection reagents formulated in 1), into which were added lentiviruses following MOI=3, mixed uniformly, and evenly plated in the culture dishes treated in 2).

After infection, the cell density was monitored to maintain the cells at 1^{∗}10⁶ cells/ml, which can be amplified by 30-1000 fold generally after 14 days.

### Example 3. Detection of memory T cells by flow cytometry (GPC3)

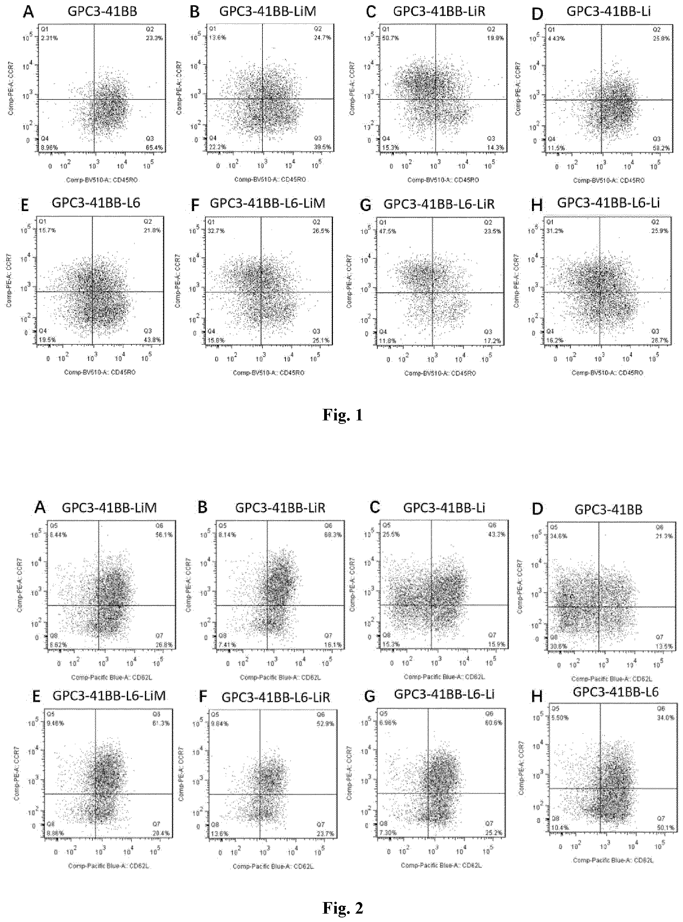

T cells expressing GPC3-41BB, GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR were cultured in vitro, and the expression of memory-related proteins such as CD45RO, CCR7, CD62L in T cells was detected by a BD Canto2 flow cytometer. As shown in Figs. 1A-1H and Figs. 2A-2H, the proportions of memory stem cell-like T cells (TSCMs) and central memory T cells (TCMs) (CD45RO-CCR7+; CCR7+CD62L+) in the total T cells expressing GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR were all significantly higher than that in the GPC3-41BB control group, and GPC3-41BB-LiR, GPC3-41BB-L6-Li, GPC3-41BB-L6-LiM, GPC3-41BB-L6-LiR were the most significant.

### Example 4. Detection of memory T cells by flow cytometry (BCMA)

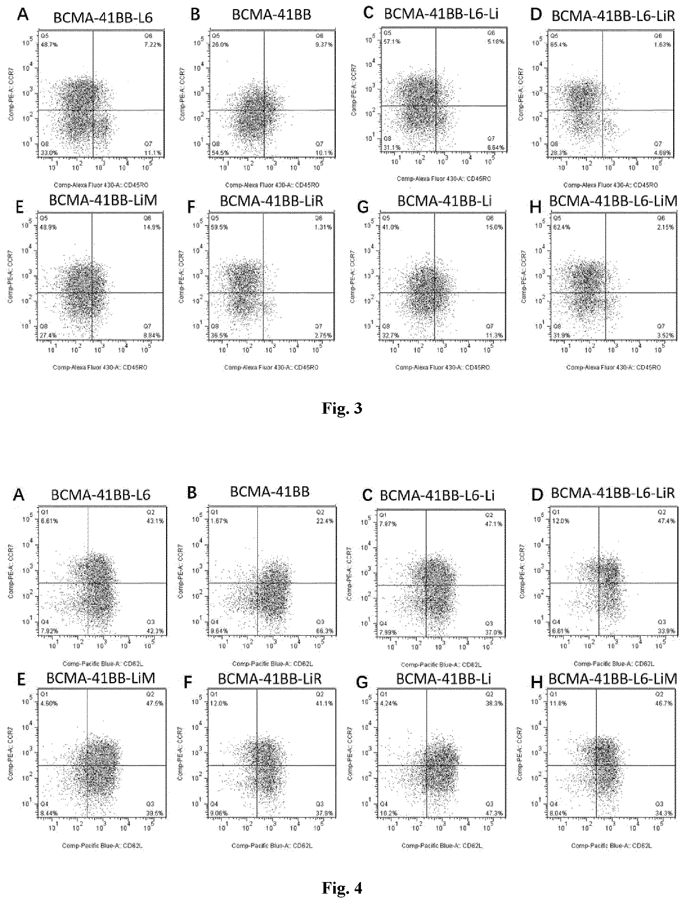

T cells expressing BCMA-41BB, BCMA-41BB-L6, BCMA-41BB-Li, BCMA-41BB-LiM, BCMA-41BB-LiR, BCMA-41BB-L6-Li, BCMA-41BB-L6-LiM, BCMA-41BB-L6-LiR were cultured in vitro, and the expression of memory-related proteins such as CD45RO, CCR7, CD62L in T cells was detected by a BD Canto2 flow cytometer. As shown in Figs. 3A-3H and Figs. 4A-4H, the proportions of memory stem cell-like T cells (TSCMs) and central memory T cell (TCMs) (CD45RO-CCR7+; CCR7+CD62L+) in the total T cells expressing BCMA-41BB-L6, BCMA-41BB-Li, BCMA-41BB-LiM, BCMA-41BB-LiR, BCMA-41BB-L6-Li, BCMA-41BB-L6-LiM, BCMA-41BB-L6-LiR were all significantly higher than that in the BCMA-41BB control group.

### Example 5. Detection of memory T cells in CD4 cells by flow cytometry

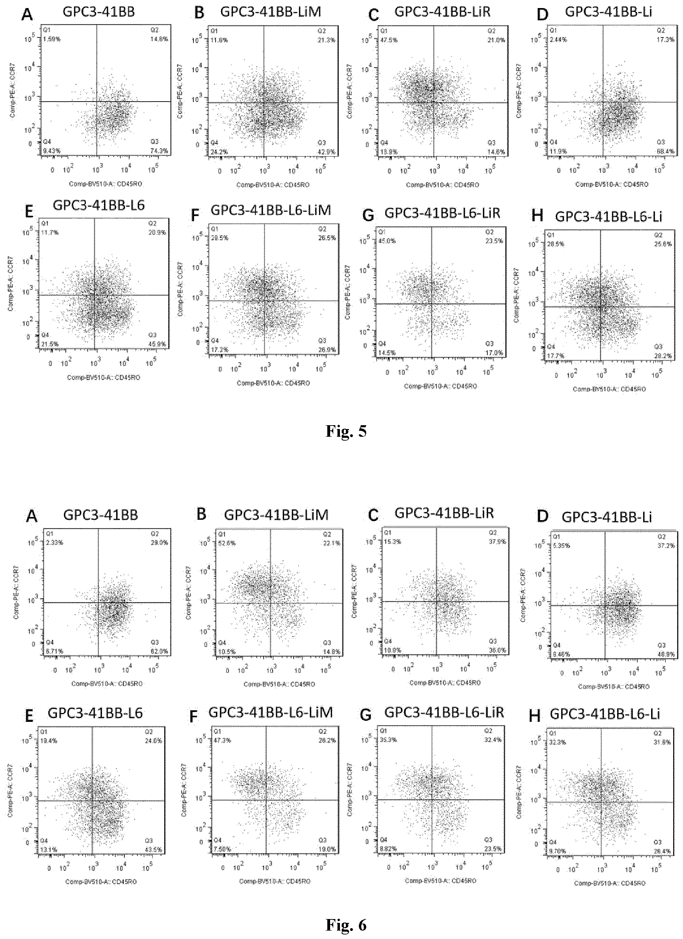

T cells expressing GPC3-41BB, GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR were cultured in vitro, and the expression of CD45RO protein in T cells was detected by a BD Canto2 flow cytometer. As shown in Figs. 5A-5H, the proportions of memory stem cell-like T cells (TSCMs) (CD45RO-CCR7+) in the total CD4 T cells expressing GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR were all significantly higher than that in the GPC3-41BB control group.

### Example 6. Detection of memory T cells in CD8 cells by flow cytometry

T cells expressing GPC3-41BB, GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR were cultured in vitro, and the expression of CD3, CD4, CD8, CD45RO, CD45RA, CD62L, CCR7, CD95, CD122, CD127, CD27, CD28 proteins in T cells was detected by a BD Canto2 flow cytometer. As shown in Figs. 6A-6H, the proportions of memory stem cell-like T cells (TSCMs) (CD45RO-CCR7+) in the total CD8 T cells expressing GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR were all significantly higher than that in the GPC3-41BB control group.

### Example 7: Detection on the expression of negative regulatory proteins in T cells by flow cytometry

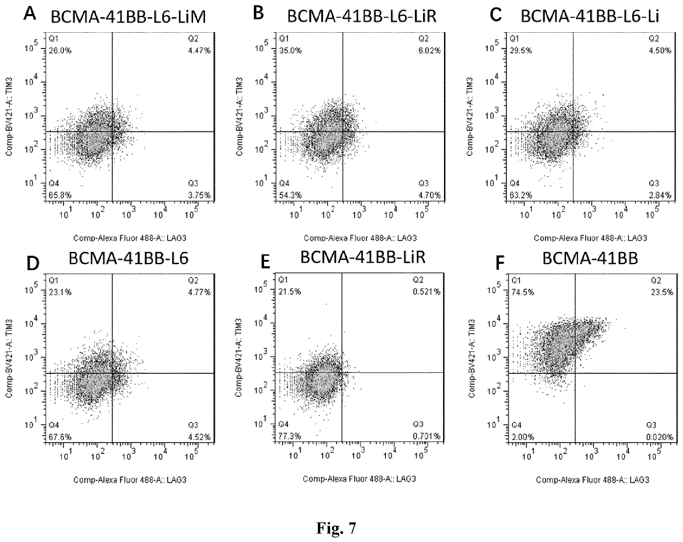

T cells expressing BCMA-41BB, BCMA-41BB-L6, BCMA-41BB-Li, BCMA-41BB-LiM, BCMA-41BB-LiR, BCMA-41BB-L6-Li, BCMA-41BB-L6-LiM, BCMA-41BB-L6-LiR were cultured in vitro, and the expression of CD3, CD4, CD8, PD1, PDL1, TIM3, LAG3 proteins in T cells was detected by a BD Canto2 flow cytometer. As shown in Figs. 7A-7F, the expression of negative regulatory proteins in T cells expressing BCMA-41BB-L6, BCMA-41BB-Li, BCMA-41BB-LiM, BCMA-41BB-LiR, BCMA-41BB-L6-Li, BCMA-41BB-L6-LiM, BCMA-41BB-L6-LiR was significantly lower than that in the BCMA-41BB control group.

### Example 8: Detection of T cell amplification

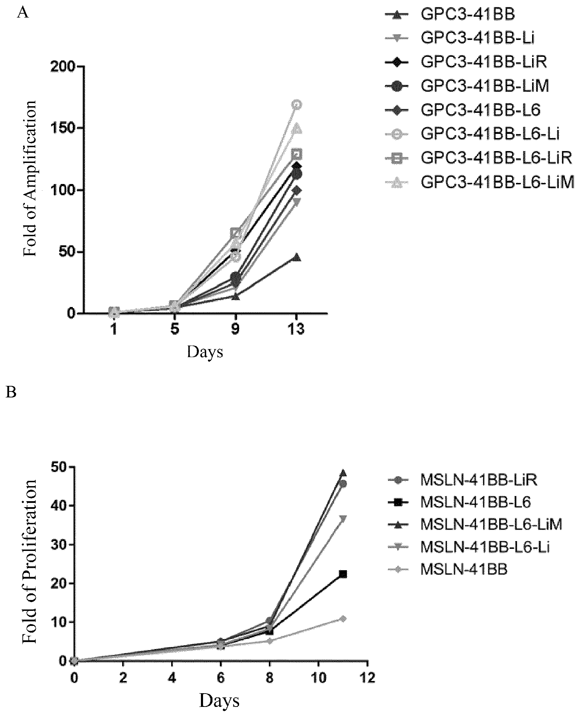

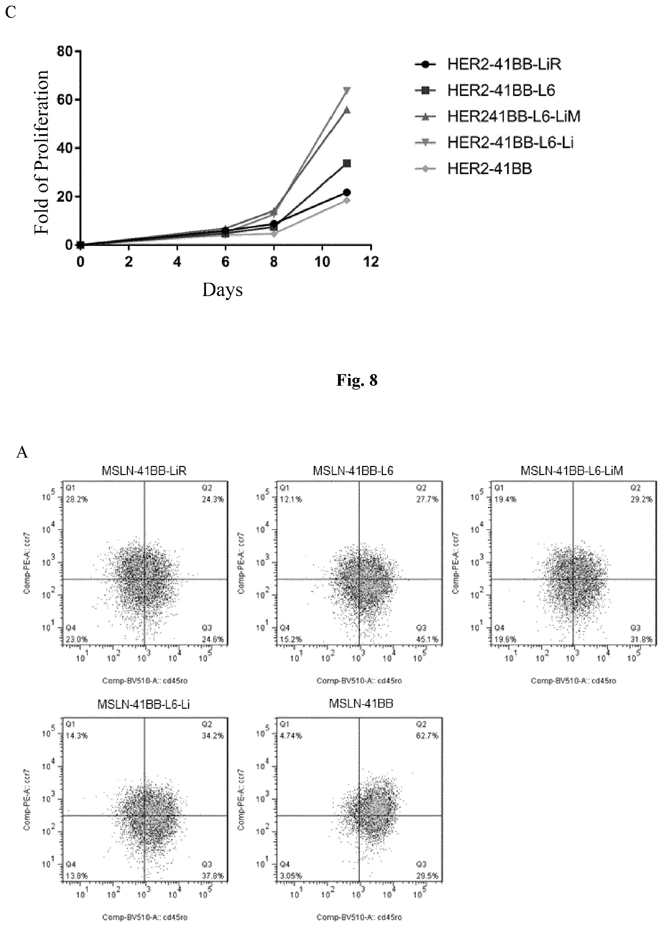

T cells expressing GPC3-41BB, GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR, MSLN-41BB, MSLN-41BB-L6, MSLN-41BB-LiR, MSLN-41BB-L6-Li, MSLN-41BB-L6-LiM, HER2-41BB, HER2-41BB-L6, HER2-41BB-LiR, HER2-41BB-L6-Li, HER2-41BB-L6-LiM were cultured in vitro, stained with trypan blue (GIBICO) and then counted by a Cellometer Mini automatic cell counter to record the amplification capacity of the cells. As shown in Figs. 8A-8C, the amplification capacities of T cells expressing GPC3-41BB-L6, GPC3-41BB-Li, GPC3-41BB-LiM, GPC3-41BB-LiR, GPC3-41BB-L6-Li, GPC3-41BB-L6-LiM, GPC3-41BB-L6-LiR, MSLN-41BB-L6, MSLN-41BB-LiR, MSLN-41BB-L6-Li, MSLN-41BB-L6-LiM, HER2-41BB-L6, HER2-41BB-LiR, HER2-41BB-L6-Li, HER2-41BB-L6-LiM were all significantly higher than that in the GPC3-41BB, MSLN-41BB and HER2-41BB control groups.

### Example 9. Detection of memory T cells by flow cytometry (MSLN)

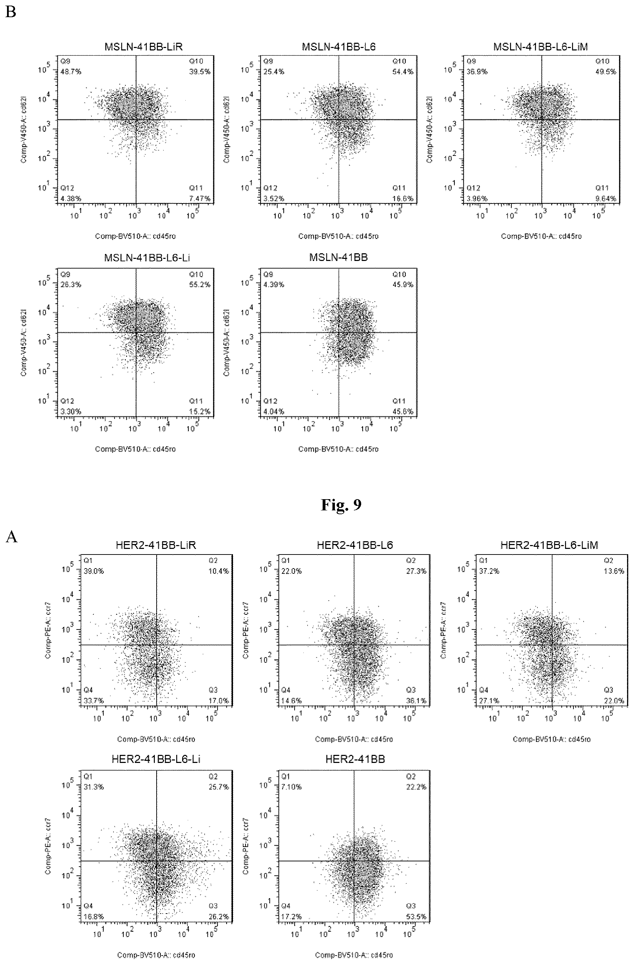

T cells expressing MSLN-41BB, MSLN-41BB-L6, MSLN-41BB-LiR, MSLN-41BB-L6-Li, MSLN-41BB-L6-LiM were cultured in vitro, and the expression of memory-related proteins such as CD45RO, CCR7, CD62L in T cells was detected by flow cytometry. As shown in Fig. 9A and Fig. 9B, the proportions of memory stem cell-like T cells (TSCMs) (CD45RO-CCR7+; CD45RO-CD62L+) in the toal T cells expressing MSLN-41BB-L6, MSLN-41BB-LiR, MSLN-41BB-L6-Li, MSLN-41BB-L6-LiM were all significantly higher than that in the MSLN-41BB control group, especially those in the MSLN-41BB-LiR and MSLN-41BB-L6-LiM groups.

### Example 10. Detection of memory T cells by flow cytometry (HER2)

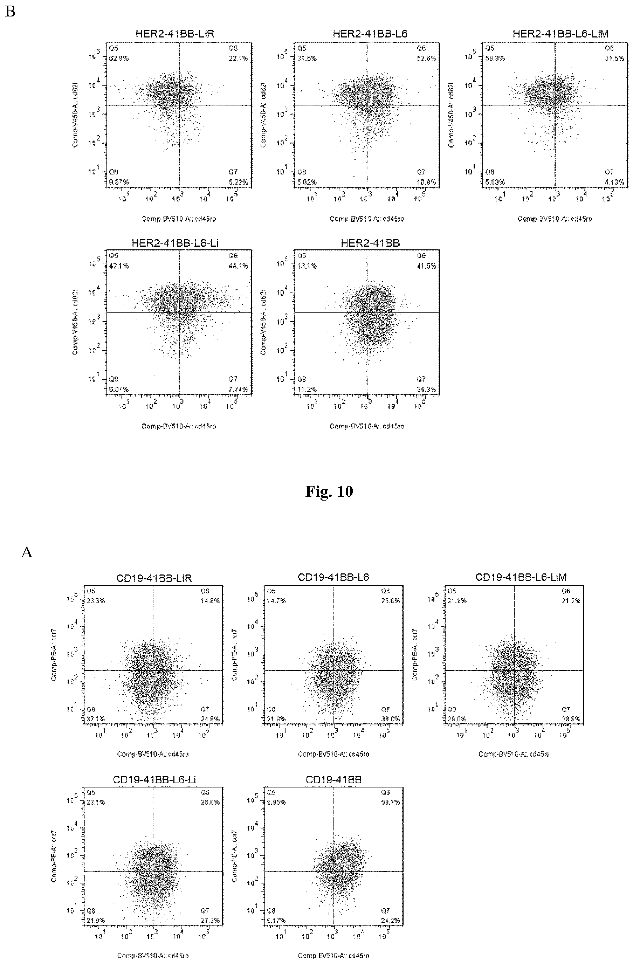

T cells expressing HER2-41BB, HER2-41BB-L6, HER2-41BB-LiR, HER2-41BB-L6-Li, HER2-41BB-L6-LiM were cultured in vitro, and the expression of memory-related proteins such as CD45RO, CCR7, CD62L in T cells was detected by flow cytometry. As shown in Fig. 10A and Fig. 10B, the proportions of memory stem cell-like T cells (TSCMs) (CD45RO-CCR7+; CD45RO-CD62L+) in the total T cells expressing HER2-41BB-L6, HER2-41BB-LiR, HER2-41BB-L6-Li, HER2-41BB-L6-LiM were all significantly higher than that in the HER2-41BB control group, especially those in the HER2-41BB-LiR, HER2-41BB-L6-Li and HER2-41BB-L6-LiM groups.

### Example 11. Detection of memory T cells by flow cytometry (CD19)

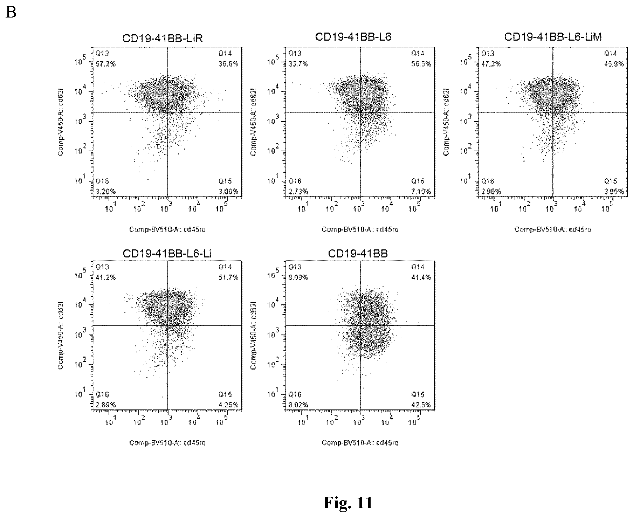

T cells expressing CD19-41BB, CD19-41BB-L6, CD19-41BB-LiR, CD19-41BB-L6-Li, CD19-41BB-L6-LiM were cultured in vitro, and the expression of memory-related proteins such as CD45RO, CCR7, CD62L in T cells was detected by flow cytometry. As shown in Fig. 11A and Fig. 11B, the proportions of memory stem cell-like T cells (TSCMs) (CD45RO-CCR7+; CD45RO-CD62L+) in the total T cells expressing CD19-41BB-L6, CD19-41BB-LiR, CD19-41BB-L6-Li, CD19-41BB-L6-LiM were all significantly higher than that in the CD19-41BB control group, especially those in the CD19-41BB-LiR, CD19-41BB-L6-Li and CD19-41BB-L6-LiM groups.

### Example 12. Detection by repeated stimulation (MSLN, L1H2 [GPC3-targeted], HER2, CD19)

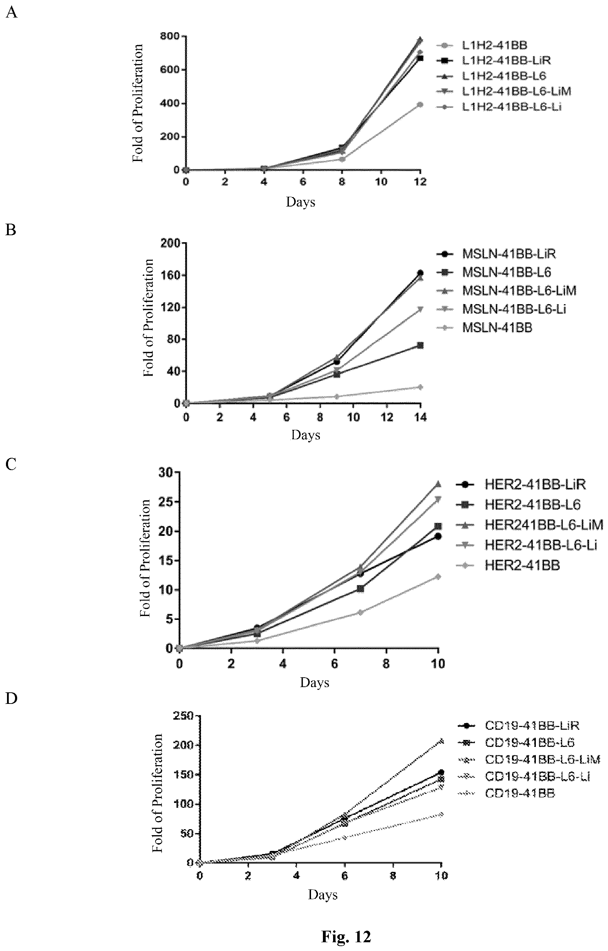

T cells expressing L1H2-41BB, L1H2-41BB-L6, L1H2-41BB-LiR, L1H2-41BB-L6-Li, L1H2-41BB-L6-LiM, MSLN-41BB, MSLN-41BB-L6, MSLN-41BB-LiR, MSLN-41BB-L6-Li, MSLN-41BB-L6-LiM, HER2-41BB, HER2-41BB-L6, HER2-41BB-LiR, HER2-41BB-L6-Li, HER2-41BB-L6-LiM, CD19-41BB, CD19-41BB-L6, CD19-41BB-LiR, CD19-41BB-L6-Li, CD19-41BB-L6-LiM were cultured in vitro, for which the positive rates were detected on day 9, and then the positive rates were adjusted with uninfected T cells to consistent. 3×10e5 CAR-positive cells and 3×10e5 irradiated target cells were taken for the first round of co-incubation stimulation (the corresponding target cells of L1H2 CAR-T are Huh7, the corresponding target cells of MSLN CAR-T are OVCAR3, the corresponding target cells of HER2 CAR-T are SKOV3, and the corresponding target cells of CD19 CAR-T are Raji), and counted every 3-5 days. And 3×10e5 total cells and 3×10e5 irradiated target cells were taken for the second round of co-incubation stimulation, the above operation was repeated after 3-5 days for the third round of co-incubation stimulation, and the above operation was repeated after 3-5 days for the fourth round of co-incubation stimulation. The counting results were converted to form a repeated stimulation amplification curve. As shown from the results in Figs. 12A-12D, the capacities of amplification by repeated stimulation in the L6, LiR, L6-Li, L6-LiM structural groups were all significantly higher than that in the 41BB control group, indicating better amplification capacity and tumor treatment ability in vivo.

### Example 13. CAR-T tumor suppression and elimination experiment (GPC3-targeted)

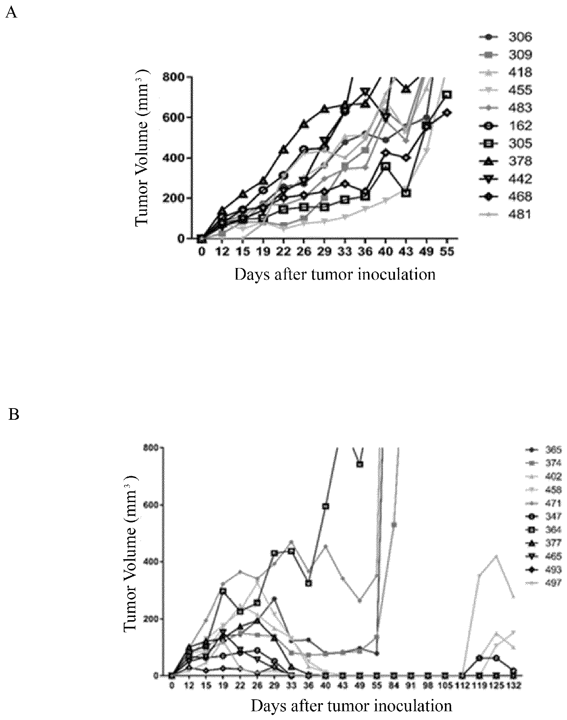

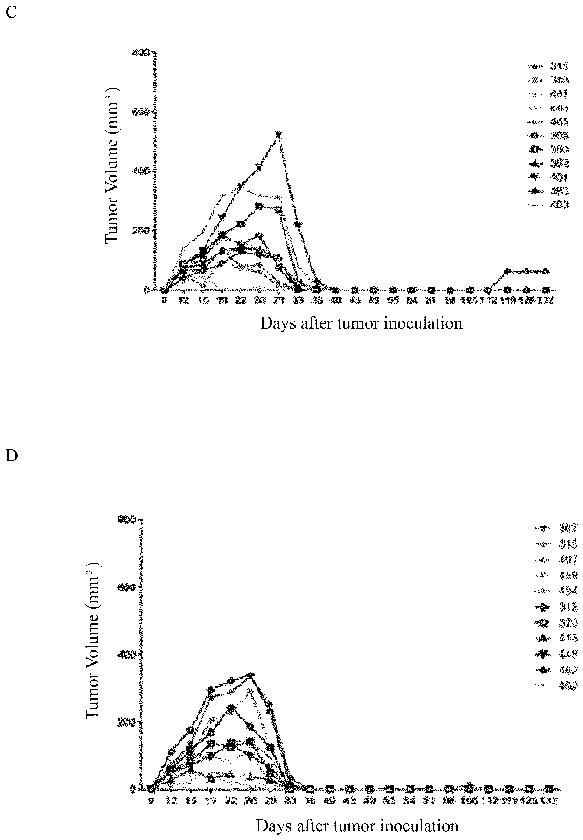

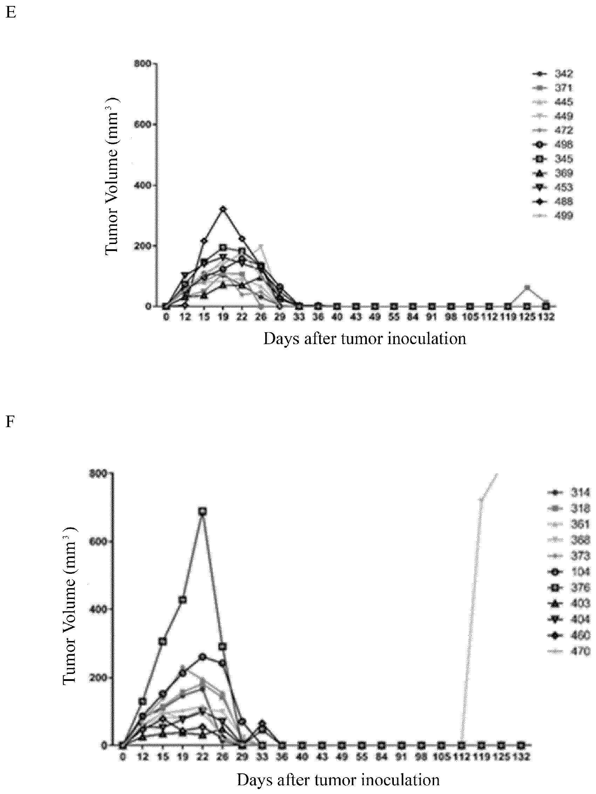

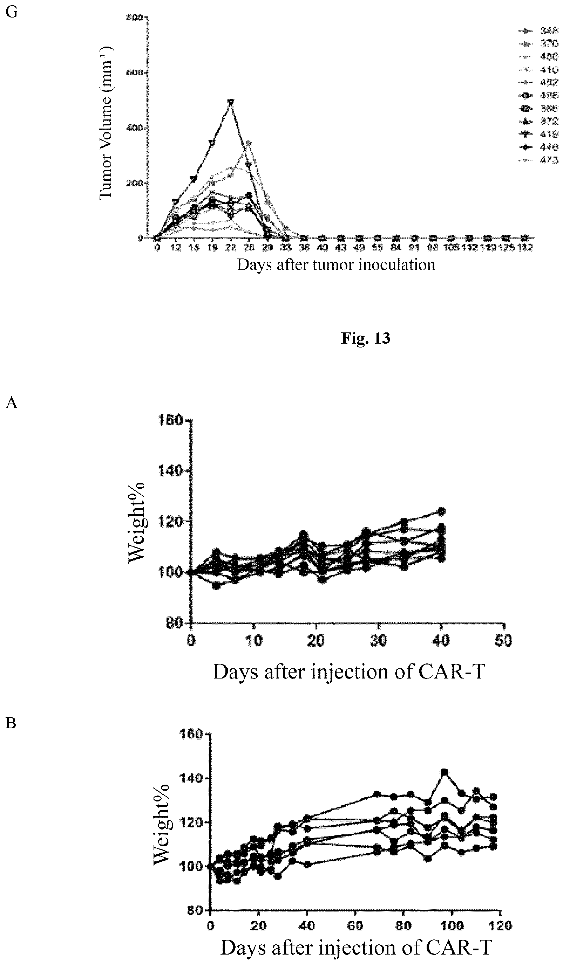

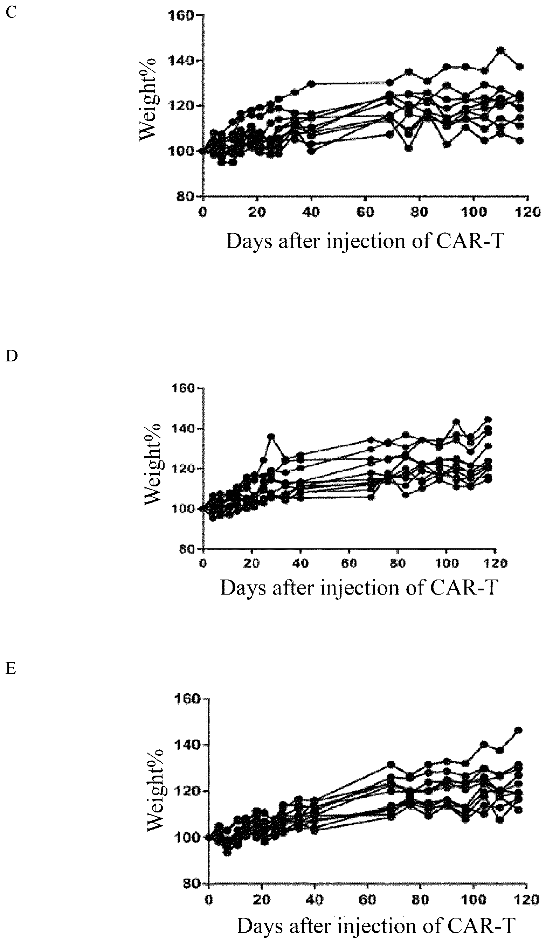

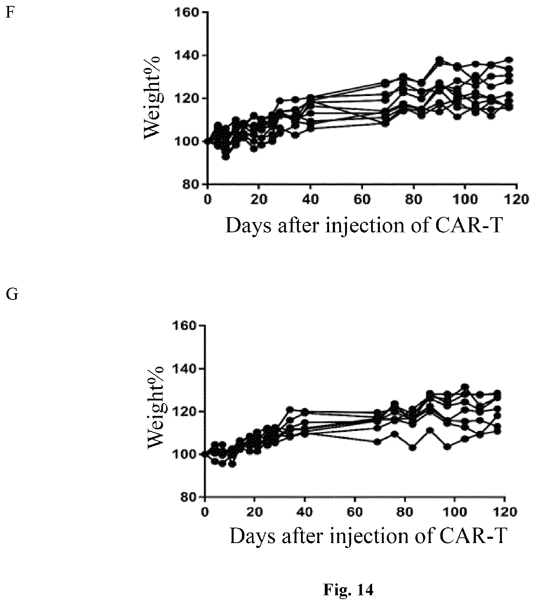

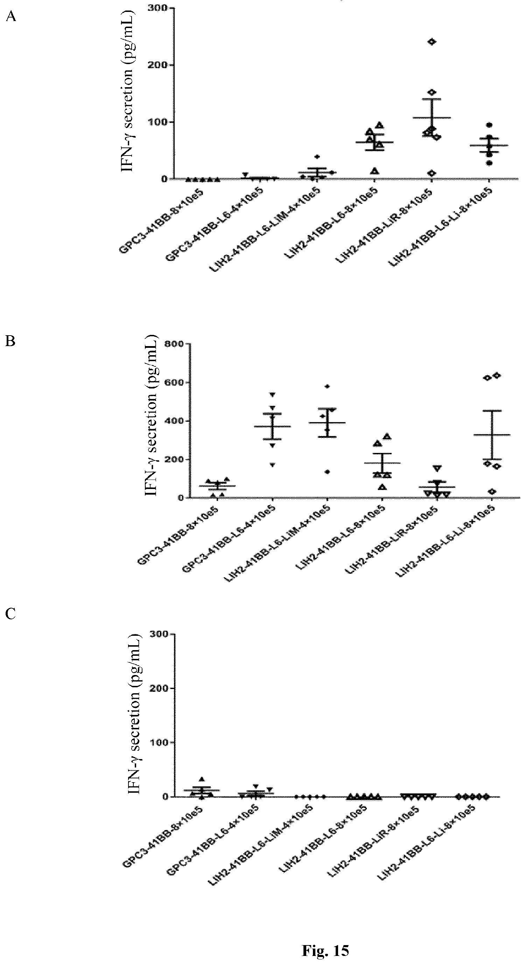

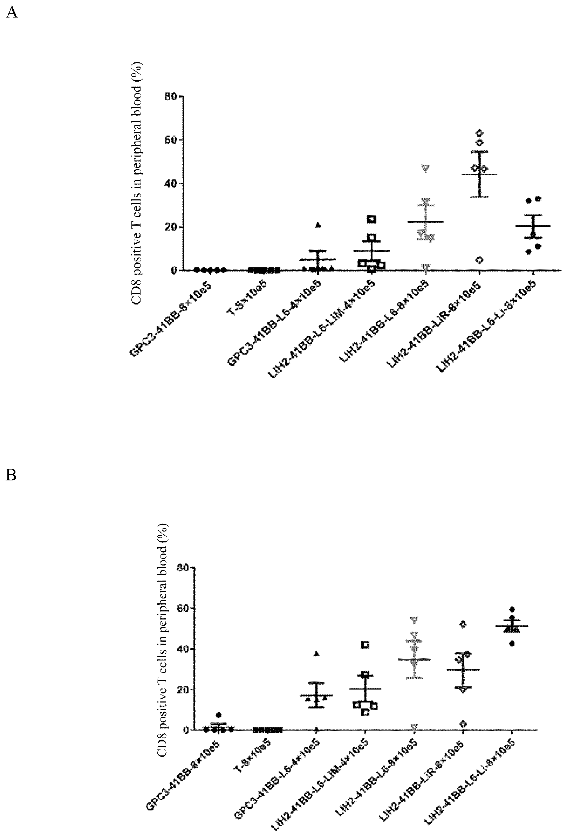

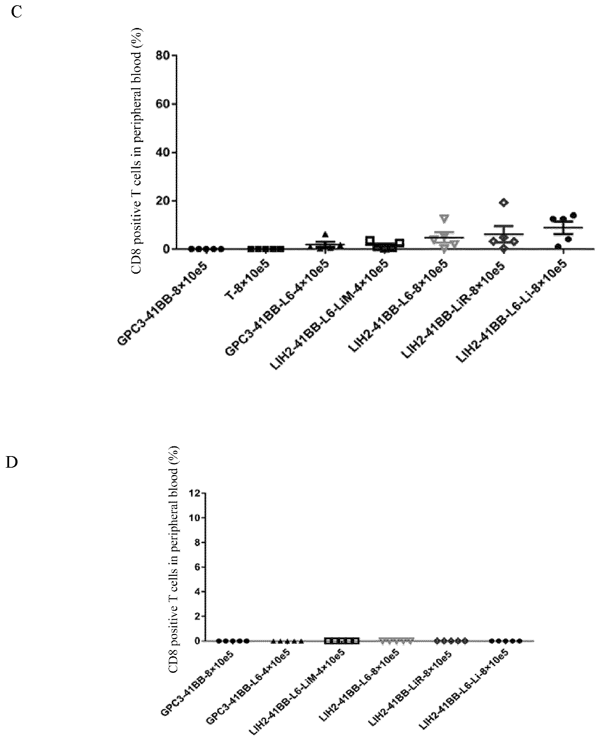

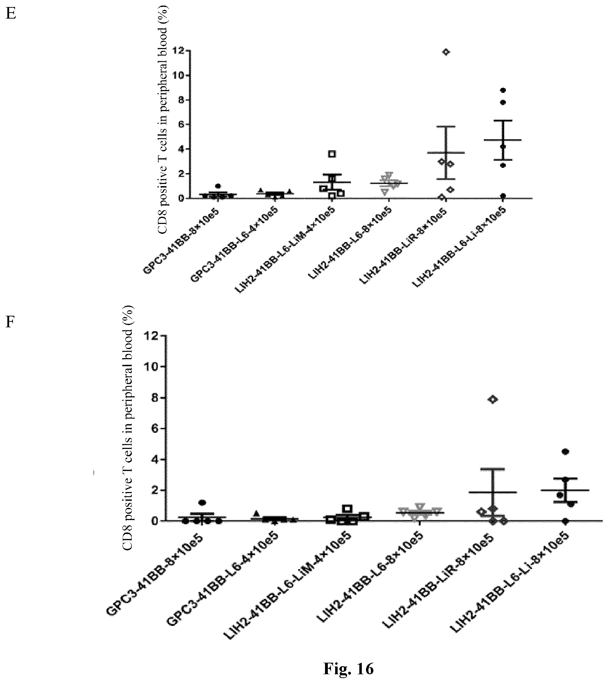

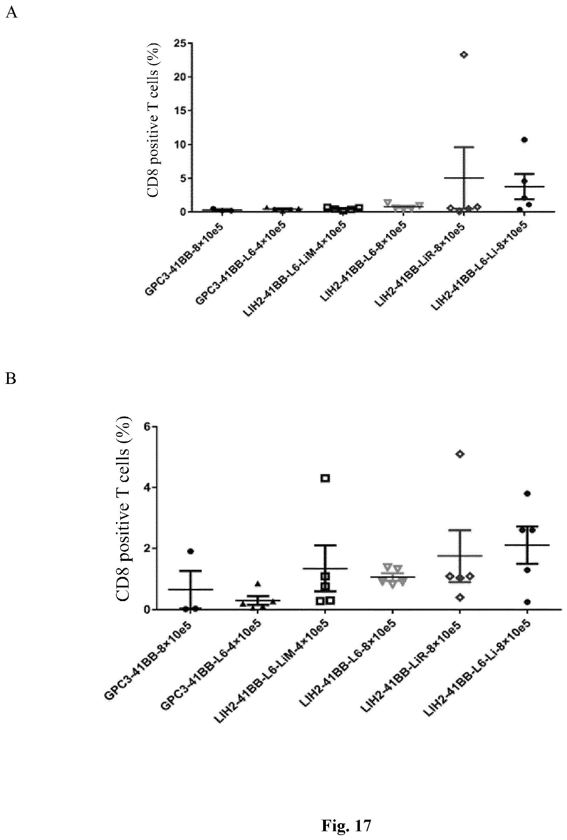

T cells expressing GPC3-41BB, GPC3-41BB-L6, L1H2-41BB-L6, L1H2-41BB-LiR, L1H2-41BB-L6-Li, L1H2-41BB-L6-LiM and T cells not infected with CAR-T were subjected to a single tail vein injection of CAR-T into 6-week-old NSG mice with subcutaneous tumor-bearing of Huh7 tumors (1×10e7/mouse) to 80-150 mm³ size at doses of 4×10e5 and 8×10e5 CAR-positive cells, with 11 mice in each group. The body weight (Figs. 14A-14G show the body weight changes of mice that have been injected with 8×10e5 T cells uninfected with CAR-T, T cells infected with 8×10e5 GPC3-41BB, T cells infected with 4×10e5 GPC3-41BB-L6, T cells infected with 4×10e5 L1H2-41BB-L6-LiM, T cells infected with 8×10e5 L1H2-41BB-L6, T cells infected with 8×10e5 L1H2-41BB-LiR, T cells infected with 8×10e5 L1H2-41BB-L6-Li, respectively) and the tumor sizes (Figs. 13A-13G show the tumor sizes of mice that have been injected with T cells not infected with 8×10e5 CAR-T, T cells infected with 8×10e5 GPC3-41BB, T cells infected with 4×10e5 GPC3-41BB-L6, T cells infected with 4×10e5 L1H2-41BB-L6-LiM, T cells infected with 8×10e5 L1H2-41BB-L6, T cells infected with 8×10e5 L1H2-41BB-LiR, T cells infected with 8×10e5 L1H2-41BB-L6-Li, respectively) were measured twice a week. On days 4, 11, 17 after the injection of CAR-T, five mice were randomly selected from each group to take anticoagulated blood (heparin sodium anticoagulation) from the tail vein for the detection of IFNg in blood (BD human th1/th2 CBAkit) (Figs. 15A-15C show the results on days 4, 11, 17 in order). On days 11, 17, 26 after the injection of CAR-T, five mice were randomly selected from each group to take anticoagulated blood (heparin sodium anticoagulation) from the tail vein for the detection of human CD8 cell content in blood by flow cytometry (Figs. 16A-16C show the results on days 11, 17, 26 in order). On day 85 after the injection of CAR-T (day 98 after the injection of tumor), the other side of the back of the mice without recurrence after tumor elimination was subjected to a secondary tumor-bearing of Huh7 (1×10e7/mouse) to simulate tumor recurrence, and the tumor formation was observed (Fig. 13). On days 91, 98, 105, five mice were randomly selected from each group of secondary tumor-bearing mice to take anticoagulated blood (heparin sodium anticoagulation) from the tail vein for the detection of human CD8 cell content in blood by flow cytometry (Figs. 16D-16F show the results on days 91, 98, 105 in order). On day 132, five mice were randomly selected from each group of secondary tumor-bearing mice to take spleen and bone marrow for the detection of human CD8 cell content by flow cytometry (Figs. 17A-17B), the experiment is over. The experimental results show that, compared with the GC33-41BB control group, the groups of GPC3-41BB-L6, L1H2-41BB-L6, L1H2-41BB-LiR, L1H2-41BB-L6-Li, L1H2-41BB-L6-LiM all show more significant tumor suppression and elimination effects and anti-recurrence ability (Fig. 13), while none of them show any obvious toxic side effects (Fig. 14, no obvious weight loss); the results of in vivo cytokine assay show that the groups of GPC3-41BB-L6, L1H2-41BB-L6, L1H2-41BB-LiR, L1H2-41BB-L6-Li, L1H2-41BB-L6-LiM all have higher factor secretion, which is beneficial to promote the in vivo amplification of CAR-T, wherein L1H2-41BB-LiR and L1H2-41BB-L6-Li are the most significant (Figs. 15A-15C); the results of in vivo cell proliferation assay show that the groups of GPC3-41BB-L6, L1H2-41BB-L6, L1H2-41BB-LiR, L1H2-41BB-L6-Li, L1H2-41BB-L6-LiM all have higher cell proliferation capability, wherein L1H2-41BB-LiR and L1H2-41BB-L6-Li are the most significant (Figs. 16A-16C). In addition, after secondary tumor-bearing, the levels of in vivo CAR-T cells that have restored to the baseline value in the groups of L1H2-41BB-L6, L1H2-41BB-LiR, L1H2-41BB-L6-Li, L1H2-41BB-L6-LiM have obvious secondary proliferation, which is positively correlated with its better anti-recurrence ability, wherein L1H2-41BB-LiR and L1H2-41BB-L6-Li are the most significant (Figs. 16D-16F). Meanwhile, the spleen and bone marrow assay after secondary tumor-bearing show that the groups of L1H2-41BB-L6, L1H2-41BB-LiR, L1H2-41BB-L6-Li, L1H2-41BB-L6-LiM have more memory T cells homing to lymph nodes, wherein L1H2-41BB-L6-Li is the most significant.

### Example 14. CAR-T tumor suppression and elimination experiment (BCMA-targeted)

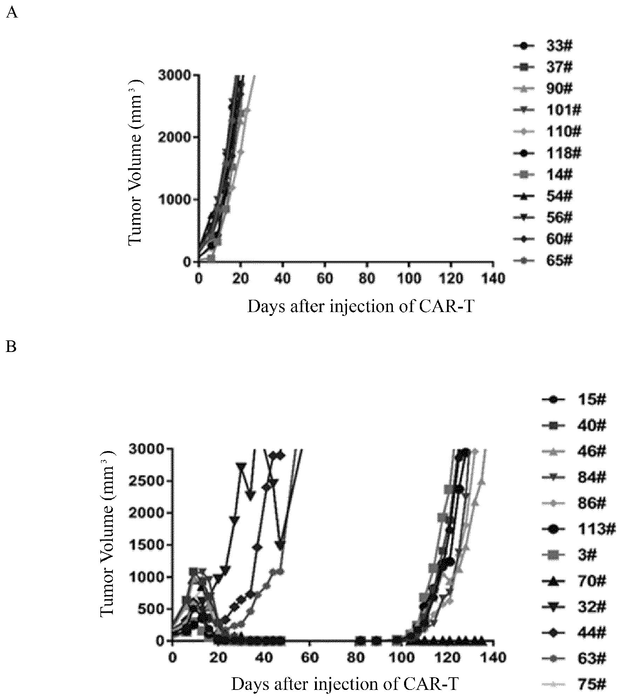

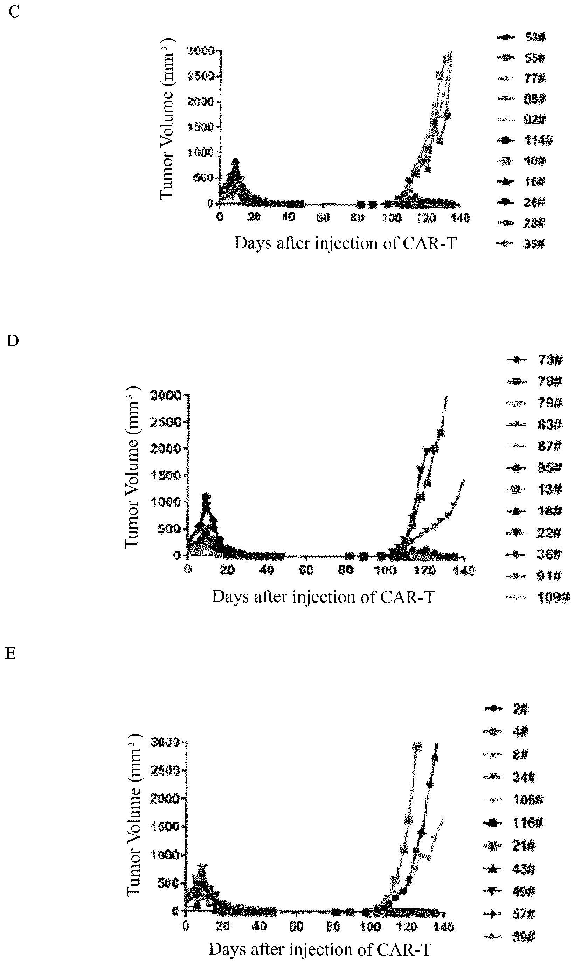

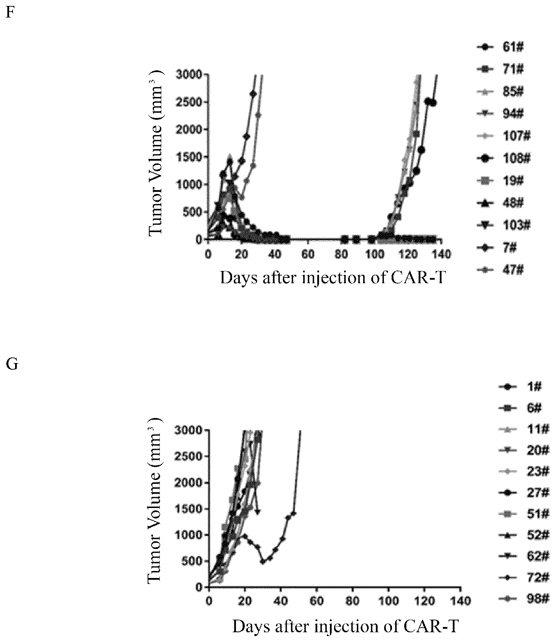

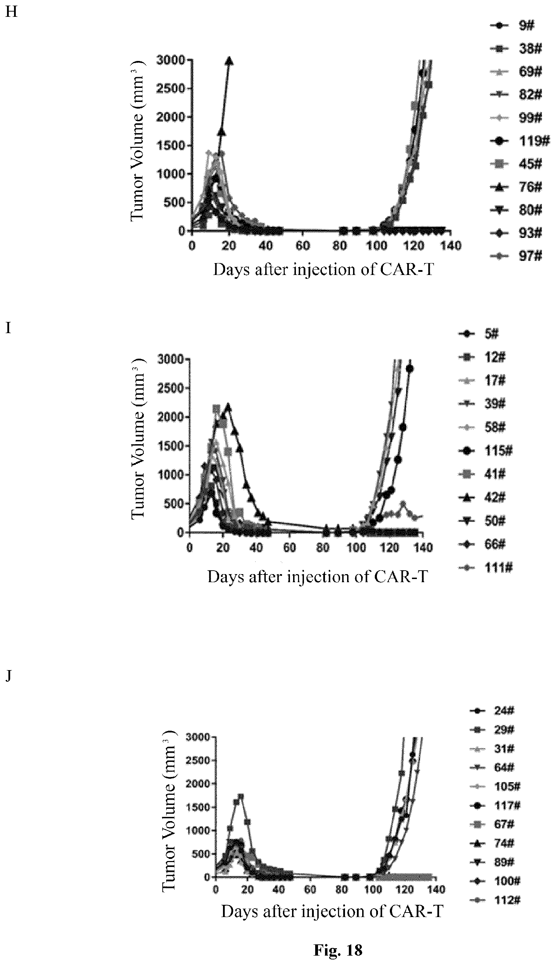

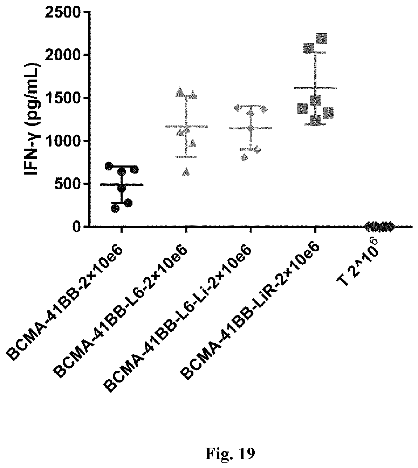

T cells expressing BCMA-41BB, BCMA-41BB-L6, BCMA-41BB-LiR, BCMA-41BB-L6-Li, BCMA-41BB-L6-LiM and T cells not infected with CAR-T were subjected to a single tail vein injection of CAR-T into 6-week-old NSG mice with subcutaneous tumor-bearing of MM1.S tumors (1×10e7/mouse) to 80-150 mm³ size at doses of 6×10e5 and 2×10e6 CAR-positive cells, with 11 mice in each group. The tumor size was measured twice a week (Figs. 18A-18J). On day 8 after the injection of CAR-T, six mice were randomly selected from each 2×10e6 dose group to take anticoagulated blood (heparin sodium anticoagulation) from the tail vein for the detection of IFNg in blood (BD human th1/th2 CBAkit) (Fig. 19). On day 80 after the injection of CAR-T, the other side of the back of the mice without recurrence after tumor elimination was subjected to a secondary tumor-bearing of MM1.S (1×10e7/mouse) to simulate tumor recurrence, and the tumor formation was observed (Figs. 18B-18J). The results of the control group inoculated with T cells not infected with CAR-T are shown in Fig. 18A. The experimental results show that, compared with the control group inoculated with 2×10e6 BCMA-41BB positive cells (Fig. 18B) and the control group inoculated with 6×10e5 BCMA-41BB positive cells (Fig. 18 G), the experimental groups inoculated with 2×10e6 BCMA-41BB-L6 positive cells (Fig. 18C) and inoculated with 6×10e5 BCMA-41BB-L6 positive cells (Fig. 18H), the experimental groups inoculated with 2×10e6 BCMA-41BB-LiR positive cells (Fig. 18E) and inoculated with 6×10e5 BCMA-41BB-LiR positive cells (Fig. 18J), the experimental groups inoculated with 2×10e6 BCMA-41BB-L6-Li positive cells (Fig. 18D) and inoculated with 6×10e5 BCMA-41BB-L6-Li positive cells (Fig. 181), and the experimental group inoculated with 6×10e5 BCMA-41BB-L6-LiM positive cells (Fig. 18F) all show more significant tumor suppression and elimination effects and anti-recurrence ability; the results of in vivo cytokine assay show that compared with BCMA-41BB group, the groups of BCMA-41BB-L6, BCMA-41BB-LiR, BCMA-41BB-L6-Li all have higher factor secretion, which is beneficial to promote the in vivo amplification of CAR-T, wherein the BCMA-41BB-LiR group is the most significant (Fig. 19). In addition, after secondary tumor-bearing, significant recurrence occurred in all of the groups due to the high tumor-bearing doses, but the numbers of recurrence in various dose groups of BCMA-41BB-L6, BCMA-41BB-LiR, BCMA-41BB-L6-Li, BCMA-41BB-L6-LiM were all significantly lower than that in the high dose group of BCMA-41BB; in low dose groups, BCMA-41BB-L6-Li, BCMA-41BB-L6-LiM are slightly better than BCMA-41BB-LiR, and significantly better than BCMA-41BB-L6, while a low dose of BCMA-41BB cannot suppress tumors at all.

The foregoing detailed description is provided by way of explanation and examples, and is not intended to limit the scope of the appended claims. Various changes of the embodiments currently enumerated in this application will be apparent to those of ordinary skills in the art, and are reserved within the scope of the appended claims and their equivalents.

## Claims

1. A modified immune cell, comprising a chimeric antigen receptor and/or a coding element therefor, or comprising a T cell receptor and/or a coding element therefor, and said modified immune cell further comprises:
a leptin and/or a functional fragment thereof; and/or,
a leptin receptor and/or a functional fragment thereof, and the expression quantity of said leptin receptor and/or the functional fragment thereof in said modified immune cell is increased compared with that in an immune cell without the corresponding modification.

2. The modified immune cell according to claim 1, wherein said modified immune cell comprises a lymphocyte.

3. The modified immune cell according to any one of claims 1-2, wherein said modified immune cell comprises a T cell.

4. The modified immune cell according to any one of claims 1-3, wherein said leptin and/or the functional fragment thereof is derived from human.

5. The modified immune cell according to any one of claims 1-4, wherein said leptin receptor and/or the functional fragment thereof is derived from human.

6. The modified immune cell according to any one of claims 1-5, wherein said leptin is a secretory leptin or a membrane-bound leptin.

7. The modified immune cell according to any one of claims 1-6, wherein said leptin and/or the functional fragment thereof comprises an amino acid sequence as set forth in any one of SEQ ID NOs: 13-14.

8. The modified immune cell according to any one of claims 1-7, wherein the functional fragment of said leptin receptor comprises a domain of said leptin receptor selected from a group consisting of: an intracellular region, a BOX 1 region, an FN3 region, a C2 region and a transmembrane region.

9. The modified immune cell according to any one of claims 1-8, wherein said leptin receptor and/or the functional fragment thereof comprises an amino acid sequence as set forth in any one of SEQ ID NOs: 15-17.

10. The modified immune cell according to any one of claims 1-9, wherein said chimeric antigen receptor does not comprise said leptin and/or the functional fragment thereof.

11. The modified immune cell according to any one of claims 1-10, wherein said chimeric antigen receptor does not comprise said leptin receptor and/or the functional fragment thereof.

12. The modified immune cell according to any one of claims 1-11, wherein said chimeric antigen receptor comprises an intracellular domain, said intracellular domain comprises a signaling domain and/or a costimulatory domain.

13. The modified immune cell according to claim 12, wherein said signaling domain comprises a signaling domain of CD3zeta.

14. The modified immune cell according to any one of claims 12-13, wherein said costimulatory domain comprises a costimulatory domain of 4-1BB.

15. The modified immune cell according to any one of claims 1-14, wherein said chimeric antigen receptor comprises a hinge region.

16. The modified immune cell according to claim 15, wherein said hinge region comprises a hinge region of CD8.

17. The modified immune cell according to any one of claims 1-16, wherein said chimeric antigen receptor comprises a transmembrane region.

18. The modified immune cell according to claim 17, wherein said transmembrane region comprises a transmembrane region of CD8.

19. The modified immune cell according to any one of claims 1-18, wherein said chimeric antigen receptor comprises a targeting moiety.

20. The modified immune cell according to claim 19, wherein said targeting moiety comprises a scFv.

21. The modified immune cell according to any one of claims 19-20, wherein said targeting moiety specifically binds to and/or recognizes a tumor antigen.

22. The modified immune cell according to any one of claims 19-21, wherein said targeting moiety specifically binds to and/or recognizes a target selected from a group consisting of: CD19, HER2, Mesothelin, GPC3 and BCMA.

23. The modified immune cell according to any one of claims 1-22, wherein said chimeric antigen receptor comprises an amino acid sequence as set forth in any one of SEQ ID NOs: 23-24.

24. A pharmaceutical composition, comprising the modified immune cell according to any one of claims 1-23, and optionally a pharmaceutically acceptable carrier.

25. A use of the modified immune cell according to any one of claims 1-23 or the pharmaceutical composition according to claim 24 in the preparation of a drug for treating tumor.

26. A method for promoting immune cell proliferation, comprising steps of:
1) providing a modified immune cell comprising a chimeric antigen receptor and/or a coding element therefor, or comprising a T cell receptor and/or a coding element therefor; and
2) allowing said modified immune cell of 1) to express leptin and/or a functional fragment thereof, and/or increasing the expression quantity of a leptin receptor and/or a functional fragment thereof in said modified immune cell of 1).

27. A method for promoting the production of memory immune cells, comprising steps of:
1) providing a modified immune cell comprising a chimeric antigen receptor and/or a coding element therefor, or comprising a T cell receptor and/or a coding element therefor; and
2) allowing said modified immune cell of 1) to express leptin and/or a functional fragment thereof, and/or increasing the expression quantity of a leptin receptor and/or a functional fragment thereof in said modified immune cell of 1).

28. A method for enhancing the killing ability of an immune cell against tumor, comprising steps of:
1) providing a modified immune cell comprising a chimeric antigen receptor and/or a coding element therefor, or comprising a T cell receptor and/or a coding element therefor; and
2) allowing said modified immune cell of 1) to express leptin and/or a functional fragment thereof, and/or increasing the expression quantity of a leptin receptor and/or a functional fragment thereof in said modified immune cell of 1).

29. A method for inhibiting an immune cell from expressing an immune negative regulatory protein, comprising steps of:
1) providing a modified immune cell comprising a chimeric antigen receptor and/or a coding element therefor, or comprising a T cell receptor and/or a coding element therefor; and
2) allowing said modified immune cell of 1) to express leptin and/or a functional fragment thereof, and/or increasing the expression quantity of a leptin receptor and/or a functional fragment thereof in said modified immune cell of 1).

30. The method according to any one of claims 26-29, wherein 2) comprises:
introducing a polynucleotide encoding said leptin and/or the functional fragment thereof into said modified immune cell, and/or introducing a polynucleotide encoding said leptin receptor and/or the functional fragment thereof into said modified immune cell.

31. The method according to claim 30, wherein said polynucleotide encoding said leptin and/or the functional fragment thereof, and/or said polynucleotide encoding said leptin receptor and/or the functional fragment thereof is contained in a plasmid.

32. The method according to claim 31, wherein said plasmid comprises a viral vector.

33. The method according to any one of claims 26-32, wherein said modified immune cell comprises a T cell.

34. The method according to any one of claims 26-33, wherein said increased expression quantity comprises that, the expression quantity of said leptin receptor and/or the functional fragment thereof in said modified immune cell is increased compared with that in an immune cell without the corresponding modification.

35. The method according to any one of claims 26-34, wherein said leptin and/or the functional fragment thereof is derived from human.

36. The method according to any one of claims 26-35, wherein said leptin receptor and/or the functional fragment thereof is derived from human.

37. The method according to any one of claims 26-36, wherein said leptin is a secretory leptin or a membrane-bound leptin.

38. The method according to any one of claims 26-37, wherein said leptin and/or the functional fragment thereof comprises an amino acid sequence as set forth in any one of SEQ ID NOs: 13-14.

39. The method according to any one of claims 26-38, wherein the functional fragment of said leptin receptor comprises a domain of said leptin receptor selected from a group consisting of: an intracellular region, a BOX 1 region, an FN3 region, a C2 region and a transmembrane region.

40. The method according to any one of claims 26-39, wherein said leptin receptor and/or the functional fragment thereof comprises an amino acid sequence as set forth in any one of SEQ ID NOs: 15-17.

41. The method according to any one of claims 26-40, wherein said chimeric antigen receptor does not comprise said leptin and/or the functional fragment thereof and/or said leptin receptor and/or the functional fragment thereof.

42. The method according to any one of claims 26-41, wherein said chimeric antigen receptor comprises an intracellular domain, said intracellular domain comprises a signaling domain and/or a costimulatory domain.

43. The method according to claim 42, wherein said signaling domain comprises a signaling domain of CD3zeta.

44. The method according to any one of claims 42-43, wherein said costimulatory domain comprises a costimulatory domain of 4-1BB.

45. The method according to any one of claims 26-44, wherein said chimeric antigen receptor comprises a hinge region.

46. The method according to claim 45, wherein said hinge region comprises a hinge region of CD8.

47. The method according to any one of claims 26-46, wherein said chimeric antigen receptor comprises a transmembrane region.

48. The method according to claim 47, wherein said transmembrane region comprises a transmembrane region of CD8.

49. The method according to any one of claims 26-48, wherein said chimeric antigen receptor comprises a targeting moiety.

50. The method according to claim 49, wherein said targeting moiety comprises a scFv.

51. The method according to any one of claims 49-50, wherein said targeting moiety specifically binds to and/or recognizes a tumor antigen.

52. The method according to any one of claims 49-51, wherein said targeting moiety specifically binds to and/or recognizes a target selected from a group consisting of: CD19, HER2, Mesothelin, GPC3 and BCMA.

53. The method according to any one of claims 26-52, wherein said chimeric antigen receptor comprises an amino acid sequence as set forth in any one of SEQ ID NOs: 23-24.

54. A method for inhibiting tumor cell proliferation and/or killing tumor cell, comprising: contacting the modified immune cell according to any one of claims 1-23 with the tumor cell.

55. The method according to claim 54, wherein said method comprises an in vitro method or an ex vivo method.

56. The method according to any one of claims 54-55, wherein said tumor comprises a solid tumor and/or a non-solid tumor.

57. The method according to any one of claims 54-56, wherein said tumor comprises B lymphocytic leukemia, breast cancer, stomach cancer, pancreatic cancer, multiple myeloma, mesothelioma, lung cancer and/or liver cancer.

58. A modified immune cell, comprising leptin and/or a functional fragment thereof, and/or, a leptin receptor and/or a functional fragment thereof, and a low-density lipoprotein-receptor-related protein or a fragment thereof.

59. The modified immune cell according to claim 58, wherein said modified immune cell comprises a T cell.

60. The modified immune cell according to any one of claims 58-59, wherein said leptin and/or the functional fragment thereof is derived from human.

61. The modified immune cell according to any one of claims 58-60, wherein said leptin receptor and/or the functional fragment thereof is derived from human.

62. The modified immune cell according to any one of claims 58-61, wherein said leptin is a secretory leptin or a membrane-bound leptin.

63. The modified immune cell according to any one of claims 58-62, wherein said leptin and/or the functional fragment thereof comprises an amino acid sequence as set forth in any one of SEQ ID NOs: 13-14.

64. The modified immune cell according to any one of claims 58-63, wherein the functional fragment of said leptin receptor comprises a domain of said leptin receptor selected from a group consisting of: an intracellular region, a BOX 1 region, an FN3 region, a C2 region and a transmembrane region.

65. The modified immune cell according to any one of claims 58-64, wherein said leptin receptor and/or the functional fragment thereof comprises an amino acid sequence as set forth in any one of SEQ ID NOs: 15-17.

66. The modified immune cell according to any one of claims 58-65, wherein said low-density lipoprotein-receptor-related protein or the fragment thereof is selected from a group consisting of: a low-density lipoprotein-receptor-related protein 6 or a truncated body thereof, and a low-density lipoprotein-receptor-related protein 5 or a truncated body thereof.

67. The modified immune cell according to claim 66, wherein the truncated body of said low-density lipoprotein-receptor-related protein 6 comprises an intracellular region of said low-density lipoprotein-receptor-related protein 6; and/or, the truncated body of said low-density lipoprotein-receptor-related protein 5 comprises an intracellular region of said low-density lipoprotein-receptor-related protein 5.

68. The modified immune cell according to any one of claims 66-67, wherein the truncated body of said low-density lipoprotein-receptor-related protein 6 comprises a functional domain selected from a group consisting of: a transmembrane region of said low-density lipoprotein-receptor-related protein 6 and an LDLR region of said low-density lipoprotein-receptor-related protein 6; and/or, wherein the truncated body of said low-density lipoprotein-receptor-related protein 5 comprises a functional domain selected from a group consisting of: a transmembrane region of said low-density lipoprotein-receptor-related protein 5 and an LDLR region of said low-density lipoprotein-receptor-related protein 5.

69. The modified immune cell according to any one of claims 58-68, wherein said low-density lipoprotein-receptor-related protein or the fragment thereof comprises an amino acid sequence as set forth in any one of SEQ ID NOs: 18-21.

70. The modified immune cell according to any one of claims 58-69, further comprising a chimeric antigen receptor or a T cell receptor.

71. The modified immune cell according to claim 70, wherein said chimeric antigen receptor does not comprise said leptin and/or the functional fragment thereof.

72. The modified immune cell according to any one of claims 70-71, wherein said chimeric antigen receptor does not comprise said leptin receptor and/or the functional fragment thereof.

73. The modified immune cell according to any one of claims 70-72, wherein said chimeric antigen receptor does not comprise said low-density lipoprotein-receptor-related protein or the fragment thereof.

74. The modified immune cell according to any one of claims 70-73, wherein said chimeric antigen receptor comprises an intracellular domain, said intracellular domain comprises a signaling domain and/or a costimulatory domain.

75. The modified immune cell according to claim 74, wherein said signaling domain comprises a signaling domain of CD3zeta.

76. The modified immune cell according to any one of claims 74-75, wherein said costimulatory domain comprises a costimulatory domain of 4-1BB.

77. The modified immune cell according to any one of claims 70-76, wherein said chimeric antigen receptor comprises a hinge region.

78. The modified immune cell according to claim 77, wherein said hinge region comprises a hinge region of CD8.

79. The modified immune cell according to any one of claims 70-78, wherein said chimeric antigen receptor comprises a transmembrane region.

80. The modified immune cell according to claim 79, wherein said transmembrane region comprises a transmembrane region of CD8.

81. The modified immune cell according to any one of claims 70-80, wherein said chimeric antigen receptor comprises a targeting moiety.

82. The modified immune cell according to claim 81, wherein said targeting moiety comprises a scFv.

83. The modified immune cell according to any one of claims 81-82, wherein said targeting moiety specifically binds to and/or recognizes a tumor antigen.

84. The modified immune cell according to any one of claims 81-83, wherein said targeting moiety specifically binds to and/or recognizes a target selected from a group consisting of: CD19, HER2, Mesothelin, GPC3 and BCMA.

85. The modified immune cell according to any one of claims 70-84, wherein said chimeric antigen receptor comprises an amino acid sequence as set forth in any one of SEQ ID NOs: 23-24.

86. A pharmaceutical composition, comprising the modified immune cell according to any one of claims 58-85 and optionally a pharmaceutically acceptable carrier.

87. A use of the modified immune cell according to any one of claims 58-85 or the pharmaceutical composition according to claim 86 in the preparation of a drug for treating tumor.

88. A method for promoting immune cell proliferation, comprising steps of:
allowing a modified immune cell to express leptin and/or a functional fragment thereof, and/or increasing the expression quantity of a leptin receptor and/or a functional fragment thereof in said modified immune cell,
and allowing said modified immune cell to express a low-density lipoprotein-receptor-related protein or a fragment thereof.

89. A method for promoting the production of memory immune cells, comprising steps of:
allowing a modified immune cell to express leptin and/or a functional fragment thereof, and/or, increasing the expression quantity of a leptin receptor and/or a functional fragment thereof in said modified immune cell,
and allowing said modified immune cell to express a low-density lipoprotein-receptor-related protein or a fragment thereof.

90. A method for enhancing the killing ability of an immune cell against tumor, comprising steps of:
allowing a modified immune cell to express leptin and/or a functional fragment thereof, and/or, increasing the expression quantity of a leptin receptor and/or a functional fragment thereof in said modified immune cell,
and allowing said modified immune cell to express a low-density lipoprotein-receptor-related protein or a fragment thereof.

91. A method for inhibiting an immune cell from expressing an immune negative regulatory protein, comprising steps of:
allowing a modified immune cell to express leptin and/or a functional fragment thereof, and/or, increasing the expression quantity of a leptin receptor and/or a functional fragment thereof in said modified immune cell,
and allowing said modified immune cell to express a low-density lipoprotein-receptor-related protein or a fragment thereof.

92. The method according to any one of claims 88-91, comprising steps of: introducing a polynucleotide encoding said leptin and/or the functional fragment thereof into said modified immune cell, and/or introducing a polynucleotide encoding said leptin receptor and/or the functional fragment thereof into said modified immune cell.

93. The method according to any one of claims 88-92, comprising steps of: introducing a polynucleotide encoding said low-density lipoprotein-receptor-related protein or the fragment thereof into said modified immune cell.

94. The method according to any one of claims 88-93, wherein said polynucleotide encoding said leptin and/or the functional fragment thereof, and/or said polynucleotide encoding said leptin receptor and/or the functional fragment thereof is contained in a plasmid.

95. The method according to any one of claims 88-94, wherein said polynucleotide encoding said low-density lipoprotein-receptor-related protein or the fragment thereof is contained in a plasmid.

96. The method according to claim 95, wherein said plasmid comprises a viral vector.

97. The method according to any one of claims 88-96, wherein said modified immune cell comprises a T cell.

98. The method according to any one of claims 88-97, wherein said leptin and/or the functional fragment thereof is derived from human.

99. The method according to any one of claims 88-98, wherein said leptin receptor and/or the functional fragment thereof is derived from human.

100. The method according to any one of claims 88-99, wherein said leptin is a secretory leptin or a membrane-bound leptin.

101. The method according to any one of claims 88-100, wherein said leptin and/or the functional fragment thereof comprises an amino acid sequence as set forth in any one of SEQ ID NOs: 13-14.

102. The method according to any one of claims 88-101, wherein the functional fragment of said leptin receptor comprises a domain of said leptin receptor selected from a group consisting of: an intracellular region, a BOX 1 region, an FN3 region, a C2 region and a transmembrane region.

103. The method according to any one of claims 88-102, wherein said leptin receptor and/or the functional fragment thereof comprises an amino acid sequence as set forth in any one of SEQ ID NOs: 15-17.

104. The method according to any one of claims 88-103, wherein said low-density lipoprotein-receptor-related protein or the fragment thereof is selected from a group consisting of: a low-density lipoprotein-receptor-related protein 6 or a truncated body thereof, and a low-density lipoprotein-receptor-related protein 5 or a truncated body thereof.

105. The method according to claim 104, wherein the truncated body of said low-density lipoprotein-receptor-related protein 6 comprises an intracellular region of said low-density lipoprotein-receptor-related protein 6; and/or, the truncated body of said low-density lipoprotein-receptor-related protein 5 comprises an intracellular region of said low-density lipoprotein-receptor-related protein 5.

106. The method according to any one of claims 104-105, wherein the truncated body of said low-density lipoprotein-receptor-related protein 6 comprises a functional domain selected from a group consisting of: a transmembrane region of said low-density lipoprotein-receptor-related protein 6 and an LDLR region of said low-density lipoprotein-receptor-related protein 6; and/or, wherein the truncated body of said low-density lipoprotein-receptor-related protein 5 comprises a functional domain selected from a group consisting of: a transmembrane region of said low-density lipoprotein-receptor-related protein 5 and an LDLR region of said low-density lipoprotein-receptor-related protein 5.

107. The method according to any one of claims 88-106, wherein said low-density lipoprotein-receptor-related protein or the fragment thereof comprises an amino acid sequence as set forth in any one of SEQ ID NOs: 18-21.

108. The method according to any one of claims 88-107, wherein said modified immune cell further comprises a chimeric antigen receptor or a T cell receptor.

109. The method according to claim 108, wherein said chimeric antigen receptor does not comprise said leptin and/or the functional fragment thereof, and/or, said leptin receptor and/or the functional fragment thereof.

110. The method according to any one of claims 108-109, wherein said chimeric antigen receptor does not comprise said low-density lipoprotein-receptor-related protein or the fragment thereof.

111. The method according to any one of claims 108-110, wherein said chimeric antigen receptor comprises an intracellular domain, said intracellular domain comprises a signaling domain and/or a costimulatory domain.

112. The method according to claim 111, wherein said signaling domain comprises a signaling domain of CD3zeta.

113. The method according to any one of claims 111-112, wherein said costimulatory domain comprises a costimulatory domain of 4-1BB.

114. The method according to any one of claims 108-113, wherein said chimeric antigen receptor comprises a hinge region.

115. The method according to claim 114, wherein said hinge region comprises a hinge region of CD8.

116. The method according to any one of claims 108-115, wherein said chimeric antigen receptor comprises a transmembrane region.

117. The method according to claim 116, wherein said transmembrane region comprises a transmembrane region of CD8.

118. The method according to any one of claims 108-117, wherein said chimeric antigen receptor comprises a targeting moiety.

119. The method according to claim 118, wherein said targeting moiety comprises a scFv.

120. The method according to any one of claims 118-119, wherein said targeting moiety specifically binds to and/or recognizes a tumor antigen.

121. The method according to any one of claims 118-120, wherein said targeting moiety specifically binds to and/or recognizes a target selected from a group consisting of: CD 19, HER2, Mesothelin, GPC3 and BCMA.

122. The method according to any one of claims 108-121, wherein said chimeric antigen receptor comprises an amino acid sequence as set forth in any one of SEQ ID NOs: 23-24.

123. A method for inhibiting tumor cell proliferation and/or killing tumor cell, comprising: contacting the modified immune cell according to any one of claims 58-85 with the tumor cell.

124. The method according to claim 123, wherein said method comprises an in vitro method or an ex vivo method.

125. The method according to any one of claims 123-124, wherein said tumor comprises solid tumor and/or non-solid tumor.

126. The method according to any one of claims 123-125, wherein said tumor comprises B lymphocytic leukemia, breast cancer, stomach cancer, pancreatic cancer, multiple myeloma, mesothelioma, lung cancer and/or liver cancer.
